# EUROPEAN PATENT SPECIFICATION

(11) **EP 2 949 724 B1**
(45) Date of publication and mention of the grant of the patent: **17.06.2020**
(21) Application number: 14742808.0
(22) Date of filing: 22.01.2014
(51) Int. Cl.: C09K 11/06, C07D 403/14, H01L 51/50

(54) **LIGHT EMITTING MATERIAL AND ORGANIC LIGHT EMITTING ELEMENT USING SAME**
LICHTEMITTIERENDES MATERIAL UND ORGANISCHES LICHTEMITTIERENDES ELEMENT DAMIT
MATÉRIAU ÉLECTROLUMINESCENT ET ÉLÉMENT ÉLECTROLUMINESCENT ORGANIQUE L'UTILISANT

(30) Priority: 23.01.2013 JP 2013010153; 23.01.2013 JP 2013010154
(43) Date of publication of application: 02.12.2015
(73) Proprietor: Kyulux, Inc., Fukuoka-shi Fukuoka 819-0388 (JP)
(72) Inventor: HIRATA Shuzo, Fukuoka-shi Fukuoka 812-8581 (JP); SAKAI Yumi, Kurume-shi Fukuoka 830-8511 (JP); MASUI Kensuke, Ashigarakami-gun Kanagawa 258-8577 (JP); SHIZU Katsuyuki, Fukuoka-shi Fukuoka 812-8581 (JP); TANAKA Hiroyuki, Fukuoka-shi Fukuoka 812-8581 (JP); ADACHI Chihaya, Fukuoka-shi Fukuoka 812-8581 (JP)
(74) Representative: Zimmermann & Partner Patentanwälte mbB
(86) International application number: PCT/JP2014/051184
(87) International publication number: WO 2014/115743

(56) References cited:
- WO-A1-2013/165192
- WO-A2-2011/049325
- WO-A2-2012/077902
- JP-A- 2002 193 952
- SEREVICIUS T ET AL.: 'Enhanced electroluminescence based on thermally activated delayed fluorescence from a carbazole -triazine derivative' PHYSICAL CHEMISTRY CHEMICAL PHYSICS vol. 15, no. 38, 01 January 2013, pages 15850 - 15855, XP055202583 DOI: 10.1039/C3CP52255E
- AYATAKA ENDO ET AL.: 'Efficient up-conversion of triplet excitons into a singlet state and its application for organic light emitting diodes' APPLIED PHYSICS LETTERS vol. 98, 01 January 2011, pages 83302 - 83302-3, XP012140060 DOI: 10.1063/1.3558906
- HIROKI UOYAMA ET AL.: 'Highly efficient organic light-emitting diodes from delayed fluorescence' NATURE vol. 492, 13 December 2012, pages 234 - 238, XP055048388 DOI: 10.1038/NATURE11687
- KENICHI GOUSHI ET AL.: 'Organic light-emitting diodes employing efficient reverse intersystem crossing for triplet-to-singlet state conversion' NATURE PHOTONICS vol. 6, 01 January 2012, pages 253 - 258, XP055133808 DOI: 10.1038/NPHOTON.2012.31
- HIRONORI KAJI ET AL.: 'Fabrication and Analysis of Organic LEDs and Solar Cells' IEICE TECHNICAL REPORT vol. 113, no. 18, 2013, pages 43 - 48, XP008180152

## Description

### Technical Field

The present invention relates to the use of a compound represented by general formula (1) as a light emitting material having a high light emission efficiency, the use of a compound represented by general formula (1) as a delayed fluorescence emitter, a compound according to general formula (2), and an organic light emitting device containing a light emitting material according to general formula (1).

### Background Art

An organic light emitting device, such as an organic electroluminescent device, has been actively studied for enhancing the light emission efficiency thereof. In particular, various studies for enhancing the light emitting efficiency have been made by newly developing and combining an electron transporting material, a hole transporting material, a light emitting material and the like constituting an organic electroluminescent device. There are studies relating to an organic electroluminescent device utilizing a compound containing a carbazole structure and a 2,4,6-triphenyl-1,3,5-triazine structure, which are found among them, and some proposals have been made hitherto.

For example, Patent Document 1 describes the use of the compound represented by the following general formula as an electron transporting material of an electron transporting layer of an organic electroluminescent device. In the following general formula, n represents 1 or 2, Ar represents an arylene group or a heteroarylene group, R₃ and R₄ each represent a hydrogen atom or an aryl group, X₁ to X₃ each represent =CR- or =N-, R represents a hydrogen atom or a substituent, and Cz represents a carbazolyl group.

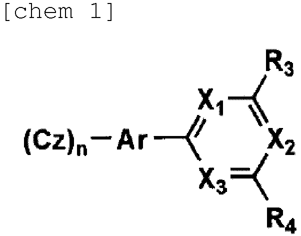

Patent Document 1 describes the compound A having the following structure as the compound represented by the general formula, and also describes the example of an organic electroluminescent device using the compound in an electron transporting layer. However, a compound obtained by introducing a substituent to the carbazolyl group of the compound A is not investigated. Furthermore, Patent Document 1 does not consider the usefulness of the compound A as a light emitting material.

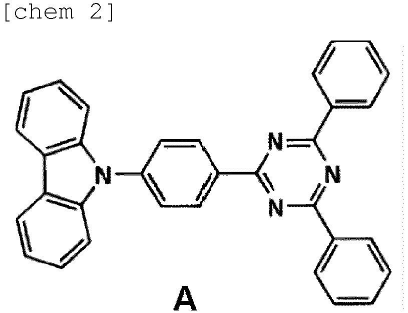

Patent Document 2 describes that the compound having the structure represented by the following general formula emits blue fluorescent light, and the compound is useful as a light emitting device material. In the following general formula, R¹¹ and R¹² each represent a hydrogen atom, an aliphatic hydrocarbon group, an aryl group or a heterocyclic group, R¹ and R² each represent a hydrogen atom or a substituent that does not contain an amino group, and L represents a linking group. Patent Document 2 describes that an organic light emitting device using the compound A as a light emitting material emits blue fluorescent light. However, Patent Document 2 also does not investigate a compound obtained by introducing a substituent to the carbazolyl group of the compound A.

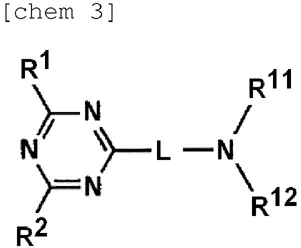

Patent Document 3 describes the compound having a partial structure containing three carbazole structures connected to each other, and describes specific examples using the compound having the partial structure as a host material of a light emitting layer of an organic light emitting device. As examples of the compound having a partial structure containing three carbazole structures connected to each other, the compound having the following structure is exemplified among the many example structures. However, there is no example that uses the compound, and the usefulness of the compound as a light emitting material is not mentioned.

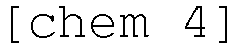

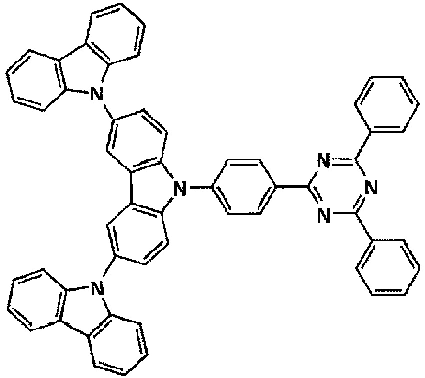

### Citation List

### Patent Documents

Patent Document 1: JP-A-2009-21336
Patent Document 2: JP-A-2002-193952
Patent Document 3: WO 2012/077902

### Summary of Invention

### Technical Problem

As described above, there have been some studies on a compound containing a carbazole structure and a 2,4,6-triphenyl-1,3,5-triazine structure, and proposals relating to application thereof to an organic electroluminescent device have been slightly made. However, like Patent Documents 1 to 3 described above, the literatures referring to the compound containing a carbazole structure and a 2,4,6-triphenyl-1,3,5-triazine structure discuss only the general usefulness of the group of compounds represented by the extensive general formulae encompassing the numerous other compounds, but do not focus the compound containing a carbazole structure and a 2,4,6-triphenyl-1,3,5-triazine structure and do not investigate the details of the compound. Accordingly, the relationship between the chemical structure of the group of compounds containing a carbazole structure and a 2,4,6-triphenyl-1,3,5-triazine structure and the usefulness of the compounds as a light emitting material has not been sufficiently clarified, and it is the current situation that it is difficult to estimate the usefulness as a light emitting material based on the chemical structure. In particular, Patent Document 1 and 2 do not evaluate the usefulness of the compounds as a light emitting material, and it is difficult to obtain any suggestion relating to the usefulness as a light emitting material based on the literatures. Furthermore, Patent Document 3 specifically describes the usefulness of the compound A as a light emitting material, but the compound A does not emit delayed fluorescent light and is not sufficiently satisfactory in the light emission efficiency.

The present inventors have considered these problems and have made investigations for evaluating in detail the usefulness of the compounds containing a carbazole structure and a 2,4,6-triphenyl-1,3,5-triazine structure as a light emitting material of an organic light emitting device. The inventors also have made investigations for providing a general formula of compounds that are particularly useful as a light emitting material and generalizing the structure of an organic light emitting device having a high light emission efficiency.

### Solution to Problem

As a result of earnest investigations for achieving the objects, the inventors have clarified that compounds that contain a carbazole structure and a 2,4,6-triphenyl-1,3,5-triazine structure and satisfy the particular structural condition are particularly useful as a light emitting material. In particular, the inventors have found compounds that are useful as a delayed fluorescent material in the compounds that contain a carbazole structure and a 2,4,6-triphenyl-1,3,5-triazine structure, and have clarified that an organic light emitting device having a high light emission efficiency may be provided inexpensively. Based on the knowledge, the inventors have provided the following inventions as measures for solving the problems.

(1) Use of a compound represented by the following general formula (1) as a light emitting material: 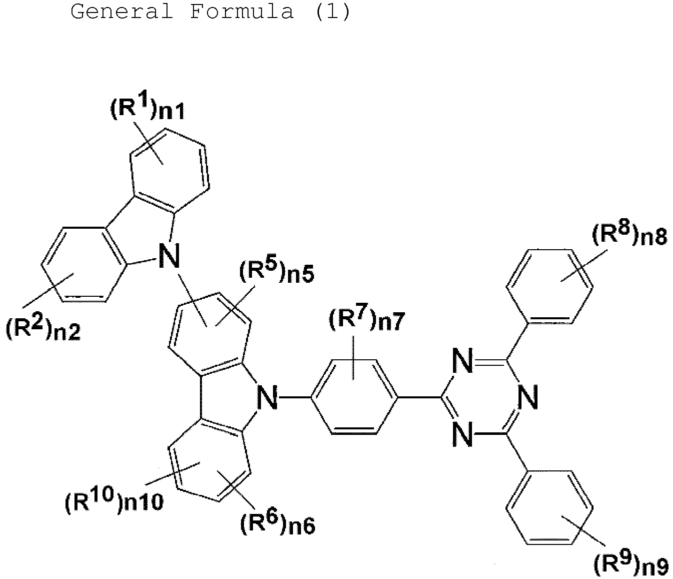 wherein in the general formula (1),
   R¹ and R² each independently represent a substituted or unsubstituted carbazolyl group, an substituted or unsubstituted aryl group having 6 to 14 carbon atoms, a heteroaryl group having 3 to 10 carbon atoms, an alkyl group having 1 to 12 carbon atoms, or a cycloalkyl group having 5 to 12 carbon atoms;
   R⁵ and R⁶ each independently represent a substituted or unsubstituted alkyl group;
   R⁷, R⁸ and R⁹ each independently represent a substituted or unsubstituted aryl group, a substituted or unsubstituted alkyl group or a substituted or unsubstituted carbazolyl group;
   whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group
   R¹⁰ represents a carbazolyl group which may be substituted with an unsubstituted carbazolyl group, an unsubstituted aryl group, an unsubstituted heteroaryl group, an unsubstituted alkyl group or an unsubstituted cycloalkyl group;
   n1, n2, n6 and n7 each independently represent an integer of from 0 to 4;
   n5 represents an integer of from 0 to 3;
   n8 and n9 each independently represent an integer of from 0 to 5; and
   n10 represents 0 or 1,
   provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.
(2) The use according to (1), wherein the compound represented by the general formula (1) is a compound represented by the following general formula (2): 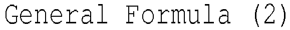 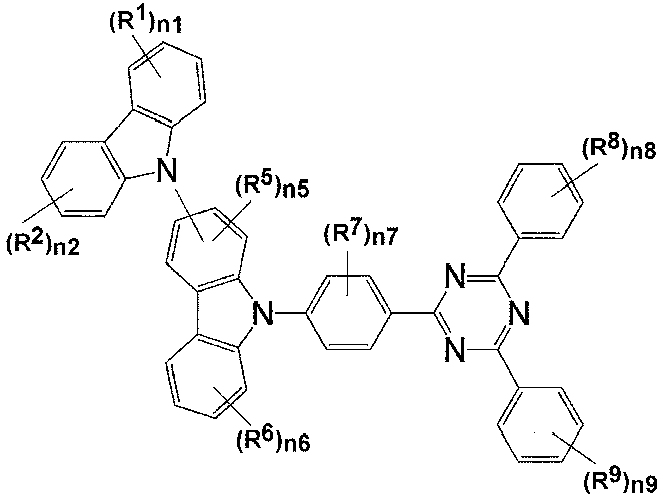 wherein in the general formula (2),
   R¹ and R² each independently represent a substituted or unsubstituted carbazolyl group, a substituted or unsubstituted aryl group, a substituted or unsubstituted heteroaryl group having 3 to 10 carbon atoms, a substituted or unsubstituted alkyl group, or a substituted or unsubstituted cycloalkyl group;
   R⁵ and R⁶ each independently represent a substituted or unsubstituted alkyl group;
   R⁷, R⁸ and R⁹ each independently represent a substituted or unsubstituted aryl group, a substituted or unsubstituted alkyl group or a substituted or unsubstituted carbazolyl group;
   whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
   n1, n2, n6 and n7 each independently represent an integer of from 0 to 4;
   n5 represents an integer of from 0 to 3; and
   n8 and n9 each independently represent an integer of from 0 to 5,
   provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.
(3) The use according to (1), wherein the compound represented by the general formula (1) is a compound represented by the following general formula (3): 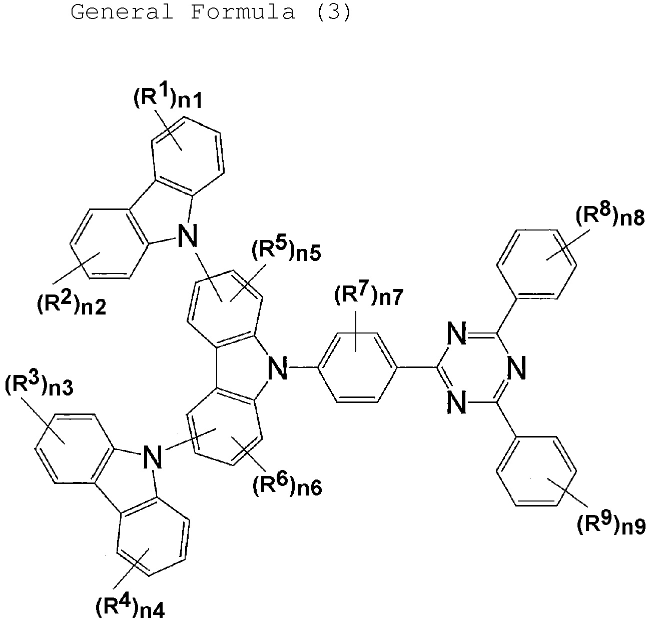 wherein in the general formula (3),
   R¹ to R⁴ each independently represent a substituted or unsubstituted carbazolyl group, an substituted or unsubstituted aryl group, a substituted or unsubstituted heteroaryl group having 3 to 10 carbon atoms, an substituted or unsubstituted alkyl grou, or a cycloalkyl group;
   R⁵ and R⁶ each independently represent a substituted or unsubstituted alkyl group;
   R⁷, R⁸ and R⁹ each independently represent a substituted or unsubstituted aryl group, a substituted or unsubstituted alkyl group or a substituted or unsubstituted carbazolyl group;
   whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
   n1 to n4 and n7 each independently represent an integer of from 0 to 4;
   n5 and n6 each represent an integer of from 0 to 3; and n8 and n9 each independently represent an integer of from 0 to 5,
   provided that when n1 to n9 each represent an integer of 2 or more, plural groups represented by R¹ to R⁹ corresponding to n1 to n9 respectively each may be the same as or different from each other.
(4) The use according to (1), wherein the light emitting material emits delayed fluorescent light.
(5) The use according to (2), wherein in the general formula (2), n6 represents 0 or n1 represents an integer of from 1 to 4.
(6) The use according to (2), wherein in the general formula (2), R¹ is bonded to the 3-position of the carbazolyl group.
(7) The use according to (2), wherein in the general formula (2), n2 represents an integer of from 1 to 4.
(8) The use according to (7), wherein in the general formula (2), R² is bonded to the 6-position of the carbazolyl group.
(9) The use according to (1) or (2), wherein in the general formulae (1) and (2), R¹ and R² each independently represent a substituted or unsubstituted 9-carbazolyl group, a substituted or unsubstituted phenyl group, a substituted or unsubstituted pyridyl group, an alkyl group having from 1 to 6 carbon atoms or a cycloalkyl group having from 5 to 7 carbon atoms, preferably R¹ and R² each independently represent a 9-carbazolyl group, a phenyl group, a tolyl group, a dimethylphenyl group, a trimethylphenyl group, a biphenyl group, a pyridyl group, a pyrrolyl group, a tert-butyl group or a cyclohexyl group.
(10) Use of a compound represented by the following general formula (1) as a delayed fluorescence emitter: 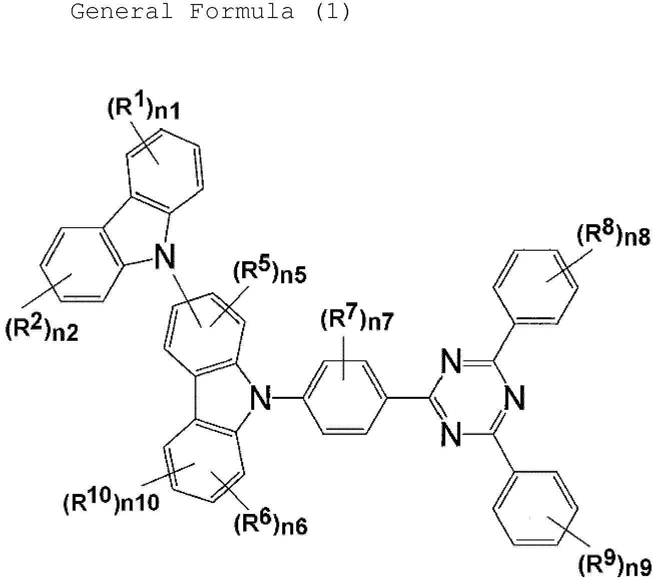 wherein in the general formula (1),
   R¹ and R² each independently represent a substituted or unsubstituted carbazolyl group, a substituted or unsubstituted aryl group having 6 to 14 carbon atoms, a substituted or unsubstituted heteroaryl group having 3 to 10 carbon atoms, a substituted or unsubstituted alkyl group having 1 to 12 carbon atoms, or a substituted or unsubstituted cycloalkyl group having 5 to 12 carbon atoms;
   R⁷, R⁸ and R⁹ each independently represent a substituted or unsubstituted aryl group, a substituted or unsubstituted alkyl group or a substituted or unsubstituted carbazolyl group;
   whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
   R¹⁰ represents a carbazolyl group which may be substituted with an unsubstituted carbazolyl group, an unsubstituted aryl group, an unsubstituted heteroaryl group, an unsubstituted alkyl group or an unsubstituted cycloalkyl group;
   n1, n2, n6 and n7 each independently represent an integer of from 0 to 4;
   n5 represents an integer of from 0 to 3;
   n8 and n9 each independently represent an integer of from 0 to 5; and
   n10 represents 0 or 1,
   provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.
(11) A compound represented by the following general formula (2) : 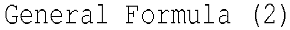 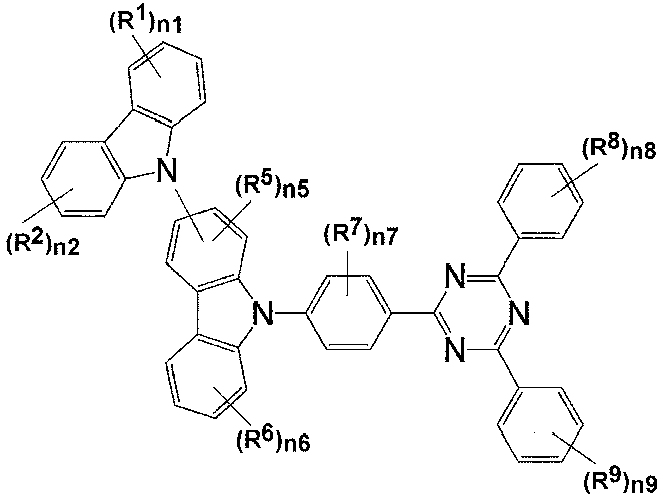 wherein in the general formula (2),
   R¹ and R² each independently represent a substituted or unsubstituted carbazolyl group, a substituted or unsubstituted aryl group having 6 to 14 carbon atoms, a substituted or unsubstituted heteroaryl group having 3 to 10 carbon atoms, a substituted or unsubstituted alkyl group having 1 to 12 carbon atoms, or a substituted or unsubstituted cycloalkyl group having 5 to 12 carbon atoms;
   R⁵ and R⁶ each independently represent a substituted or unsubstituted alkyl group;
   R⁷, R⁸ and R⁹ each independently represent a substituted or unsubstituted aryl group, a substituted or unsubstituted alkyl group or a substituted or unsubstituted carbazolyl group;
   whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
   n1, n2, n6 and n7 each independently represent an integer of from 0 to 4;
   n5 represents an integer of from 0 to 3; and
   n8 and n9 each independently represent an integer of from 0 to 5,
   provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.
(12) The compound according to claim 11, wherein the general formula (2) is represented by the following general formula (4): 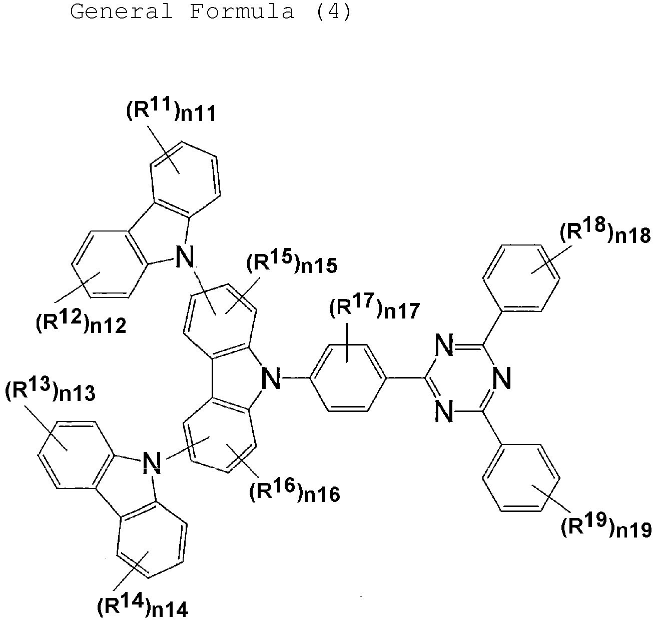 wherein in the general formula (4),
   R¹¹ to R¹⁴ each independently represent a carbazolyl group which may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group; an aryl group having 6 to 14 carbon atoms which may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group; a heteroaryl group having 3 to 10 carbon atoms, which heteroaryl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group; an alkyl group having 1 to 12 carbon atoms which may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group; or a cycloalkyl group having 5 to 12 carbon atoms which may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
   R¹⁵ and R¹⁶ each independently represent an alkyl group;
   R¹⁷, R¹⁸ and R¹⁹ each independently represent a substituted or unsubstituted aryl group, a substituted or unsubstituted alkyl group or a substituted or unsubstituted carbazolyl group;
   n11 to n14 and n17 each independently represent an integer of from 0 to 4;
   n15 and n16 each represent an integer of from 0 to 3; and
   n18 and n19 each independently represent an integer of from 0 to 5,
   provided that at least one of n11 to n14 represents an integer of from 1 to 4, and
   when n11 to n19 each represent an integer of 2 or more, plural groups represented by R¹¹ to R¹⁹ corresponding to n11 to n19 respectively each may be the same as or different from each other.
(13) An organic light emitting device containing a substrate having thereon a light emitting layer containing a host material and a light emitting material represented by the following general formula (1): 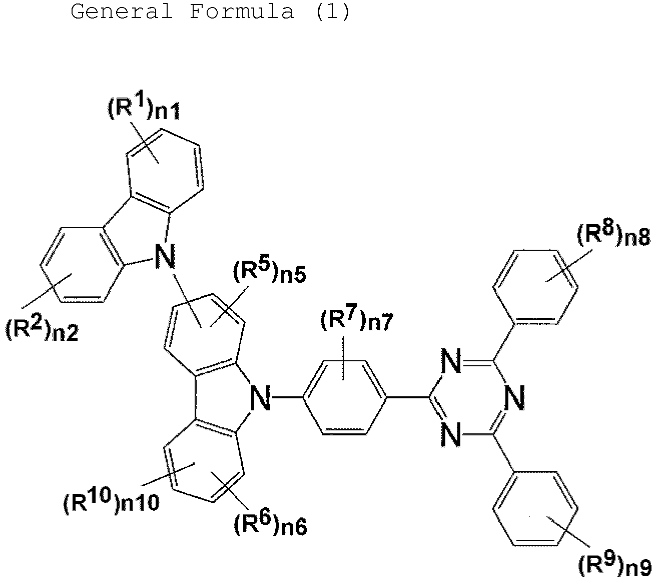 wherein in the general formula (1),
   R¹ and R² each independently represent a substituted or unsubstituted carbazolyl group, a substituted or unsubstituted aryl group having 6 to 14 carbon atoms, a substituted or unsubstituted heteroaryl group having 3 to 10 carbon atoms, a substituted or unsubstituted alkyl group having 1 to 12 carbon atoms, or a substituted or unsubstituted cycloalkyl group having 5 to 12 carbon atoms;
   R⁷, R⁸ and R⁹ each independently represent a substituted or unsubstituted aryl group, a substituted or unsubstituted alkyl group or a substituted or unsubstituted carbazolyl group;
   whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
   R¹⁰ represents a carbazolyl group which may be substituted with an unsubstituted carbazolyl group, an unsubstituted aryl group, an unsubstituted heteroaryl group, an unsubstituted alkyl group or an unsubstituted cycloalkyl group;
   n1, n2, n6 and n7 each independently represent an integer of from 0 to 4;
   n5 represents an integer of from 0 to 3;
   n8 and n9 each independently represent an integer of from 0 to 5; and
   n10 represents 0 or 1,
   provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.
(14) The organic light emitting device according to (13), wherein the organic light emitting device emits delayed fluorescent light.
(15) The organic light emitting device according to (13) or (14), wherein the organic light emitting device is an organic electroluminescent device.

### Advantageous Effects of Invention

The organic light emitting device of the invention has such a feature that the device has a high light emission efficiency. The compound and the light emitting material of the invention may be effectively used for producing the organic light emitting device. In particular, the delayed fluorescence emitter of the invention has such a feature that when the material is used in a light emitting layer of an organic light emitting device, the organic light emitting device emits delayed fluorescent light with a light emission efficiency that is drastically enhanced.

### Brief Description of Drawings

[Fig. 1] Fig. 1 is a schematic cross sectional view showing an example of a layer structure of an organic electroluminescent device.
[Fig. 2] Fig. 2 is the light emission spectra of the organic photoluminescent device and the organic electroluminescent device using the compound 1.
[Fig. 3] Fig. 3 is the transient decay curves of the organic electroluminescent device using the compound 1.
[Fig. 4] Fig. 4 is a graph showing the voltage-electric current density characteristics of the organic electroluminescent device using the compound 1.
[Fig. 5] Fig. 5 is a graph showing the electric current density-external quantum efficiency characteristics of the organic electroluminescent device using the compound 1.
[Fig. 6] Fig. 6 is the light emission spectra of the organic photoluminescent device and the organic electroluminescent device using the compound 2.
[Fig. 7] Fig. 7 is the transient decay curves of the organic electroluminescent device using the compound 2.
[Fig. 8] Fig. 8 is a graph showing the voltage-electric current density characteristics of the organic electroluminescent device using the compound 2.
[Fig. 9] Fig. 9 is a graph showing the electric current density-external quantum efficiency characteristics of the organic electroluminescent device using the compound 2.
[Fig. 10] Fig. 10 is the light emission spectra of the organic photoluminescent device and the organic electroluminescent device using the compound 3.
[Fig. 11] Fig. 11 is the transient decay curves of the organic electroluminescent device using the compound 3.
[Fig. 12] Fig. 12 is a graph showing the voltage-electric current density characteristics of the organic electroluminescent device using the compound 3.
[Fig. 13] Fig. 13 is a graph showing the electric current density-external quantum efficiency characteristics of the organic electroluminescent device using the compound 3.
[Fig. 14] Fig. 14 is the light emission spectra of the organic photoluminescent device and the organic electroluminescent device using the compound 4.
[Fig. 15] Fig. 15 is the transient decay curves of the organic electroluminescent device using the compound 4.
[Fig. 16] Fig. 16 is a graph showing the voltage-electric current density characteristics of the organic electroluminescent device using the compound 4.
[Fig. 17] Fig. 17 is a graph showing the electric current density-external quantum efficiency characteristics of the organic electroluminescent device using the compound 4.
[Fig. 18] Fig. 18 is the light emission spectra of the organic photoluminescent device and the organic electroluminescent device using the compound 27.
[Fig. 19] Fig. 19 is the transient decay curves of the organic electroluminescent device using the compound 27.
[Fig. 20] Fig. 20 is a graph showing the voltage-electric current density characteristics of the organic electroluminescent device using the compound 27.
[Fig. 21] Fig. 21 is a graph showing the electric current density-external quantum efficiency characteristics of the organic electroluminescent device using the compound 27.
[Fig. 22] Fig. 22 is the light emission spectra of the organic photoluminescent device and the organic electroluminescent device using the compound 28.
[Fig. 23] Fig. 23 is the transient decay curves of the organic electroluminescent device using the compound 28.
[Fig. 24] Fig. 24 is a graph showing the voltage-electric current density characteristics of the organic electroluminescent device using the compound 28.
[Fig. 25] Fig. 25 is a graph showing the electric current density-external quantum efficiency characteristics of the organic electroluminescent device using the compound 28.
[Fig. 26] Fig. 26 is the light emission spectra of the organic photoluminescent device and the organic electroluminescent device using the compound 29.
[Fig. 27] Fig. 27 is the transient decay curves of the organic electroluminescent device using the compound 29.
[Fig. 28] Fig. 28 is a graph showing the voltage-electric current density characteristics of the organic electroluminescent device using the compound 29.
[Fig. 29] Fig. 29 is a graph showing the electric current density-external quantum efficiency characteristics of the organic electroluminescent device using the compound 29.
[Fig. 30] Fig. 30 is the light emission spectrum of the toluene solution of the compound A.
[Fig. 31] Fig. 31 is the transient decay curves of the toluene solution of the compound A.

### Description of Embodiments

The contents of the invention will be described in detail below. The constitutional elements may be described below with reference to representative embodiments and specific examples of the invention, but the invention is not limited to the embodiments and the examples. In the description, a numerical range expressed with reference to an upper limit and/or a lower limit means a range that includes the upper limit and/or the lower limit. In the invention, the hydrogen atom that is present in the molecule in the compound used in the invention is not particularly limited in isotope species, and for example, all the hydrogen atoms in the molecule may be ¹H, and all or a part of them may be ²H (deuterium (D)).

### Compound represented by General Formula (1)

The light emitting material used according to the invention contains the compound represented by the following general formula (1) . The organic light emitting device of the invention contains the compound represented by the following general formula (1) as a light emitting material of a light emitting layer. The compound represented by the following general formula (1) will be described.

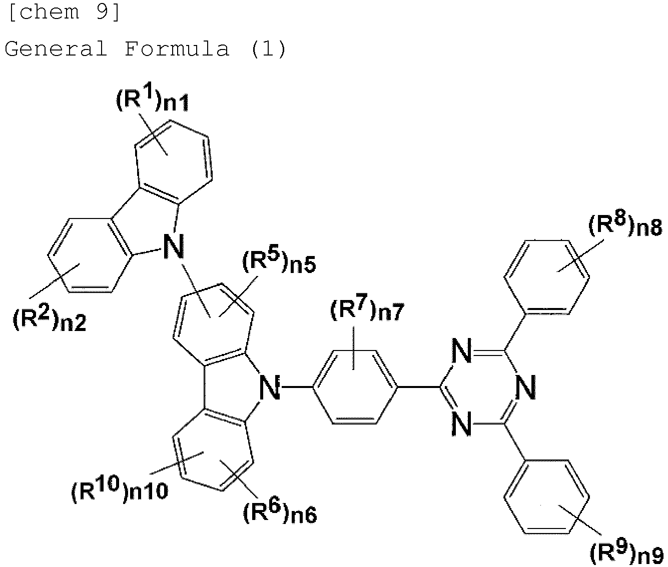

In the general formula (1), R¹ and R² each independently represent a substituted or unsubstituted carbazolyl group, a substituted or unsubstituted aryl group having 6 to 14 carbon atoms, a heteroaryl group having3 to 10 carbon atoms, an alkyl group having 1 to 10 carbon atoms or a cycloalkyl group having 5 to 12 carbon atoms.

The bonding position of the carbazolyl group herein is not limited, and is preferably a 9-carbazolyl group or a 3-carbazolyl group, and more preferably a 9-carbazolyl group.

The aryl group may be a monocyclic ring or a fused ring, and has from 6 to 14 carbon atoms, and preferably from 6 to 10 carbon atoms. Preferred examples thereof include a phenyl group.

The heteroaryl group may be a monocyclic ring or a fused ring, and from 3 to 10 carbon atoms, and preferably from 3 to 6 carbon atoms. Specific examples thereof include a pyridyl group and a pyrrolyl group, and preferred examples thereof include a 1-pyridyl group, a 2-pyridyl group, a 3-pyridyl group, a 1-pyrrolyl group, a 2-pyrrolyl group and a 3-pyrrolyl group.

The alkyl group may be linear or branched, and has from 1 to 12 carbon atoms, preferably from 1 to 6 carbon atoms, and further preferably from 1 to 4 carbon atoms. Specific examples thereof include a methyl group, an ethyl group, a n-propyl group, an isopropyl group, a n-butyl group, an isobutyl group and a tert-butyl group, and preferred examples thereof include a tert-butyl group.

The cycloalkyl group may be a monocyclic ring or a fused ring, and has from 5 to 12 carbon atoms, and preferably from 5 to 7 carbon atoms. Specific examples thereof include a cyclopentyl group, a cyclohexyl group and a cycloheptyl group, and preferred examples thereof include a cyclohexyl group.

The carbazolyl group and the aryl group, capable of being represented by R¹ and R² each may have a substituent. In the case where the group has a substituent, the substitution position and the number of the substituent are not particularly limited. The number of the substituent on the group is preferably from 0 to 6, and more preferably from 0 to 4, and for example, may be preferably from 0 to 2. In the case where the group has plural substituents, the substituents may be the same as or different from each other, and preferably the same as each other. Examples of the substituent include a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, and a nitro group. In these specific examples, the substituent that is capable of being further substituted with a substituent may be substituted. The carbazolyl group, the aryl group, the heteroaryl group, the alkyl group and the cycloalkyl group capable of being represented by R¹ and R² in the general formula (1) that are unsubstituted are also preferred. An alkyl-substituted aryl group is also preferred, and examples thereof include a tolyl group, a dialkylphenyl group and a trialkylphenyl group, specific examples of which include a 1-tolyl group, a 2-tolyl group, a 3-tolyl group, a 2,6-dimethylphenyl group, a 2,4-dimethylphenyl group and a 2,4,6-trimethylphenyl group.

In the general formula (1), R¹ and R² each preferably independently represent a substituted or unsubstituted 9-carbazolyl group, a substituted or unsubstituted phenyl group, a substituted or unsubstituted pyridyl group, an alkyl group having from 1 to 6 carbon atoms or a cycloalkyl group having from 5 to 7 carbon atoms. R¹ and R² each more preferably independently represent a 9-carbazolyl group, a phenyl group, a tolyl group, a dimethylphenyl group, a trimethylphenyl group, a biphenyl group, a pyridyl group, a pyrrolyl group, a tert-butyl group or a cyclohexyl group.

In the general formula (1), n1 and n2 each independently represent an integer of from 0 to 4, preferably an integer of from 0 to 3, and more preferably an integer of from 0 to 2. In the case where n1 is 2 or more, plural groups represented by R¹ may be the same or different, and in the case where n2 is 2 or more, plural groups represented by R² may be the same or different. n1 and n2 may be the same as or different from each other. Examples of the case where n1 and n2 are the same as each other include the case where both of them are 0, the case where both of them are 1, and the case where both of them are 2. Examples of the case where n1 and n2 are different from each other include the case where n1 is 1 and n2 is 0.

In the general formula (1), R⁵ and R⁶ each independently represent a substituted or unsubstituted alkyl group. For the description and the preferred range of the alkyl group, reference may be made to the description for the alkyl group capable of being represented by R¹ and R². For example, an unsubstituted alkyl group may be used as R⁵ and R⁶, and a methyl group may be preferably used as R⁵ and R⁶.

n5 represents an integer of from 0 to 3, and n6 represents an integer of from 0 to 4. n5 and n6 each preferably independently represent an integer of from 0 to 2, and more preferably 0 or 1, and the case where both of them are 0 is also preferred. In particular, the case where n6 is 0 is preferably used. In the case where n5 is 2 or more, plural groups represented by R⁵ may be the same or different, and in the case where n6 is 2 or more, plural groups represented by R⁶ may be the same or different. n5 and n6 may be the same as or different from each other. Preferred examples of the case where n5 and n6 are the same as each other include the case where both of them are 0 and the case where both of them are 1.

In the general formula (1), R⁷, R⁸ and R⁹ each independently represent a substituted or unsubstituted aryl group, a substituted or unsubstituted alkyl group or a substituted or unsubstituted carbazolyl group, whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group. For the descriptions and the preferred ranges of the aryl group, the alkyl group, the carbazolyl group and the substituent, reference may be made to the corresponding descriptions for R¹ and R².

n7 represents an integer of from 0 to 4, and n8 and n9 each independently represent an integer of from 0 to 5. n7 preferably represents an integer of from 0 to 2, and more preferably 0 or 1, and n7 also preferably represents 0. N8 and n9 each preferably represent an integer of from 0 to 2, and more preferably 0 or 1. In the case where n7 is 2 or more, plural groups represented by R⁷ may be the same or different, in the case where n8 is 2 or more, plural groups represented by R⁸ may be the same or different, and in the case where n9 is 2 or more, plural groups represented by R⁹ may be the same or different. n8 and n9 may be the same as or different from each other. Preferred examples of the case where n8 and n9 are the same as each other include the case where both of them are 0 and the case where both of them are 1. Examples of the case where n8 and n9 are different from each other include the case where n8 is 1 and n9 is 0.

Preferred examples of R⁸ and R⁹ in the general formula (1) include a phenyl group, a tolyl group, a dimethylphenyl group and a trimethylphenyl group, and more specific examples thereof include a phenyl group, a 1-tolyl group, a 2-tolyl group, a 3-tolyl group, a 2,6-dimethylphenyl group, a 2,4-dimethylphenyl group and a 2,4,6-trimethylphenyl group. The case where R⁸ and R⁹ in the general formula (1) each represent a substituted or unsubstituted carbazoyl group is also preferred. In this case, the substituted or unsubstituted carbazoyl group is preferably bonded to the 4-position of the phenyl group bonded to the triazine ring. Examples of the substituted carbazolyl group herein include a carbazolyl group substituted with a carbazolyl group. Preferred examples thereof include a structure represented by the following general formula (5).

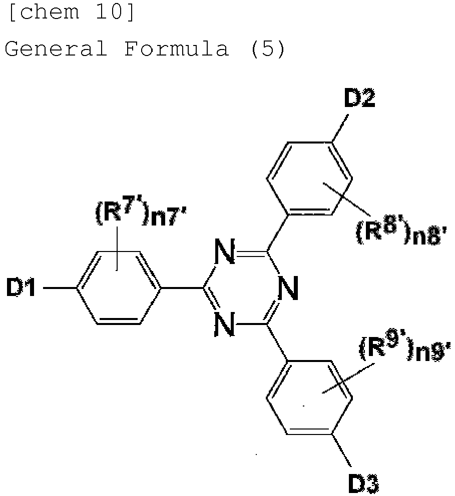

In the general formula (5), D1 to D3 each independently have a structure represented by the following general formula (6), provided that D3 may represent a hydrogen atom, a substituted or unsubstituted aryl group or a substituted or unsubstituted alkyl group; R^{7'}, R^{8'} and R^{9'} each independently represent a substituted or unsubstituted aryl group or a substituted or unsubstituted alkyl group; and n7', n8' and n9' each independently represent an integer of from 0.4.

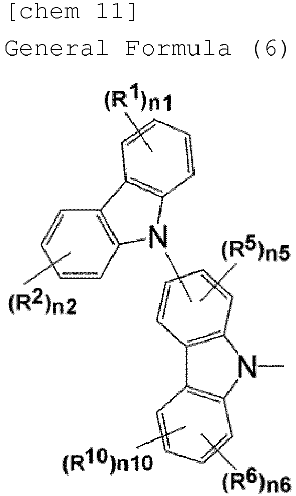

In the general formula (6), for the definitions and the preferred ranges of R¹, R², R⁵, R⁶, n1, n2, n5 and n6, reference may be made to the corresponding descriptions therefor in the general formula (1).

In the general formula (5), D1 and D2 may be the same as or different from each other. In the case where D6 has the structure represented by the general formula (6), all D1 to D3 may be the same as each other, only two of them may be the same as each other, or all of them may be different from each other. The compound having D1 to D3 that are the same as each other has such an advantage that the compound may be easily synthesized.

In the general formulae (1) and (6), R¹⁰ represents a carbazolyl group, provided that the carbazolyl group may be substituted with a substituted or unsubstituted carbazolyl group, a substituted or unsubstituted aryl group, a substituted or unsubstituted heteroaryl group, a substituted or unsubstituted alkyl group or a substituted or unsubstituted cycloalkyl group. For the descriptions and the preferred ranges of the carbazolyl group, the aryl group, the heteroaryl group, the alkyl group, the cycloalkyl group and the substituent, reference may be made to the corresponding descriptions for R¹ and R².

n10 represents 0 or 1. The compound represented by the general formula (1), in which n10 represents 0, may be represented by the following general formula (2), and the compound represented by the general formula (1), in which n10 represents 1, may be represented by the following general formula (3).

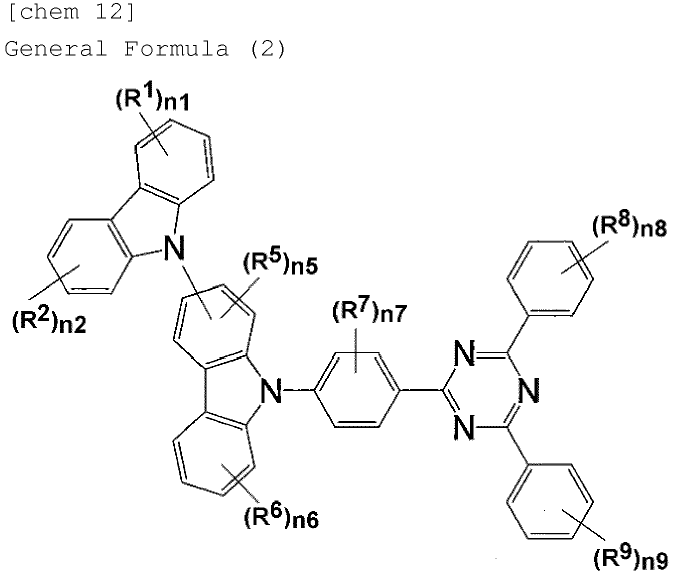

In the general formula (2), for the definitions and the preferred ranges of R¹, R², R⁵ to R⁹, n1, n2, and n5 to n9, reference may be made to the corresponding descriptions therefor in the general formula (1).

In the general formula (2), in the case where at least one of R¹ and R² is present, the bonding position thereof may be any of the 1- to 8-positions of the carbazole ring, and is preferably any of the 2- to 7-positions, more preferably any of the 3-, 4-, 6- and 7-positions, and further preferably any of the 3- and 6-positions. Preferred examples of the case include the case where n1 represents an integer of from 1 to 4, and R¹ is bonded to the 3-position of the carbazole ring, the case where n2 represents an integer of from 1 to 4, and R² is bonded to the 6-position of the carbazole ring, and the case where both n1 and n2 each represent an integer of from 1 to 4, and R¹ is bonded to the 3-position of the carbazole ring, whereas R² is bonded to the 6-position thereof. In the general formula (2), in the case where both R¹ and R² are present, R¹ and R² may be the same as or different from each other.

In the general formula (2), in the case where R⁵ is present, the substitution position thereof may be any of the positions among the 1- to 4-positions of the carbazole ring that do not have the carbazolyl group substituted thereon. The carbazolyl group is preferably substituted on the 3-position of the carbazole ring. In the case where R⁶ is present, the substitution position thereof is preferably any of the 5-, 6-and 7-positions among the 5- to 8-positions of the carbazole ring, more preferably any of the 6- and 7-positions thereof, and further preferably the 6-position thereof.

In the case where R⁷ is present, the substitution position thereof may be any position on the benzene ring. In the case where R⁸ and R⁹ are present, the substitution positions thereof may be any of the 2- to 6-positions.

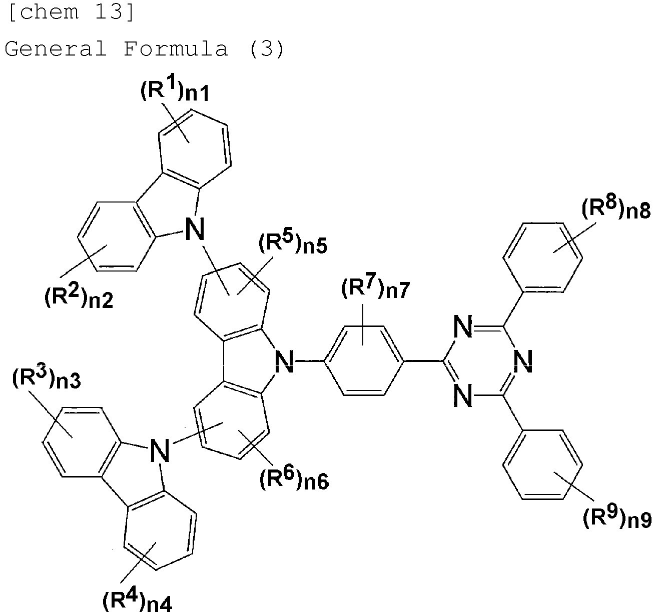

In in the general formula (3), for the definitions and the preferred ranges of R¹, R², R⁵ to R⁹, n1, n2, and n5 to n9, reference may be made to the corresponding descriptions therefor in the general formula (1).

In the general formula (3), R³ and R⁴ each independently represent a substituted or unsubstituted carbazolyl group, a substituted or unsubstituted aryl group, a substituted or unsubstituted heteroaryl group having 3 to 10 carbon atoms, a substituted or unsubstituted alkyl group or a cycloalkyl group. For the descriptions and the preferred ranges of the carbazolyl group, the aryl group, the heteroaryl group, the alkyl group, the cycloalkyl group and the substituent, reference may be made to the corresponding descriptions for R¹ and R² in the general formula (1).

In the general formula (3), n3 and n4 each independently represent an integer of from 0 to 4, preferably an integer of from 0 to 3, and more preferably an integer of from 0 to 2. In the case where n3 is 2 or more, plural groups represented by R³ may be the same or different, and in the case where n4 is 2 or more, plural groups represented by R⁴ may be the same or different.

n1 to n4 may be the same as or different from each other. Examples of the case where n1 to n4 are the same as each other include the case where all of them are 0, the case where all of them are 1, and the case where all of them are 2. Examples of the case where n1 to n4 are different from each other include the case where n1 and n3 are 1, and n2 and n4 are 0, and the case where n1 and n2 are 1, and n3 and n4 are 0. In the general formula (3), at least one of n1 to n4 is preferably an integer of from 1 to 4, and n1 to n4 each preferably independently represent an integer of from 1 to 4.

In the general formula (3), in the case where at least one of R¹ to R⁴ is present, the bonding position thereof may be any of the 1- to 8-positions of the carbazole ring, and is preferably any of the 2- to 7-positions, more preferably any of the 3-, 4-, 6- and 7-positions, and further preferably any of the 3- and 6-positions. Examples of the case include the case where R¹ is bonded only to the 3-position, the case where R¹ is bonded to the 3-position, and R² is bonded to the 6-position, the case where R¹ and R³ are bonded to the 3-position, and the case where R¹ and R³ are bonded to the 3-position, and R² and R⁴ are bonded to the 6-position. In the general formula (3), in the case where two or more of R¹ to R⁴ are present, they may be the same as or different from each other.

In the general formula (3), in the case where R⁵ is present, the substitution position thereof may be any of the positions among the 1- to 4-positions of the carbazole ring that do not have the carbazolyl group substituted thereon. In the case where R⁶ is present, the substitution position thereof may be any of the positions among the 5- to 8-positions of the carbazole ring that do not have the carbazolyl group substituted thereon. The carbazolyl group is preferably substituted on the 3- and 6-positions of the carbazole ring.

In the case where R⁷ is present, the substitution position thereof may be any position on the benzene ring. In the case where R⁸ and R⁹ are present, the substitution positions thereof may be any of the 2- to 6-positions.

Specific examples of the compound represented by the general formula (1) are shown below, but the compound represented by the general formula (1) capable of being used in the invention is not construed as being limited to the specific examples.

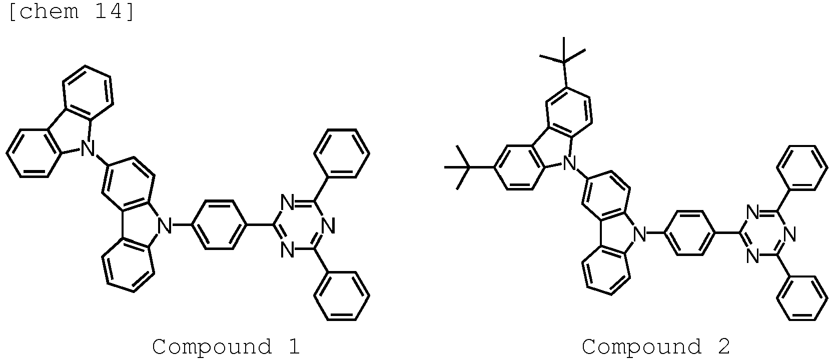

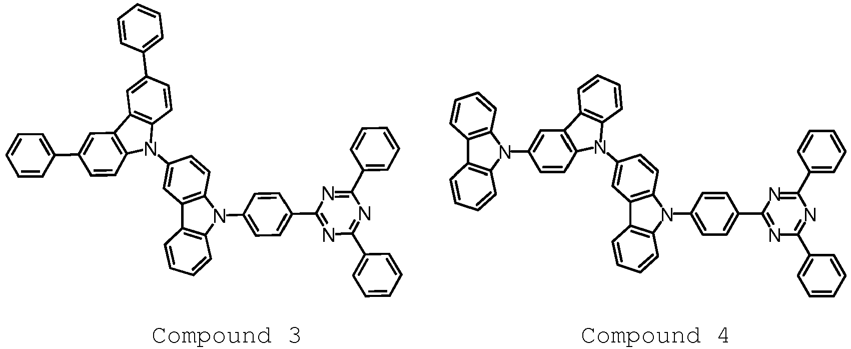

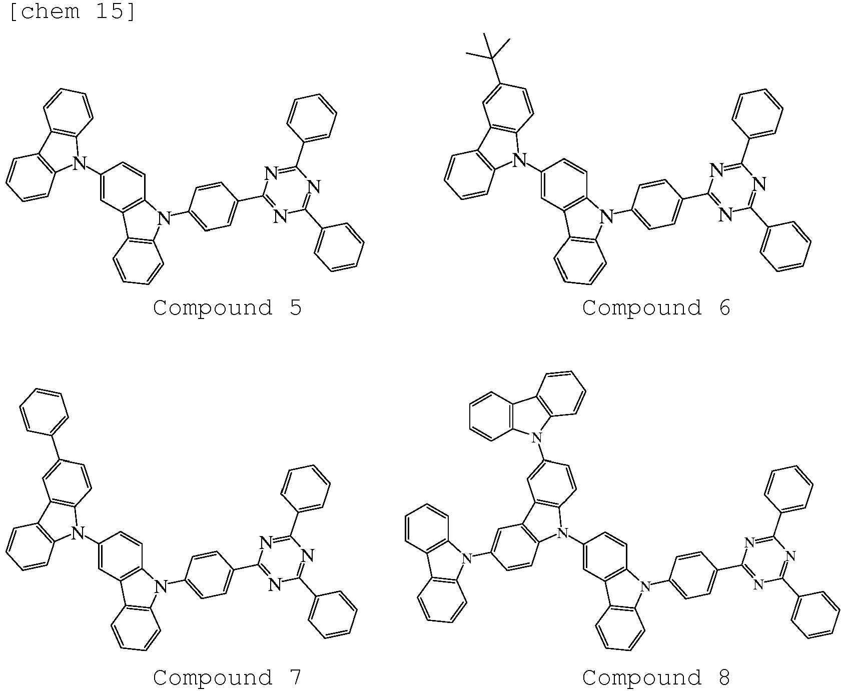

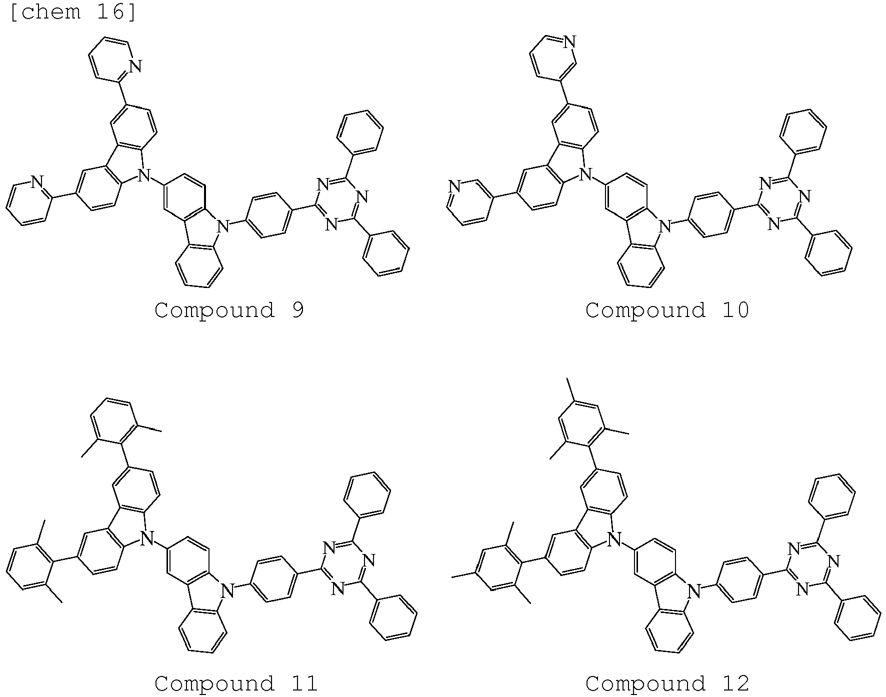

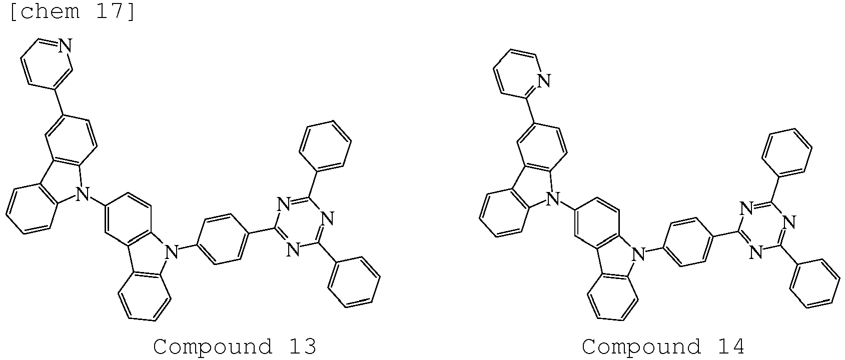

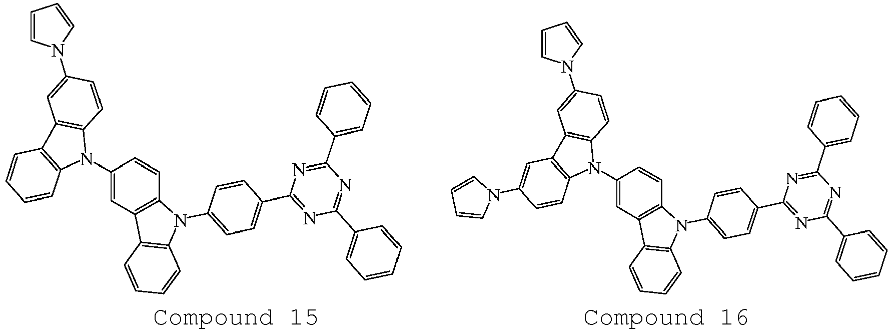

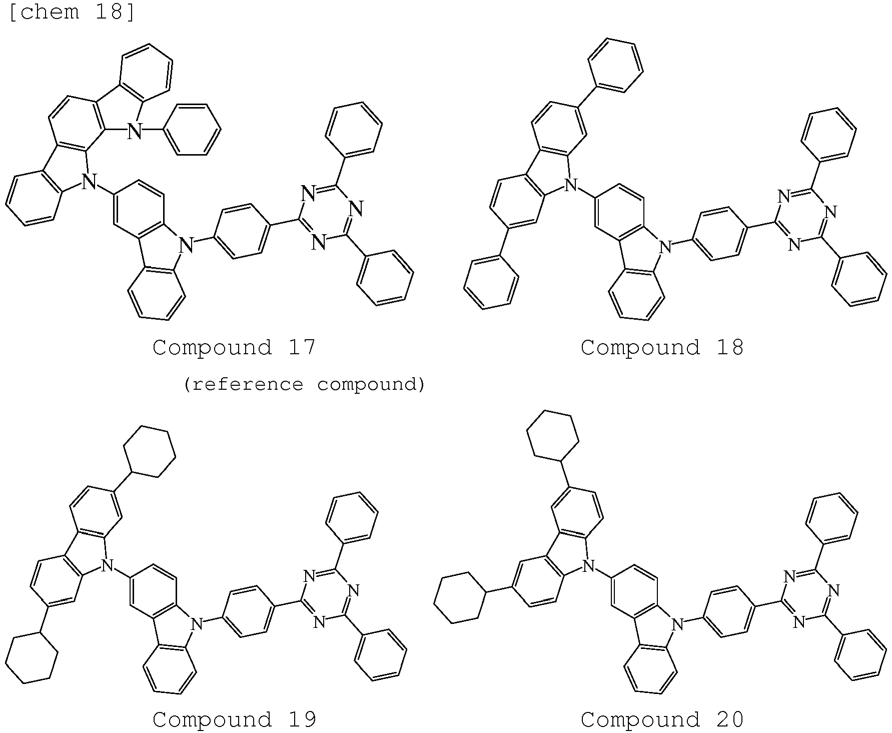

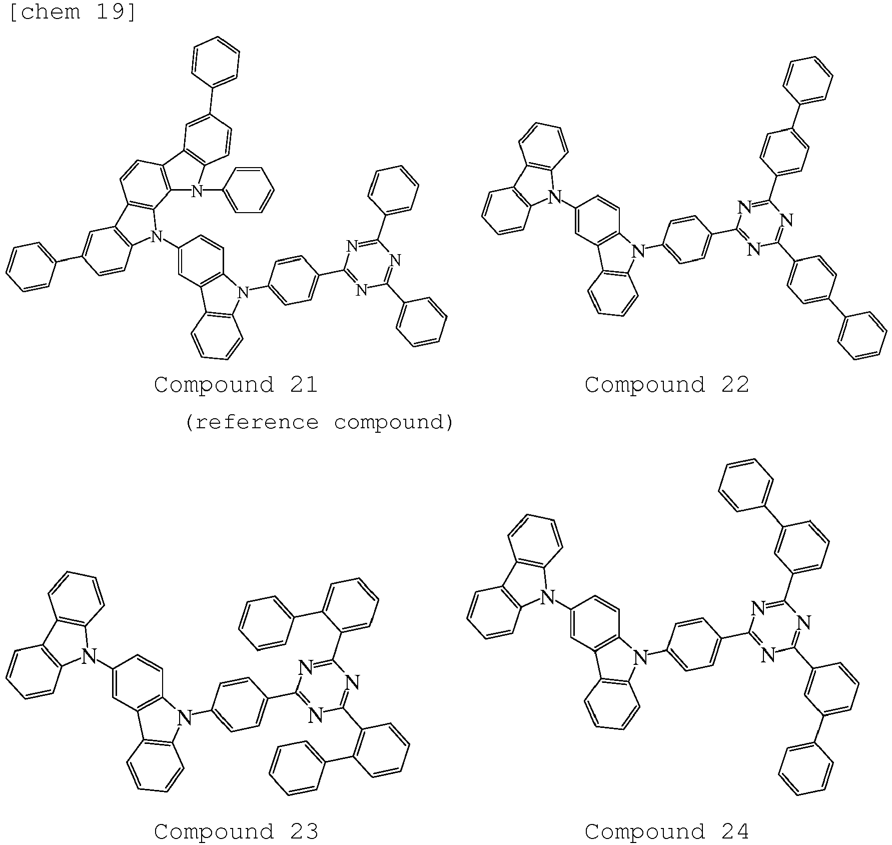

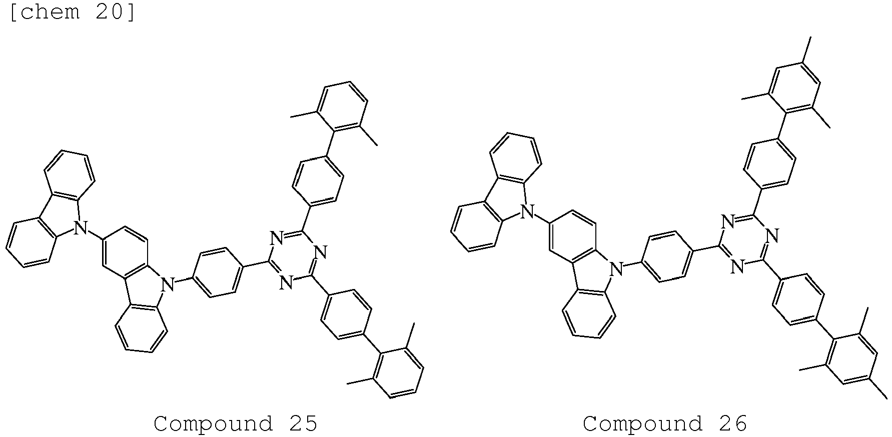

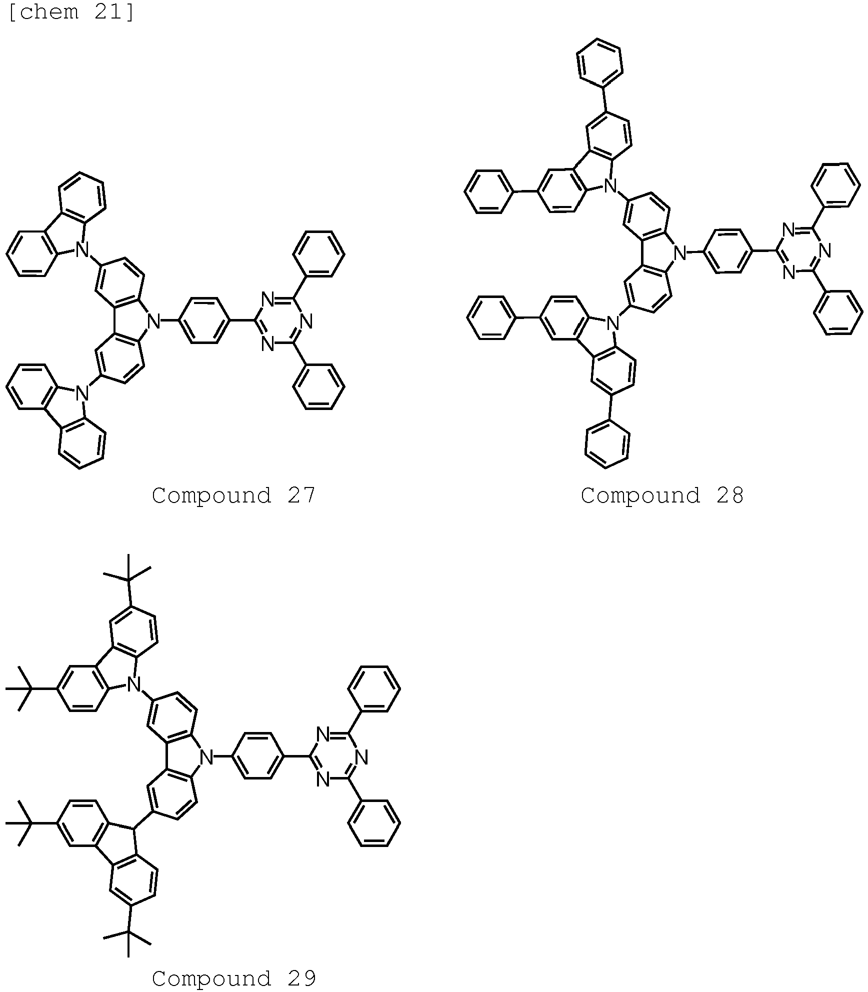

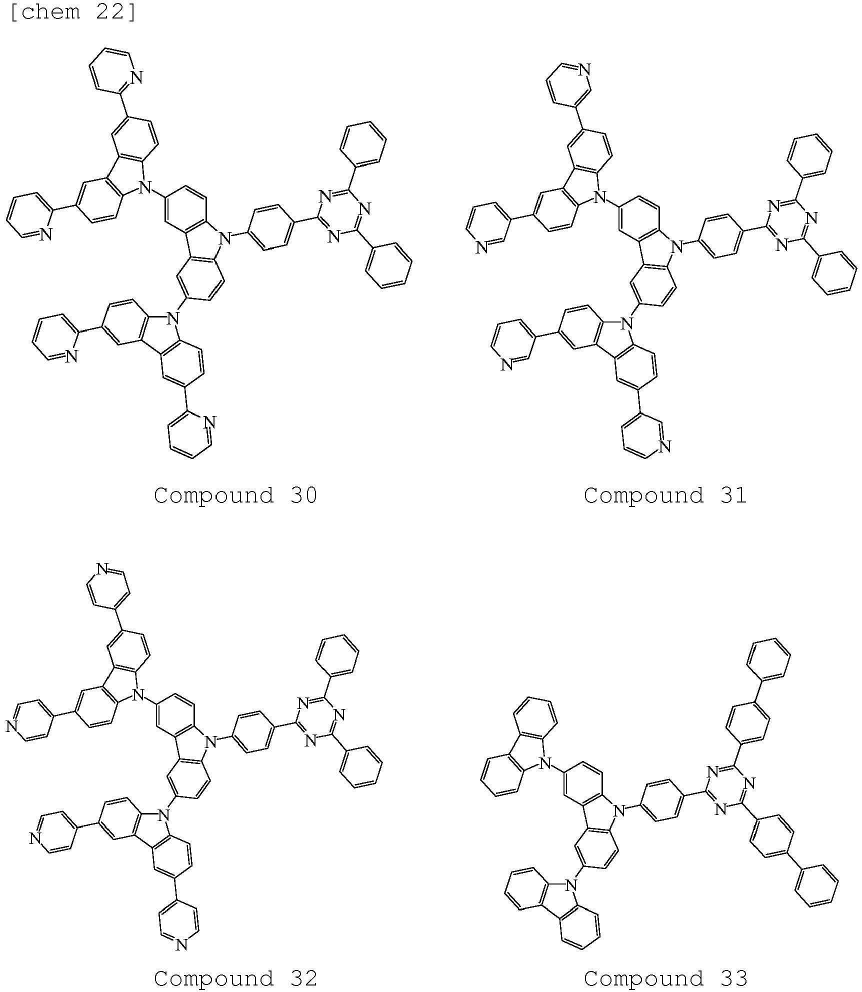

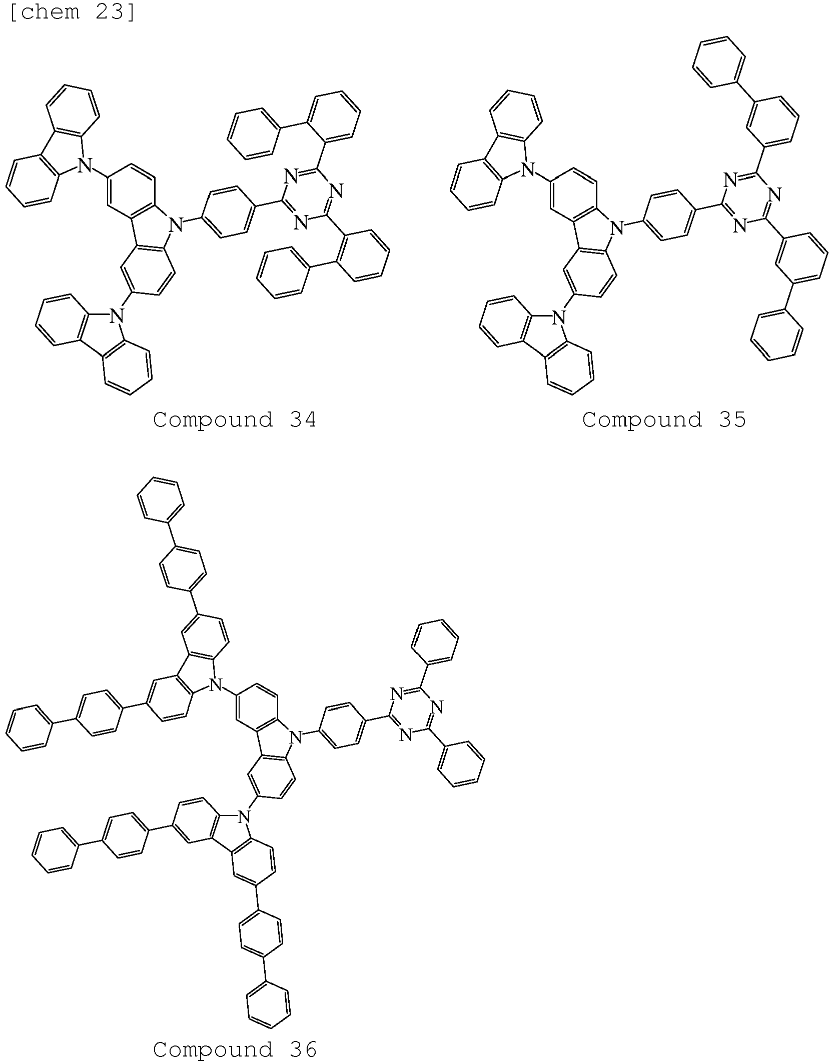

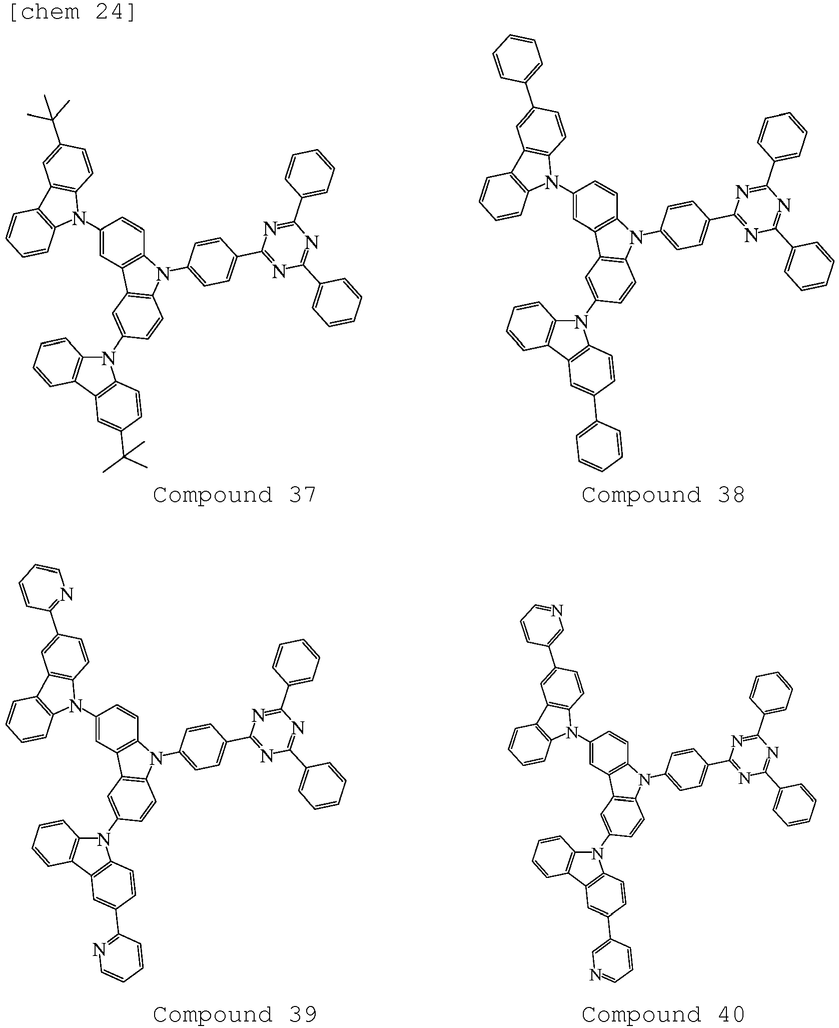

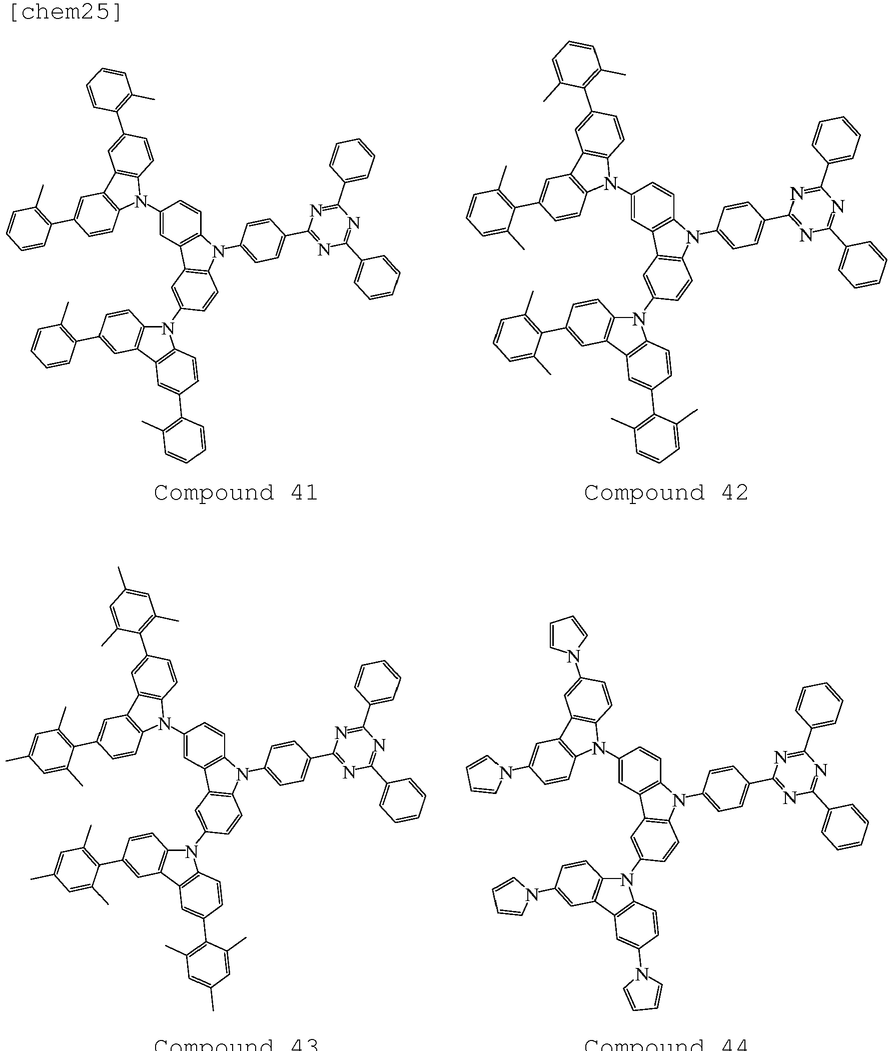

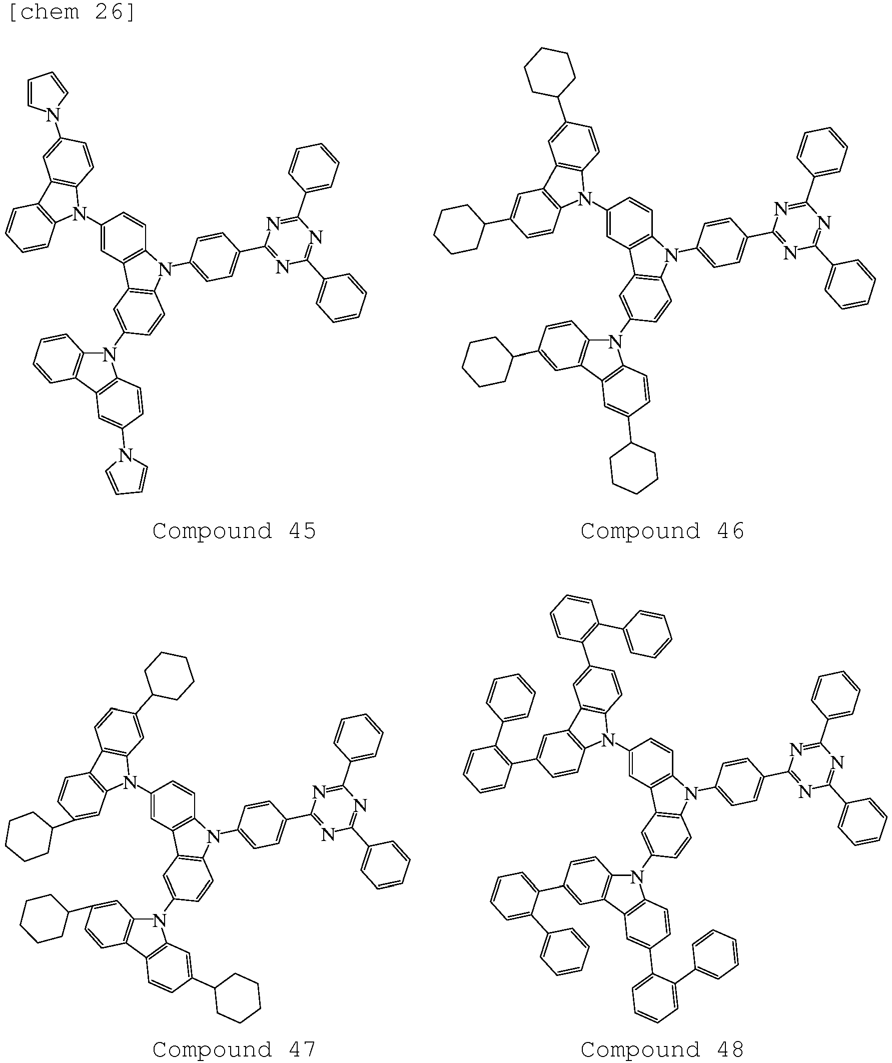

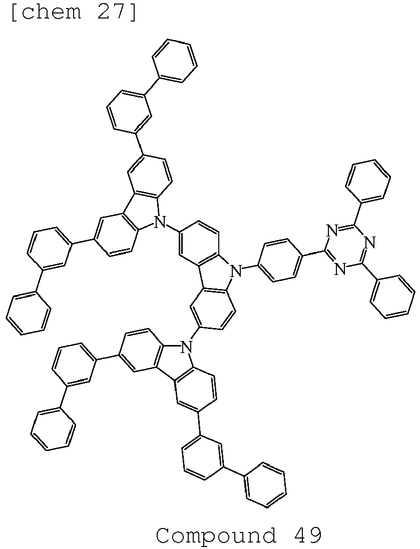

The molecular weight of the compound represented by the general formula (1) is preferably 1, 500 or less, more preferably 1,200 or less, further preferably 1,000 or less, and still further preferably 800 or less, for example, in the case where an organic layer containing the compound represented by the general formula (1) is intended to be formed as a film by a vapor deposition method. The lower limit of the molecular weight is 639 or more for the compound represented by the general formula (2), and 804 or more for the compound represented by the general formula (3).

The compound represented by the general formula (1) may be formed into a film by a coating method irrespective of the molecular weight thereof. The compound that has a relatively large molecular weight may be formed into a film by a coating method.

As an application of the invention, it may be considered that a compound that contains plural structures each represented by the general formula (1) in the molecule is used in a light emitting layer of an organic light emitting device.

For example, it may be considered that a polymerizable monomer having a structure represented by the general formula (1) is polymerized to form a polymer, and the polymer is used in a light emitting layer of an organic light emitting device. Specifically, it may be considered that a monomer that has a polymerizable functional group at any of R¹, R² and R⁵ to R¹⁰, preferably any of R¹, R², R⁸, R⁹ and R¹⁰, in the general formula (1) is prepared, and is homopolymerized or copolymerized with another monomer to prepare polymer containing repeating units, and the polymer is used in a light emitting layer of an organic light emitting device. In alternative, it may be considered that the compounds represented by the general formula (1) are coupled to form a dimer or a trimer, and the dimer or the trimer is used in a light emitting layer of an organic light emitting device.

Examples of the structure of the repeating unit constituting the polymer containing the structure represented by the general formula (1) include a structure represented by the general formula (1), in which any of R¹, R² and R⁵ to R¹⁰, preferably any of R¹, R², R⁸, R⁹ and R¹⁰, in the general formula (1) has a structure represented by the following general formula (17) or (18).

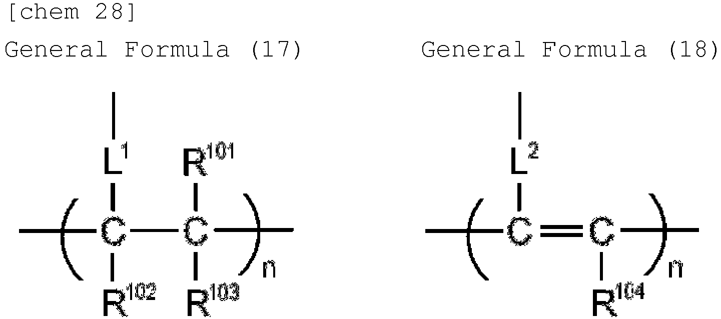

In the general formulae (17) and (18), L¹ and L² each represent a linking group. The linking group preferably has from 0 to 20 carbon atoms, more preferably from 1 to 15 carbon atoms, and further preferably from 2 to 10 carbon atoms. The linking group prefer ably has a structure represented by -X¹¹-L¹¹-, wherein X¹¹ represents an oxygen atom or a sulfur atom, and preferably an oxygen atom, and L¹¹ represents a linking group, preferably a substituted or unsubstituted alkylene group or a substituted or unsubstituted arylene group, and more preferably a substituted or unsubstituted alkylene group having from 1 to 10 carbon atoms or a substituted or unsubstituted phenylene group.

In the general formulae (17) and (18), R¹⁰¹, R¹⁰², R¹⁰³ and R¹⁰⁴ each independently represent a substituent, preferably a substituted or unsubstituted alkyl group having from 1 to 6 carbon atoms, a substituted or unsubstituted alkoxy group having from 1 to 6 carbon atoms, or a halogen atom, more preferably a substituted or unsubstituted alkyl group having from 1 to 3 carbon atoms, a substituted or unsubstituted alkoxy group having from 1 to 3 carbon atoms, a fluorine atom or a chlorine atom, and further preferably a substituted or unsubstituted alkyl group having from 1 to 3 carbon atoms or a substituted or unsubstituted alkoxy group having from 1 to 3 carbon atoms.

Specific examples of the structure of the repeating unit include a structure, in which any of R¹, R² and R⁵ to R¹⁰, preferably any of R¹, R², R⁸, R⁹ and R¹⁰, in the general formula (1) has a structure represented by any of the following formulae (21) to (24). Two or more of R¹, R² and R⁵ to R¹⁰ may have a structure represented by any of the following formulae (21) to (24), and the case where only one of R¹, R² and R⁵ to R¹⁰ has a structure represented by any of the following formulae (21) to (24) is preferred.

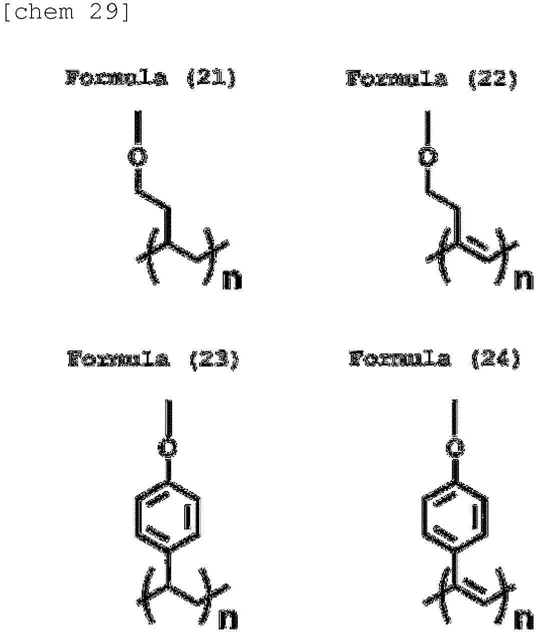

The polymer having the repeating unit containing the structure represented by any of the formulae (21) to (24) may be synthesized in such a manner that a hydroxyl group is introduced to least one of R¹, R² and R⁵ to R¹⁰, preferably at least one of R¹, R², R⁸, R⁹ and R¹⁰, in the general formula (1), and the hydroxyl group as a linker is reacted with the following compound to introduce a polymerizable group thereto, followed by polymerizing the polymerizable group.

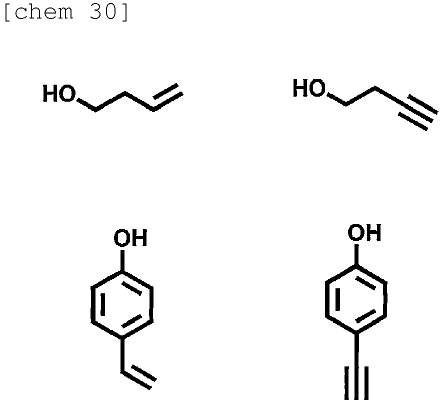

The polymer containing the structure represented by the general formula (1) may be a polymer containing only a repeating unit having the structure represented by the general formula (1), or a polymer further containing a repeating unit having another structure. The repeating unit having the structure represented by the general formula (1) contained in the polymer may be only one kind or two or more kinds. Examples of the repeating unit that does not have the structure represented by the general formula (1) include a repeating unit derived from a monomer that is used for ordinary copolymerization. Examples of the repeating unit include a repeating unit derived from a monomer having an ethylenic unsaturated bond, such as ethylene and styrene.

### Synthesis Method of Compound represented by General Formula (2)

In the compound represented by the general formula (1), the compound represented by the general formula (2) is a novel compound. The synthesis method of the compound represented by the general formula (2) is not particularly limited, and the compound represented by the general formula (2) may be synthesized by appropriately combining the known synthesis methods and conditions. For example, the compound represented by the general formula (2) may be synthesized through the following reaction.

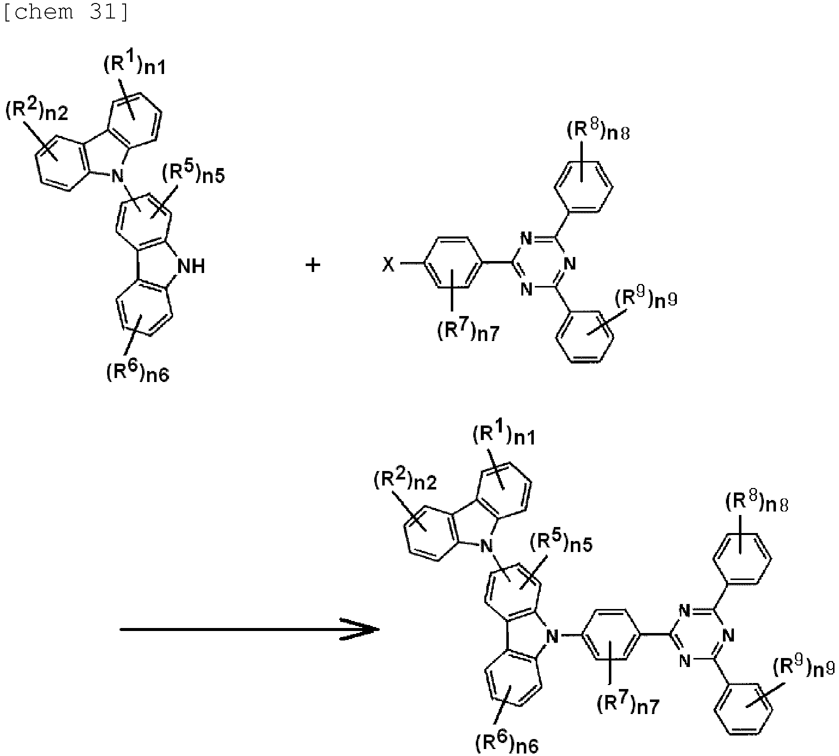

In the above formulae, R¹ R², R⁵ to R⁹, n1, n2, and n5 to n9 have the same definition as in the general formula (2), and X represents a halogen atom, preferably a chlorine atom, a bromine atom or an iodine atom, and more preferably a bromine atom. The aforementioned reaction may be performed by appropriately optimizing the conditions that are used for the ordinary coupling reaction of an amine and a halide. The amine and the halide as reaction raw materials may be synthesized by utilizing the known synthesis methods. For example, for the synthesis method of a carbazolylcarbazole, reference may be made to Appl. Phys. Lett., 101, 093306 (2012), and the like. For the details of the reaction, reference may be made to the synthesis examples described later. The compound represented by the general formula (2) may also be synthesized by combining the other known synthesis reactions.

### Compound represented by General Formula (4)

In the compound represented by the general formula (1), the compound represented by the following general formula (4) is a novel compound.

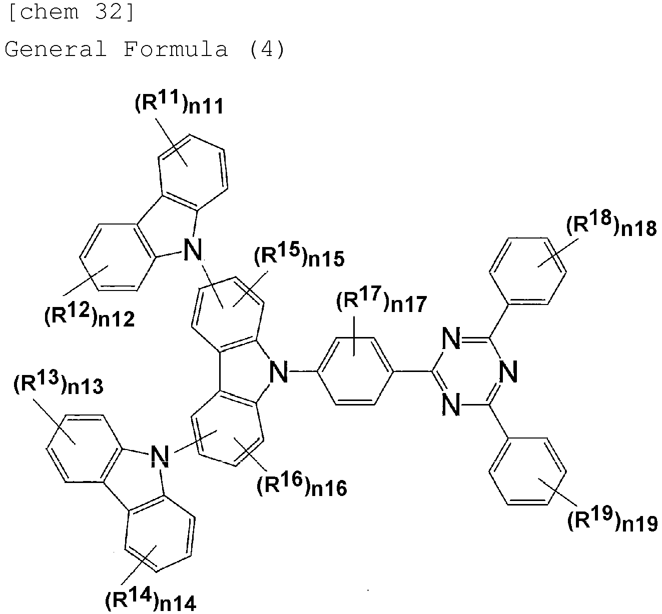

In the general formula (4), R¹¹ to R¹⁴ each independently represent a substituted or unsubstituted carbazolyl group, a substituted or unsubstituted aryl group, a substituted or unsubstituted heteroaryl group, a substituted or unsubstituted alkyl group or a substituted or unsubstituted cycloalkyl group; R¹⁵ and R¹⁶ each independently represent an alkyl group; R¹⁷, R¹⁸ and R¹⁹ each independently represent a substituted or unsubstituted aryl group, a substituted or unsubstituted alkyl group or a substituted or unsubstituted carbazolyl group; n11 to n14 and n17 each independently represent an integer of from 0 to 4; n15 and n16 each represent an integer of from 0 to 3; and n18 and n19 each independently represent an integer of from 0 to 5, provided that at least one of n11 to n14 represents an integer of from 1 to 4, and when n11 to n19 each represent an integer of 2 or more, plural groups represented by R¹¹ to R¹⁹ corresponding to n11 to n19 respectively each may be the same as or different from each other.

In the general formula (4), the preferred ranges of R¹¹ to R¹⁹ and n11 and n19, reference may be made to the descriptions for R¹ to R⁹ and n1 to n9 in the general formula (1), provided that n11 to n14 in the general formula (4) are different from n1 to n4 in the general formula (1) in such a point that that at least one of n11 to n14 represents an integer of from 1 to 4. In the general formula (4), at least two of n11 to n14 each preferably represent an integer of from 1 to 4. For example, all n11 to n14 each preferably represent an integer of from 1 to 4, and examples of the case include the case where all n11 to n14 represent 1, the case where n11 and n12 represent 1, and n13 and n14 represent 0, and the case where n11 and n13 represent 1, and n12 and n14 represent 0. In the case where R¹¹ to R¹⁴ are present, R¹¹ and R¹³ are preferably bonded to the 3-position of the carbazole ring, and R¹² and R¹⁴ are preferably bonded to the 6-position of the carbazole ring.

### Synthesis Method of Compound represented by General Formula (4)

The synthesis method of the compound represented by the general formula (4) is not particularly limited, and the compound represented by the general formula (4) may be synthesized by appropriately combining the known synthesis methods and conditions. For example, the compound represented by the general formula (4) may be synthesized through the following reaction.

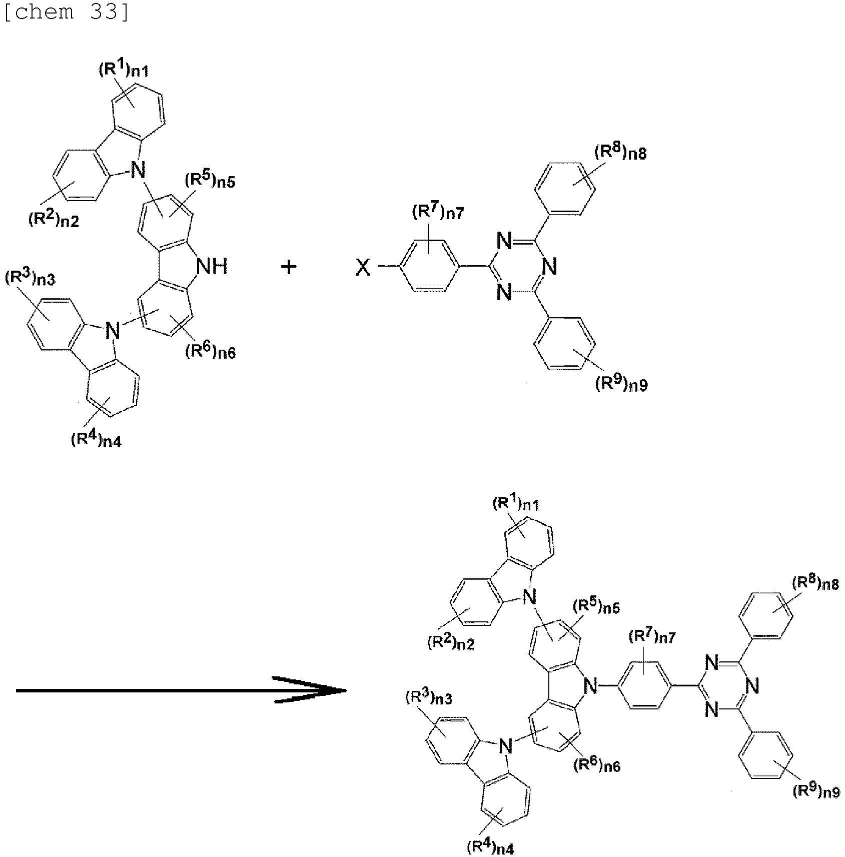

In the above formulae, R¹ to R⁹, n1 to n9 have the same definition as in the general formula (3), and X represents a halogen atom, preferably a chlorine atom, a bromine atom or an iodine atom, and more preferably a bromine atom. The aforementioned reaction may be performed by appropriately optimizing the conditions that are used for the ordinary coupling reaction of an amine and a halide. The amine and the halide as reaction raw materials may be synthesized by utilizing the known synthesis methods. For example, for the synthesis method of a carbazolylcarbazole, reference may be made to Appl. Phys. Lett., 101, 093306 (2012), and the like. For the details of the reaction, reference may be made to the synthesis examples described later. The compound represented by the general formula (4) may also be synthesized by combining the other known synthesis reactions.

### Organic Light Emitting Device

The compound represented by the general formula (1) is useful as a light emitting material of an organic light emitting device. Accordingly, the compound represented by the general formula (1) may be effectively used as a light emitting material in a light emitting layer of an organic light emitting device. The compound represented by the general formula (1) includes a delayed fluorescent material emitting delayed fluorescent light, i.e. a delayed fluorescence emitter. Thus, the invention relates to the use of a compound having the structure represented by the general formula (1) as a light emitting material, to the use of the compound represented by the general formula (1) as a delayed fluorescence emitter, and to an organic light emitting device containing a substrate having thereon a light emitting layer containing a host material and a light emitting material represented by the general formula (1). An organic light emitting device that uses the compound as a light emitting material has features that the device emits delayed fluorescent light and has a high light emission efficiency. The principle of the features may be described as follows for an organic electroluminescent device as an example.

In an organic electroluminescent device, carriers are injected from an anode and a cathode to a light emitting material to form an excited state for the light emitting material, with which light is emitted. In the case of a carrier injection type organic electroluminescent device, in general, excitons that are excited to the excited singlet state are 25% of the total excitons generated, and the remaining 75% thereof are excited to the excited triplet state. Accordingly, the use of phosphorescence, which is light emission from the excited triplet state, provides a high energy utilization. However, the excited triplet state has a long lifetime and thus causes saturation of the excited state and deactivation of energy through mutual action with the excitons in the excited triplet state, and therefore the quantum yield of phosphorescence may generally be often not high. A delayed fluorescent material emits fluorescent light through the mechanism that the energy of excitons transits to the excited triplet state through intersystem crossing or the like, and then transits to the excited singlet state through reverse intersystem crossing due to triplet-triplet annihilation or absorption of thermal energy, thereby emitting fluorescent light. It is considered that among the materials, a thermal activation type delayed fluorescent material emitting light through absorption of thermal energy is particularly useful for an organic electroluminescent device. In the case where a delayed fluorescent material is used in an organic electroluminescent device, the excitons in the excited singlet state normally emit fluorescent light. On the other hand, the excitons in the excited triplet state emit fluorescent light through intersystem crossing to the excited singlet state by absorbing the heat generated by the device. At this time, the light emitted through reverse intersystem crossing from the excited triplet state to the excited single state has the same wavelength as fluorescent light since it is light emission from the excited single state, but has a longer lifetime (light emission lifetime) than the normal fluorescent light and phosphorescent light, and thus the light is observed as fluorescent light that is delayed from the normal fluorescent light and phosphorescent light. The light may be defined as delayed fluorescent light. The use of the thermal activation type exciton transition mechanism may raise the proportion of the compound in the excited single state, which is generally formed in a proportion only of 25%, to 25% or more through the absorption of the thermal energy after the carrier injection. A compound that emits strong fluorescent light and delayed fluorescent light at a low temperature of lower than 100 ºC undergoes the intersystem crossing from the excited triplet state to the excited singlet state sufficiently with the heat of the device, thereby emitting delayed fluorescent light, and thus the use of the compound may drastically enhance the light emission efficiency.

The use of the compound represented by the general formula (1) as a light emitting material of a light emitting layer may provide an excellent organic light emitting device, such as an organic photoluminescent device (organic PL device) and an organic electroluminescent device (organic EL device). At this time, the compound represented by the general formula (1) may have a function of assisting light emission of another light emitting material contained in the light emitting layer, i.e., as a so-called assist dopant. Specifically, the compound represented by the general formula (1) contained in the light emitting layer may have a lowest excited singlet energy that is between the lowest excited singlet energy of the host material contained in the light emitting layer and the lowest excited singlet energy of the another light emitting material contained in the light emitting layer.

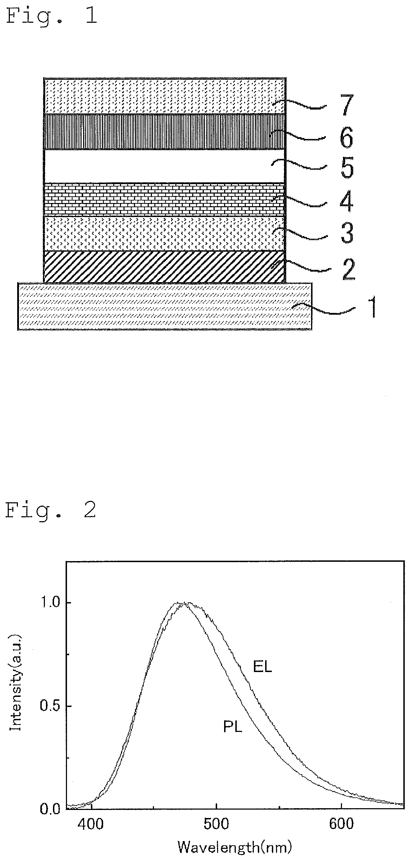

The organic photoluminescent device has a structure containing a substrate having formed thereon at least a light emitting layer. The organic electroluminescent device has a structure containing at least an anode, a cathode and an organic layer formed between the anode and the cathode. The organic layer contains at least a light emitting layer, and may be formed only of a light emitting layer, or may have one or more organic layer in addition to the light emitting layer. Examples of the organic layer include a hole transporting layer, a hole injection layer, an electron barrier layer, a hole barrier layer, an electron injection layer, an electron transporting layer and an exciton barrier layer. The hole transporting layer may be a hole injection and transporting layer having a hole injection function, and the electron transporting layer may be an electron injection and transporting layer having an electron injection function. A specific structural example of an organic electroluminescent device is shown in Fig. 1. In Fig. 1, the numeral 1 denotes a substrate, 2 denotes an anode, 3 denotes a hole injection layer, 4 denotes a hole transporting layer, 5 denotes a light emitting layer, 6 denotes an electron transporting layer, and 7 denotes a cathode.

The members and the layers of the organic electroluminescent device will be described below. The descriptions for the substrate and the light emitting layer may also be applied to the substrate and the light emitting layer of the organic photoluminescent device.

### Substrate

The organic electroluminescent device of the invention is preferably supported by a substrate. The substrate is not particularly limited and may be those that have been commonly used in an organic electroluminescent device, and examples thereof used include those formed of glass, transparent plastics, quartz and silicon.

### Anode

The anode of the organic electroluminescent device used is preferably formed of as an electrode material a metal, an alloy or an electroconductive compound each having a large work function (4 eV or more), or a mixture thereof. Specific examples of the electrode material include a metal, such as Au, and an electroconductive transparent material, such as CuI, indium tin oxide (ITO), SnO₂ and ZnO. A material that is amorphous and is capable of forming a transparent electroconductive film, such as IDIXO (In₂O₃-ZnO), may also be used. The anode may be formed in such a manner that the electrode material is formed into a thin film by such a method as vapor deposition or sputtering, and the film is patterned into a desired pattern by a photolithography method, or in the case where the pattern may not require high accuracy (for example, approximately 100 µm or more), the pattern may be formed with a mask having a desired shape on vapor deposition or sputtering of the electrode material. In alternative, in the case where a material capable of being applied as a coating, such as an organic electroconductive compound, is used, a wet film forming method, such as a printing method and a coating method, may be used. In the case where emitted light is to be taken out through the anode, the anode preferably has a transmittance of more than 10%, and the anode preferably has a sheet resistance of several hundred Ohm per square or less. The thickness thereof may be generally selected from a range of from 10 to 1,000 nm, and preferably from 10 to 200 nm, while depending on the material used.

### Cathode

The cathode is preferably formed of an electrode material a metal (referred to as an electron injection metal), an alloy or an electroconductive compound each having a small work function (4 eV or less), or a mixture thereof. Specific examples of the electrode material include sodium, a sodium-potassium alloy, magnesium, lithium, a magnesium-cupper mixture, a magnesium-silver mixture, a magnesium-aluminum mixture, a magnesium-indium mixture, an aluminum-aluminum oxide (Al₂O₃) mixture, indium, a lithium-aluminum mixture, and a rare earth metal. Among these, a mixture of an electron injection metal and a second metal that is a stable metal having a larger work function than the electron injection metal, for example, a magnesium-silver mixture, a magnesium-aluminum mixture, a magnesium-indium mixture, an aluminum-aluminum oxide (Al₂O₃) mixture, a lithium-aluminum mixture, and aluminum, are preferred from the standpoint of the electron injection property and the durability against oxidation and the like. The cathode may be produced by forming the electrode material into a thin film by such a method as vapor deposition or sputtering. The cathode preferably has a sheet resistance of several hundred Ohm per square or less, and the thickness thereof may be generally selected from a range of from 10 nm to 5 µm, and preferably from 50 to 200 nm. For transmitting the emitted light, any one of the anode and the cathode of the organic electroluminescent device is preferably transparent or translucent, thereby enhancing the light emission luminance.

The cathode may be formed with the electroconductive transparent materials described for the anode, thereby forming a transparent or translucent cathode, and by applying the cathode, a device having an anode and a cathode, both of which have transmittance, may be produced.

### Light Emitting Layer

The light emitting layer is a layer, in which holes and electrons injected from the anode and the cathode, respectively, are recombined to form excitons, and then the layer emits light. The light emitting layer contains a light emitting material and a host material. The light emitting material used may be one kind or two or more kinds selected from the group of compounds represented by the general formula (1) of the invention. In order that the organic electroluminescent device and the organic photoluminescent device of the invention exhibit a high light emission efficiency, it is important that the singlet excitons and the triplet excitons generated in the light emitting material are confined in the light emitting material. Accordingly, a host material is used in addition to the light emitting material in the light emitting layer. The host material used may be an organic compound that has excited singlet energy and excited triplet energy, at least one of which is higher than those of the light emitting material of the invention. As a result, the singlet excitons and the triplet excitons generated in the light emitting material of the invention are capable of being confined in the molecules of the light emitting material of the invention, thereby eliciting the light emission efficiency thereof sufficiently. Even though the singlet excitons and the triplet excitons are not confined sufficiently, a high light emission efficiency may be obtained in some cases, and thus a host material that is capable of achieving a high light emission efficiency may be used in the invention without any particular limitation. In the organic light emitting device and the organic electroluminescent device of the invention, the light emission occurs in the light emitting material of the invention contained in the light emitting layer. The emitted light contains both fluorescent light and delayed fluorescent light. However, a part of the emitted light may contain emitted light from the host material, or the emitted light may partially contain emitted light from the host material.

The amount of the compound represented by general formula (1) as the light emitting material contained in the light emitting layer is preferably 0.1% by weight or more, and more preferably 1% by weight or more, and is preferably 50% by weight or less, more preferably 20% by weight or less, and further preferably 10% by weight or less.

The host material in the light emitting layer is preferably an organic compound that has a hole transporting function and an electron transporting function, prevents the emitted light from being increased in wavelength, and has a high glass transition temperature.

### Injection Layer

The injection layer is a layer that is provided between the electrode and the organic layer, for decreasing the driving voltage and enhancing the light emission luminance, and includes a hole injection layer and an electron injection layer, which may be provided between the anode and the light emitting layer or the hole transporting layer and between the cathode and the light emitting layer or the electron transporting layer. The injection layer may be provided depending on necessity.

### Barrier Layer

The barrier layer is a layer that is capable of inhibiting charges (electrons or holes) and/or excitons present in the light emitting layer from being diffused outside the light emitting layer. The electron barrier layer may be disposed between the light emitting layer and the hole transporting layer, and inhibits electrons from passing through the light emitting layer toward the hole transporting layer. Similarly, the hole barrier layer may be disposed between the light emitting layer and the electron transporting layer, and inhibits holes from passing through the light emitting layer toward the electron transporting layer. The barrier layer may also be used for inhibiting excitons from being diffused outside the light emitting layer. Thus, the electron barrier layer and the hole barrier layer each may also have a function as an exciton barrier layer. The term "the electron barrier layer" or "the exciton barrier layer" referred herein is intended to include a layer that has both the functions of an electron barrier layer and an exciton barrier layer by one layer.

### Hole Barrier Layer

The hole barrier layer has the function of an electron transporting layer in a broad sense. The hole barrier layer has a function of inhibiting holes from reaching the electron transporting layer while transporting electrons, and thereby enhances the recombination probability of electrons and holes in the light emitting layer. As the material for the hole barrier layer, the materials for the electron transporting layer described later may be used depending on necessity.

### Electron Barrier Layer

The electron barrier layer has the function of transporting holes in a broad sense. The electron barrier layer has a function of inhibiting electrons from reaching the hole transporting layer while transporting holes, and thereby enhances the recombination probability of electrons and holes in the light emitting layer.

### Exciton Barrier Layer

The exciton barrier layer is a layer for inhibiting excitons generated through recombination of holes and electrons in the light emitting layer from being diffused to the charge transporting layer, and the use of the layer inserted enables effective confinement of excitons in the light emitting layer, and thereby enhances the light emission efficiency of the device. The exciton barrier layer may be inserted adjacent to the light emitting layer on any of the side of the anode and the side of the cathode, and on both the sides. Specifically, in the case where the exciton barrier layer is present on the side of the anode, the layer may be inserted between the hole transporting layer and the light emitting layer and adjacent to the light emitting layer, and in the case where the layer is inserted on the side of the cathode, the layer may be inserted between the light emitting layer and the cathode and adjacent to the light emitting layer. Between the anode and the exciton barrier layer that is adjacent to the light emitting layer on the side of the anode, a hole injection layer, an electron barrier layer and the like may be provided, and between the cathode and the exciton barrier layer that is adjacent to the light emitting layer on the side of the cathode, an electron injection layer, an electron transporting layer, a hole barrier layer and the like may be provided. In the case where the barrier layer is provided, the material used for the barrier layer preferably has excited singlet energy and excited triplet energy, at least one of which is higher than the excited singlet energy and the excited triplet energy of the light emitting layer, respectively.

### Hole Transporting Layer

The hole transporting layer is formed of a hole transporting material having a function of transporting holes, and the hole transporting layer may be provided as a single layer or plural layers.

The hole transporting material has one of injection or transporting property of holes and barrier property of electrons, and may be any of an organic material and an inorganic material. Examples of known hole transporting materials that may be used herein include a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an arylamine derivative, an amino-substituted chalcone derivative, an oxazole derivative, a styrylanthracene derivative, a fluorenone derivative, a hydrazone derivative, a stilbene derivative, a silazane derivative, an aniline copolymer and an electroconductive polymer, particularly a thiophene oligomer. Among these, a porphyrin compound, an aromatic tertiary amine compound and a styrylamine compound are preferably used, and an aromatic tertiary amine compound is more preferably used.

### Electron Transporting Layer

The electron transporting layer is formed of a material having a function of transporting electrons, and the electron transporting layer may be provided as a single layer or plural layers.

The electron transporting material (which may also function as a hole barrier material in some cases) needs only to have a function of transporting electrons, which are injected from the cathode, to the light emitting layer. Examples of the electron transporting layer that may be used herein include a nitro-substituted fluorene derivative, a diphenylquinone derivative, a thiopyran dioxide derivative, carbodiimide, a fluorenylidene methane derivative, anthraquinodimethane and anthrone derivatives, and an oxadiazole derivative. The electron transporting material used may be a thiadiazole derivative obtained by replacing the oxygen atom of the oxadiazole ring of the oxadiazole derivative by a sulfur atom, or a quinoxaline derivative having a quinoxaline ring, which is known as an electron attracting group. Furthermore, polymer materials having these materials introduced to the polymer chain or having these materials used as the main chain of the polymer may also be used.

In the production of the organic electroluminescent device, the compound represented by the general formula (1) may be used not only in the light emitting layer but also in the other layers than the light emitting layer. In this case, the compound represented by the general formula (1) used in the light emitting layer and the compound represented by the general formula (1) used in the other layers than the light emitting layer may be the same or different from each other. For example, the compound represented by the general formula (1) may be used in the injection layer, the barrier layer, the hole barrier layer, the electron barrier layer, the exciton barrier layer, the hole transporting layer, the electron transporting layer and the like described above. The film forming method of the layers are not particularly limited, and the layers may be produced by any of a dry process and a wet process.

Specific examples of preferred materials that may be used in the organic electroluminescent device are shown below, but the materials that may be used in the invention are not construed as being limited to the example compounds. The compound that is shown as a material having a particular function may also be used as a material having another function. In the structural formulae of the example compounds, R and R₁ to R₁₀ each independently represent a hydrogen atom or a substituent, and n represents an integer of from 3 to 5.

Preferred examples of a compound that may also be used as the host material of the light emitting layer are shown below.

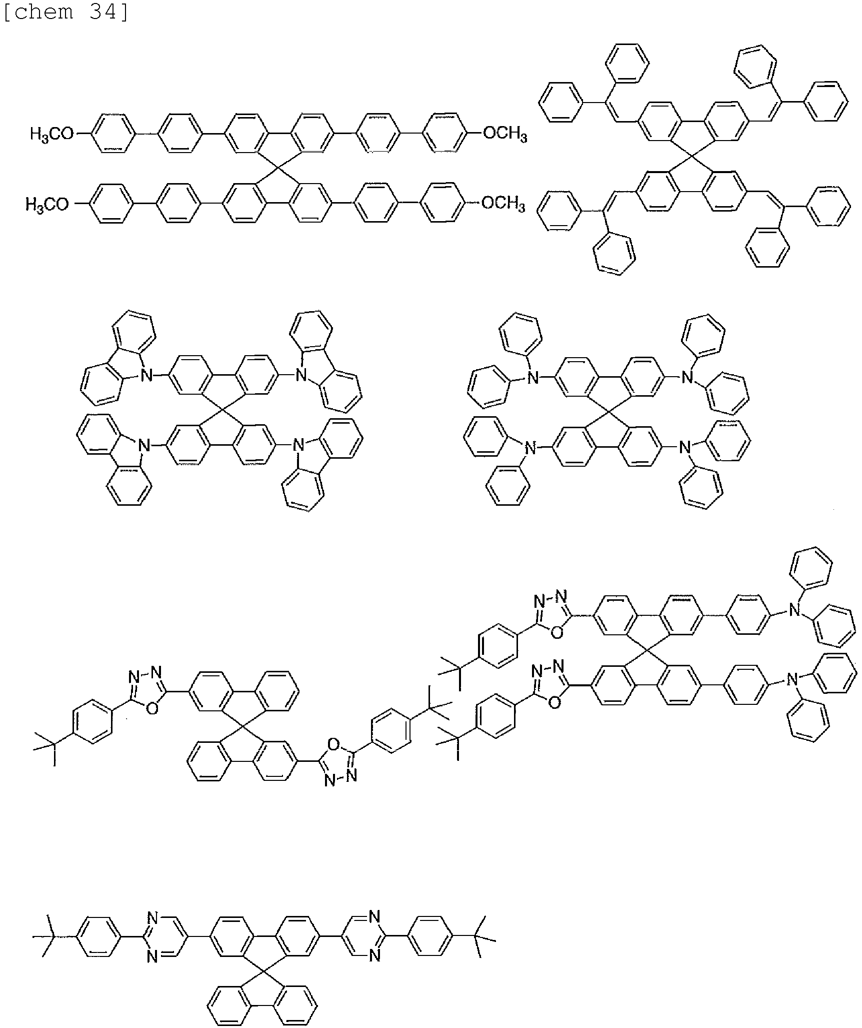

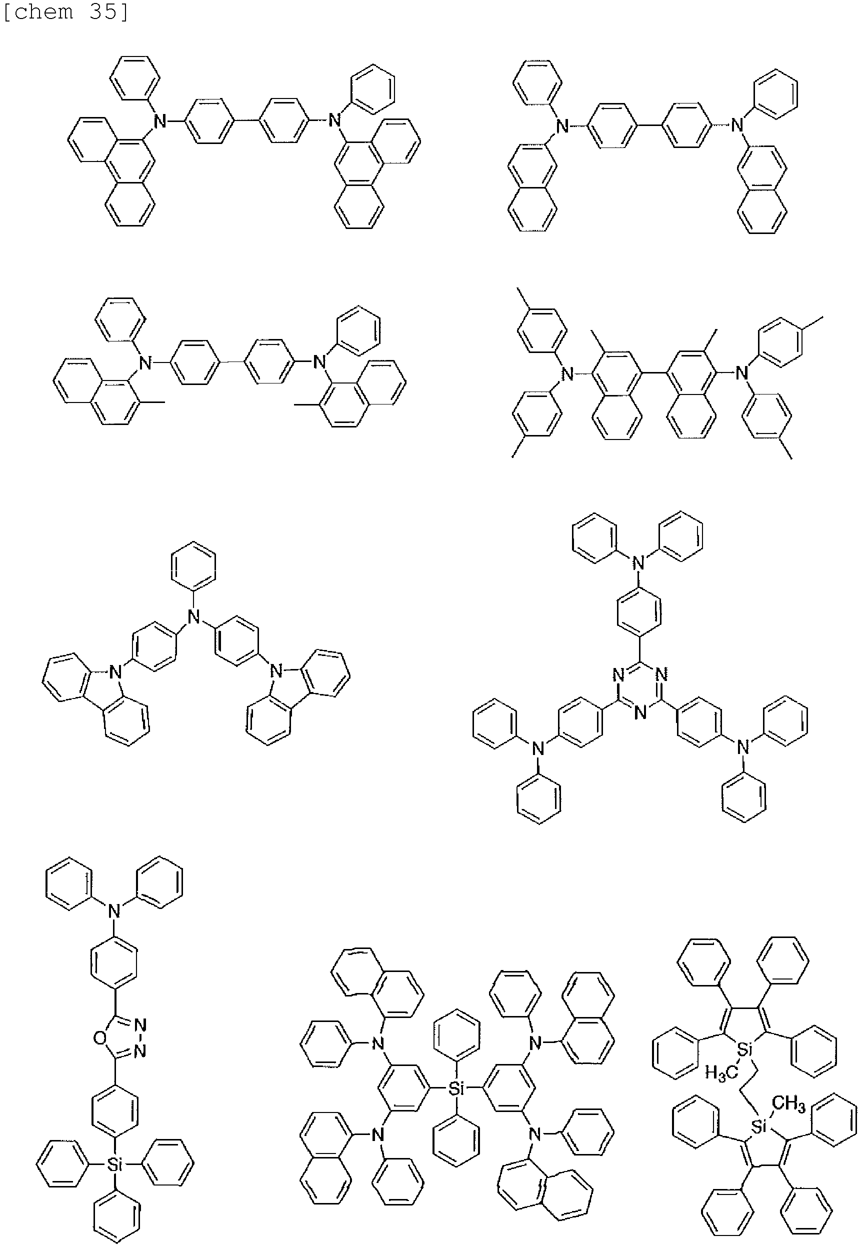

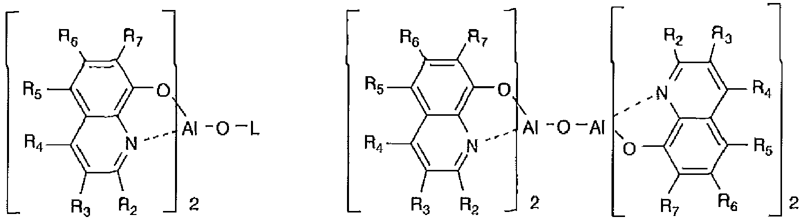

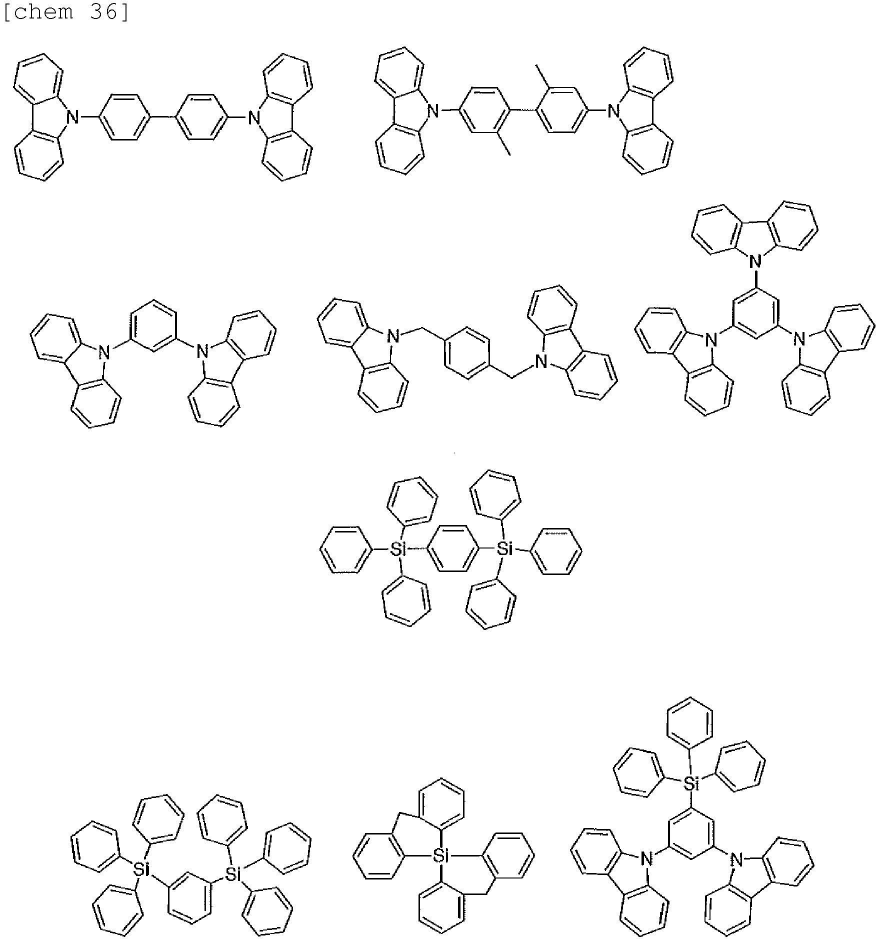

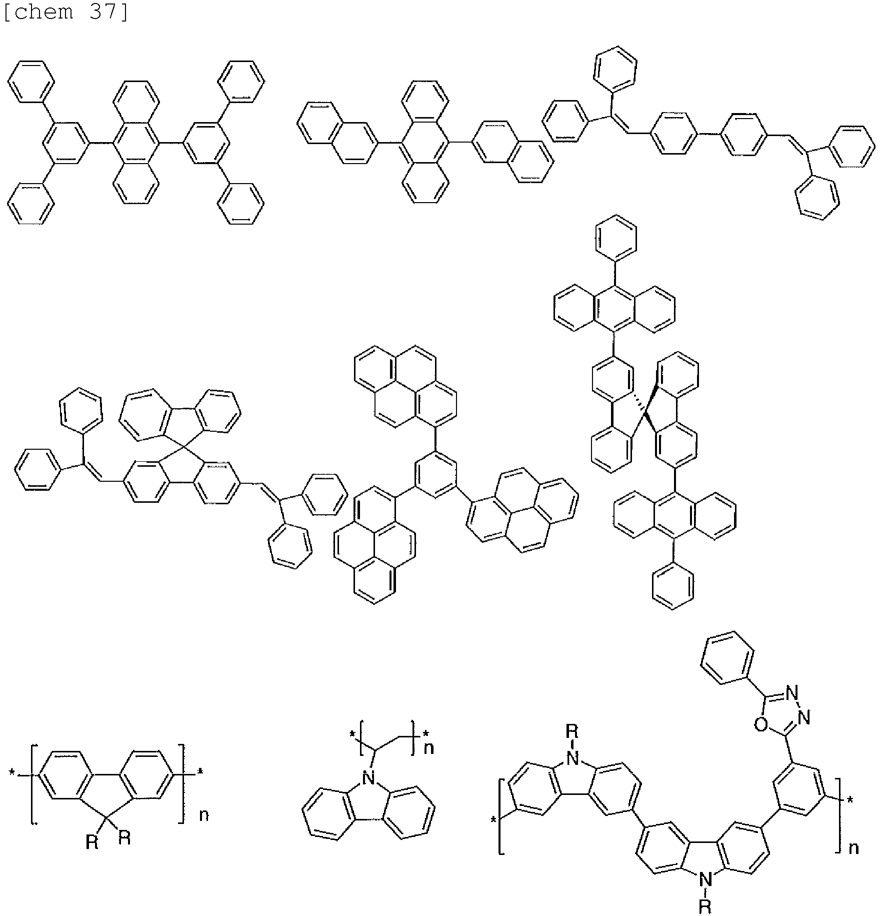

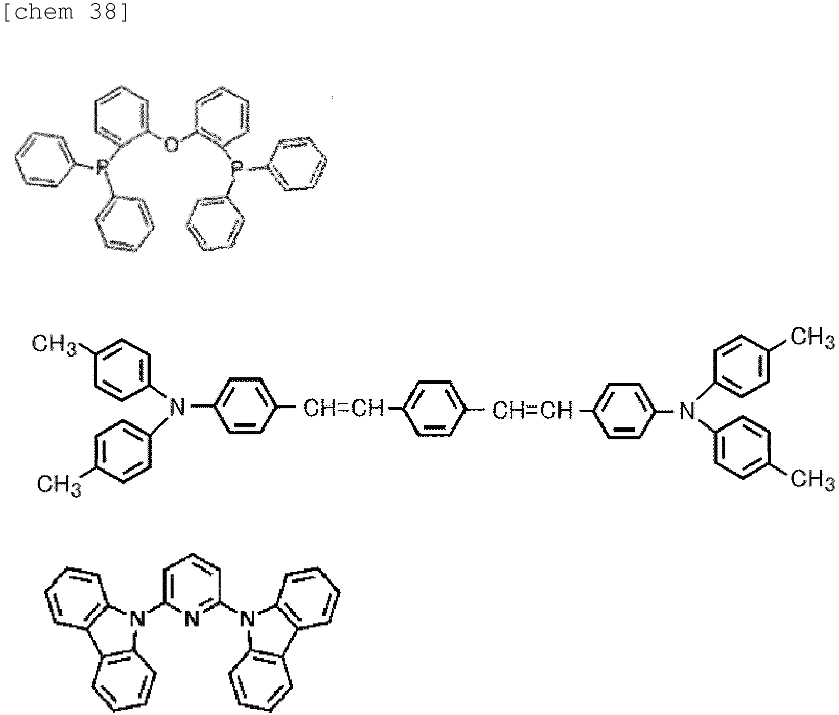

Preferred examples of a compound that may be used as the hole injection material are shown below.

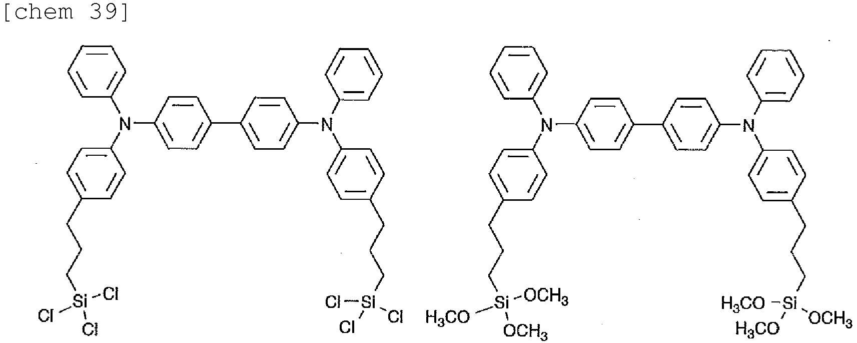

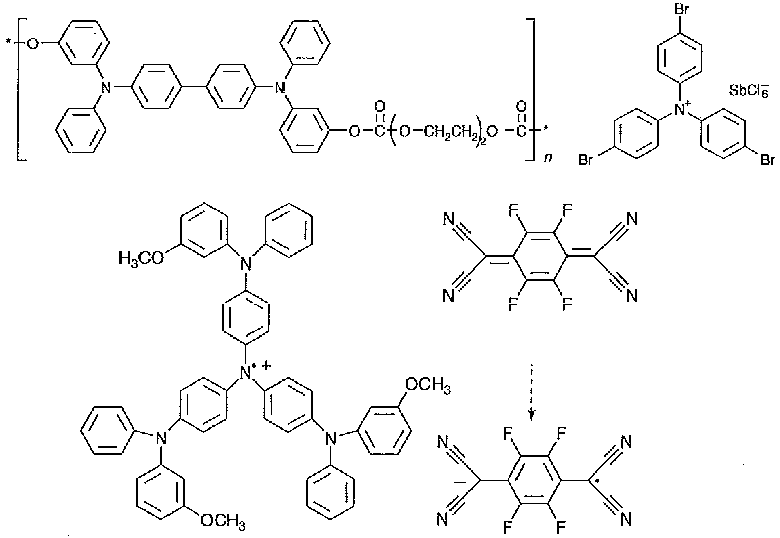

Preferred examples of a compound that may be used as the hole transporting material are shown below.

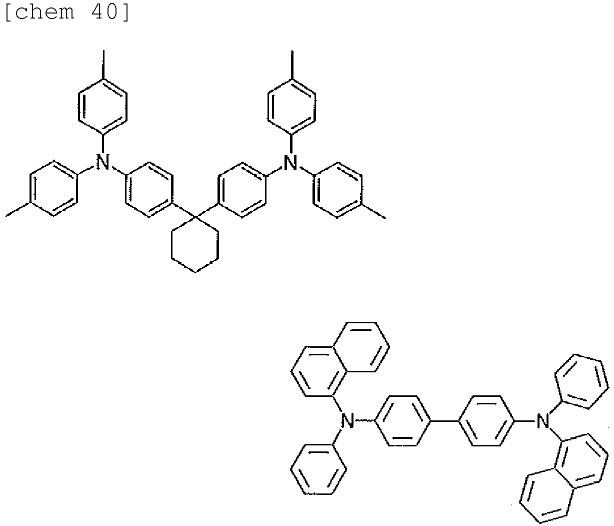

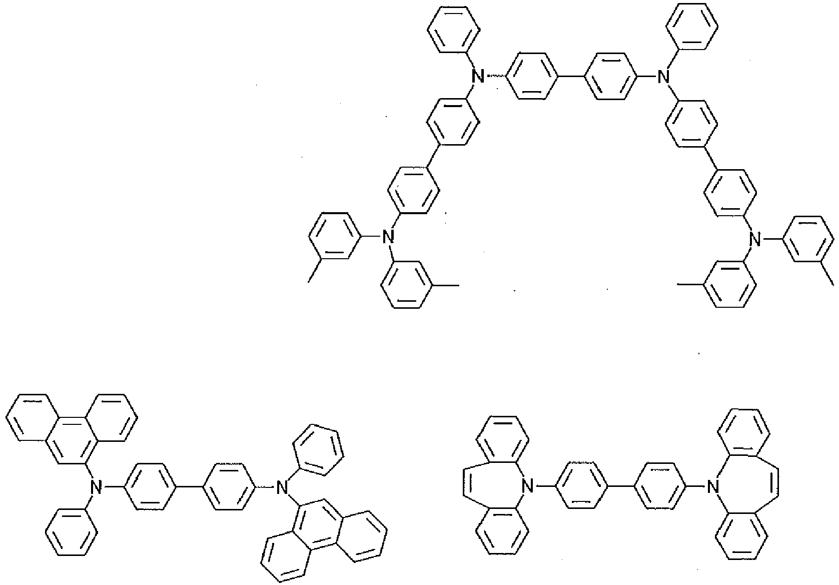

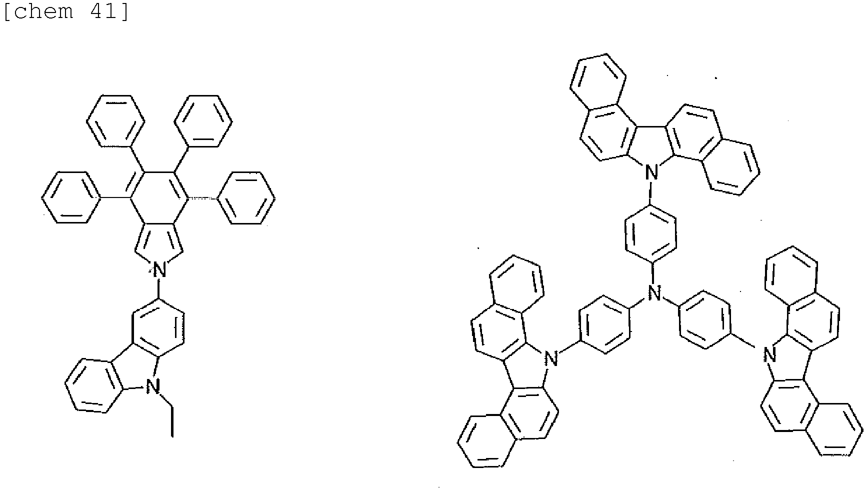

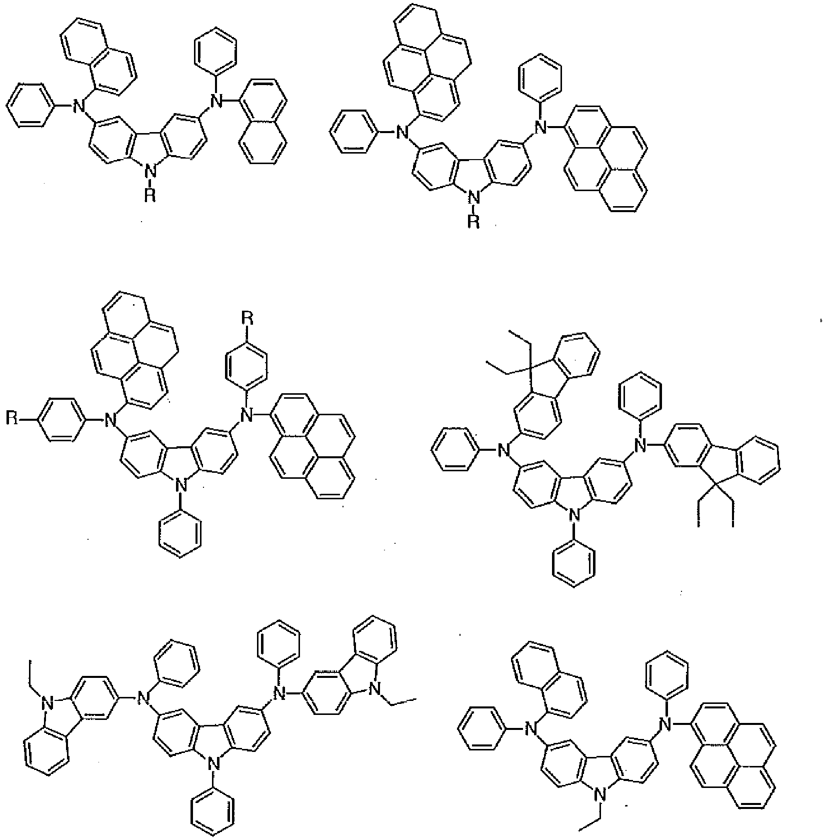

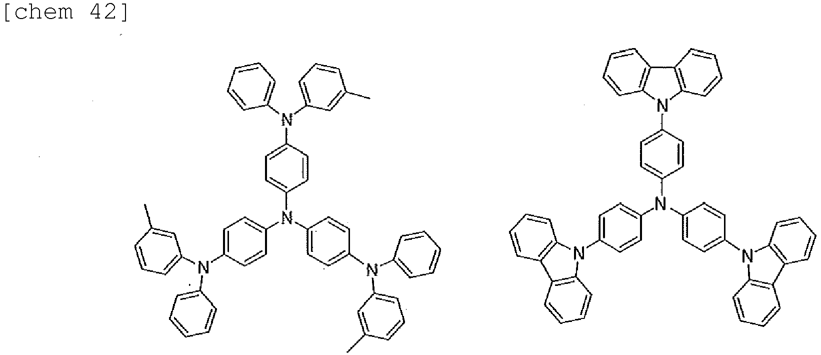

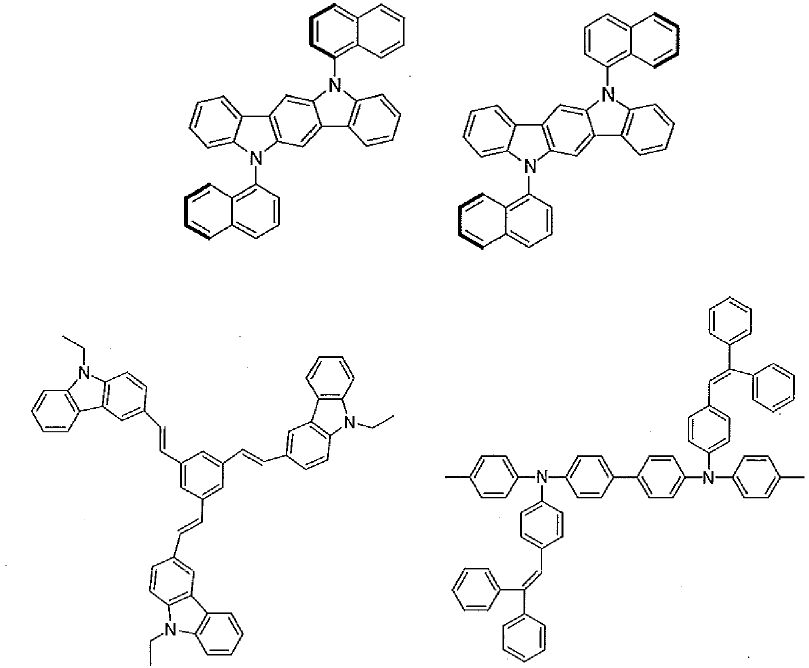

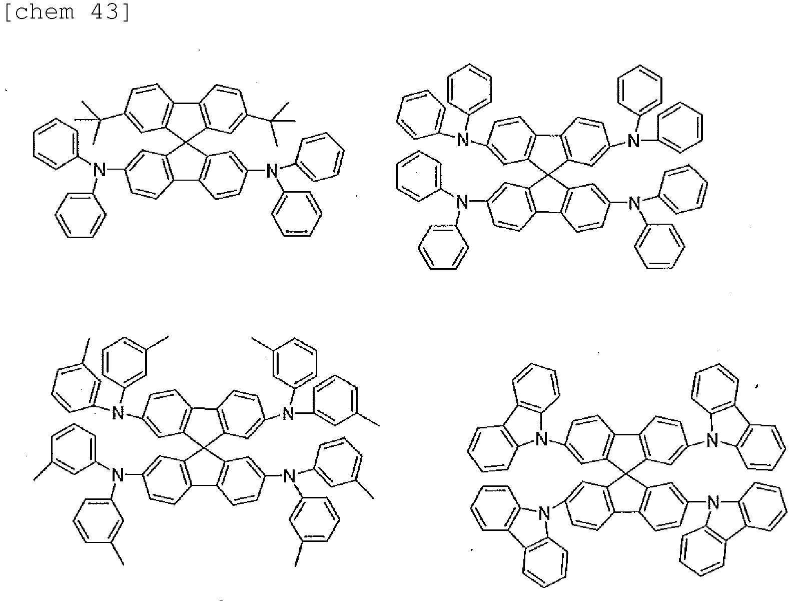

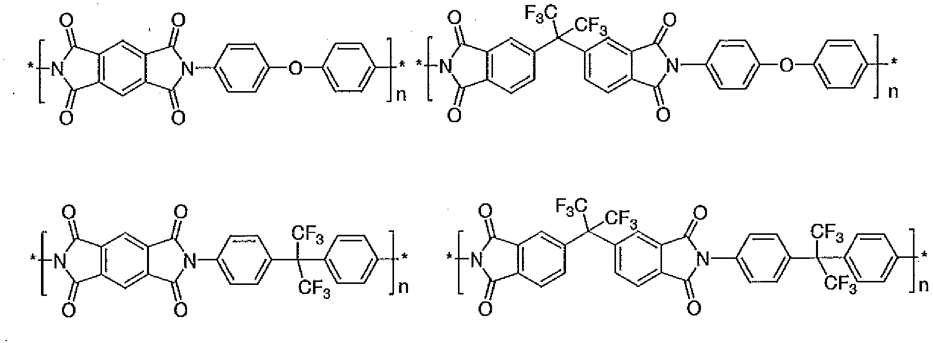

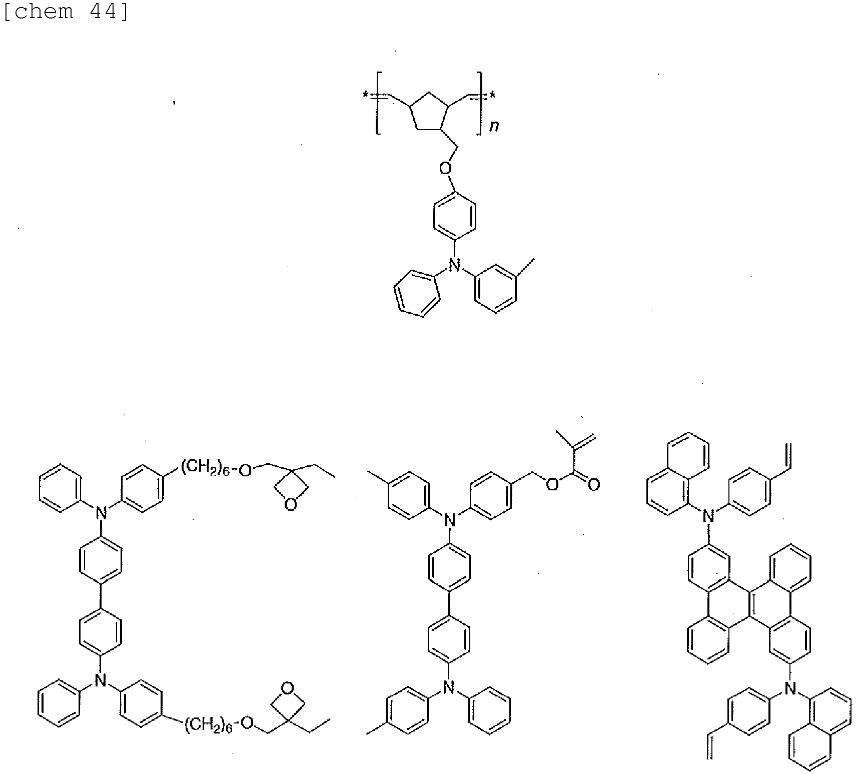

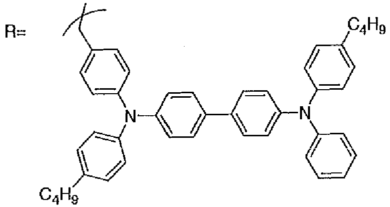

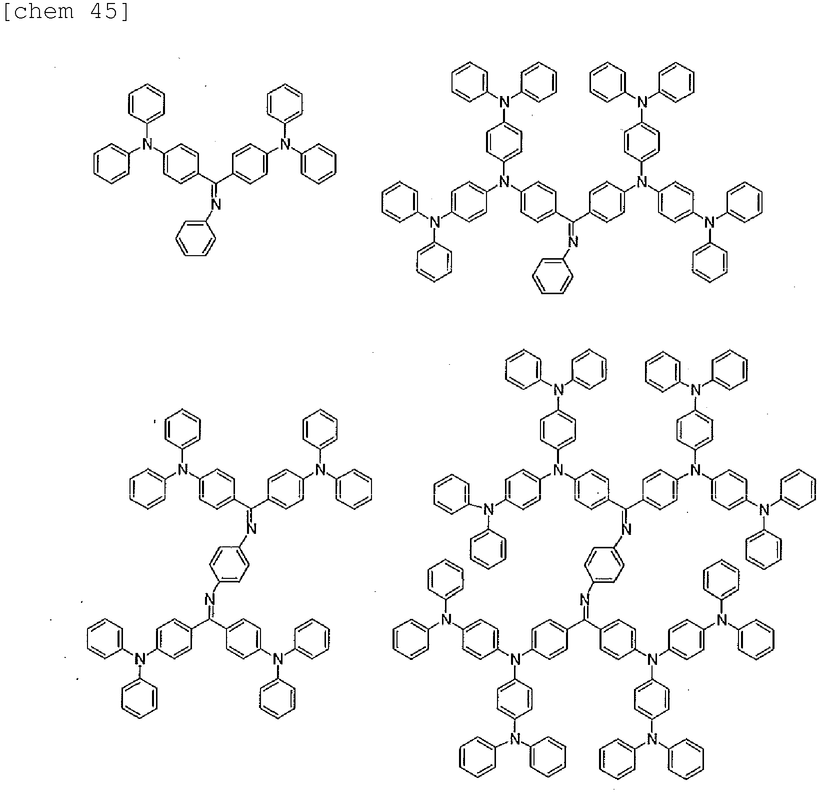

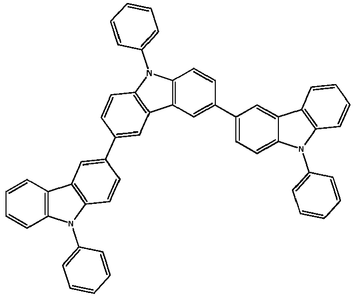

Preferred examples of a compound that may be used as the electron barrier material are shown below.

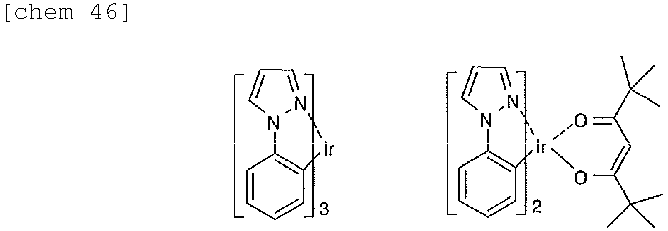

Preferred examples of a compound that may be used as the hole barrier material are shown below.

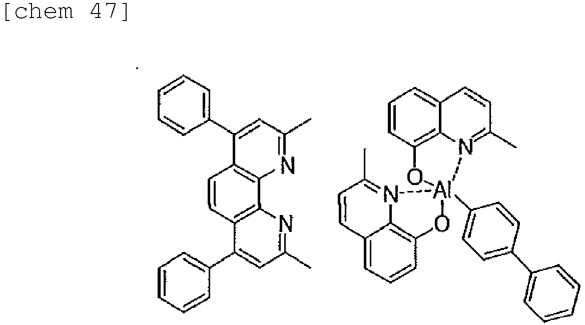

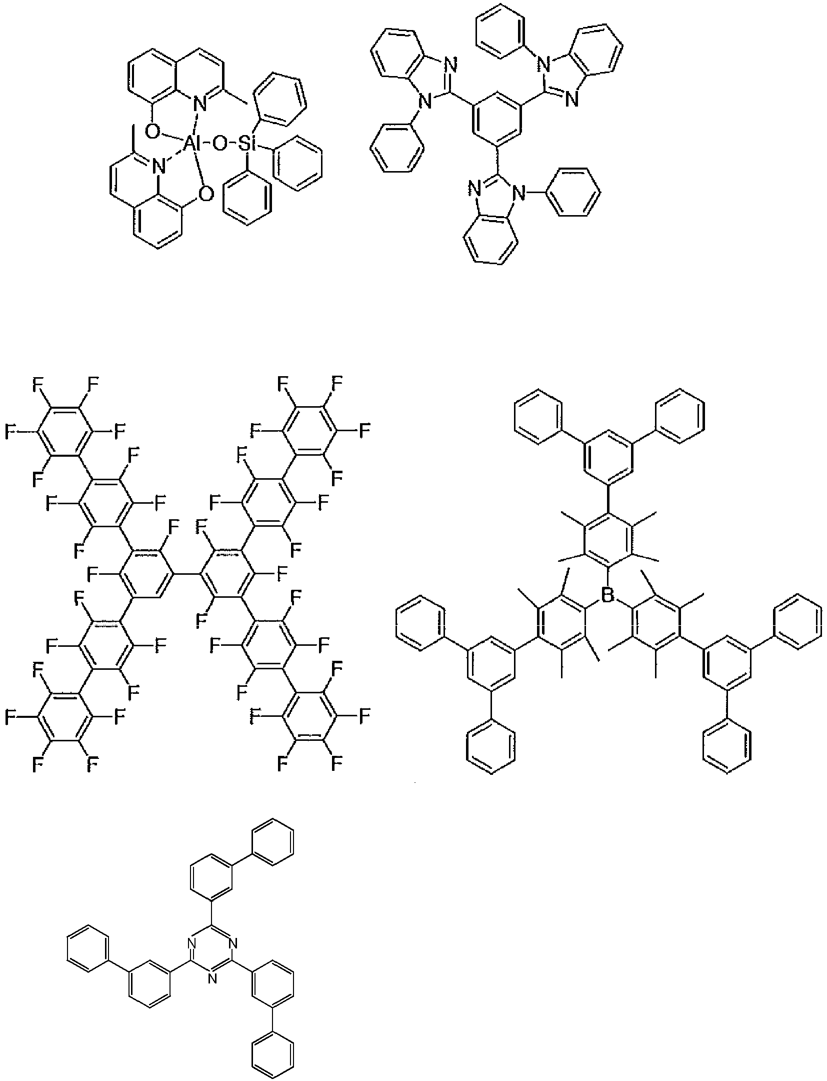

Preferred examples of a compound that may be used as the electron transporting material are shown below.

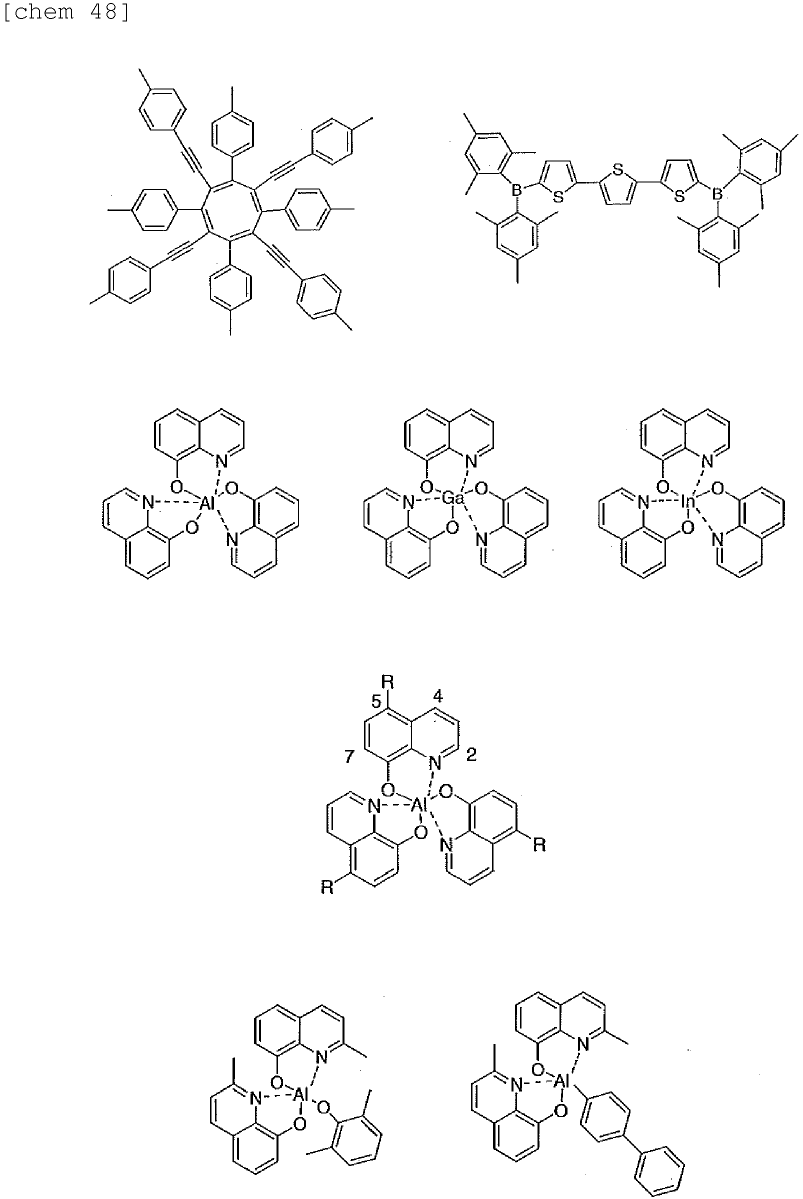

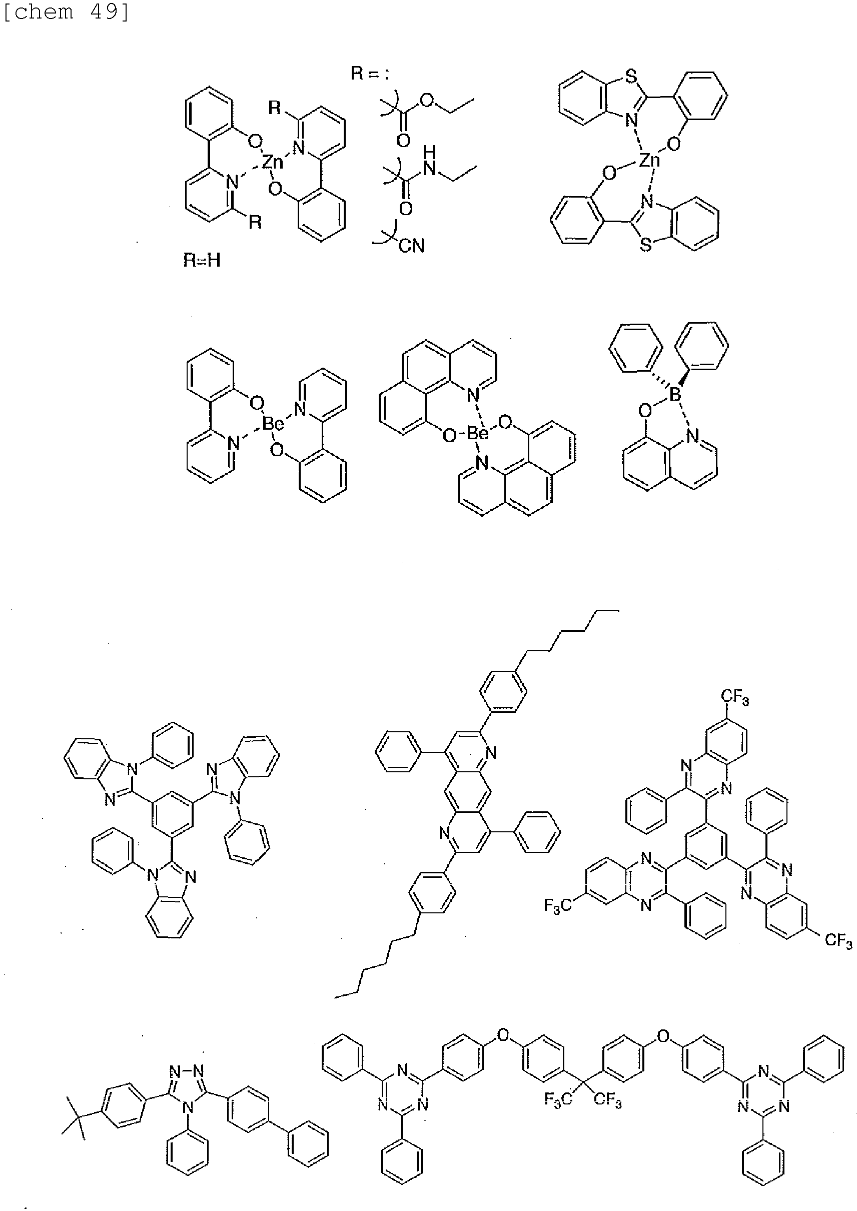

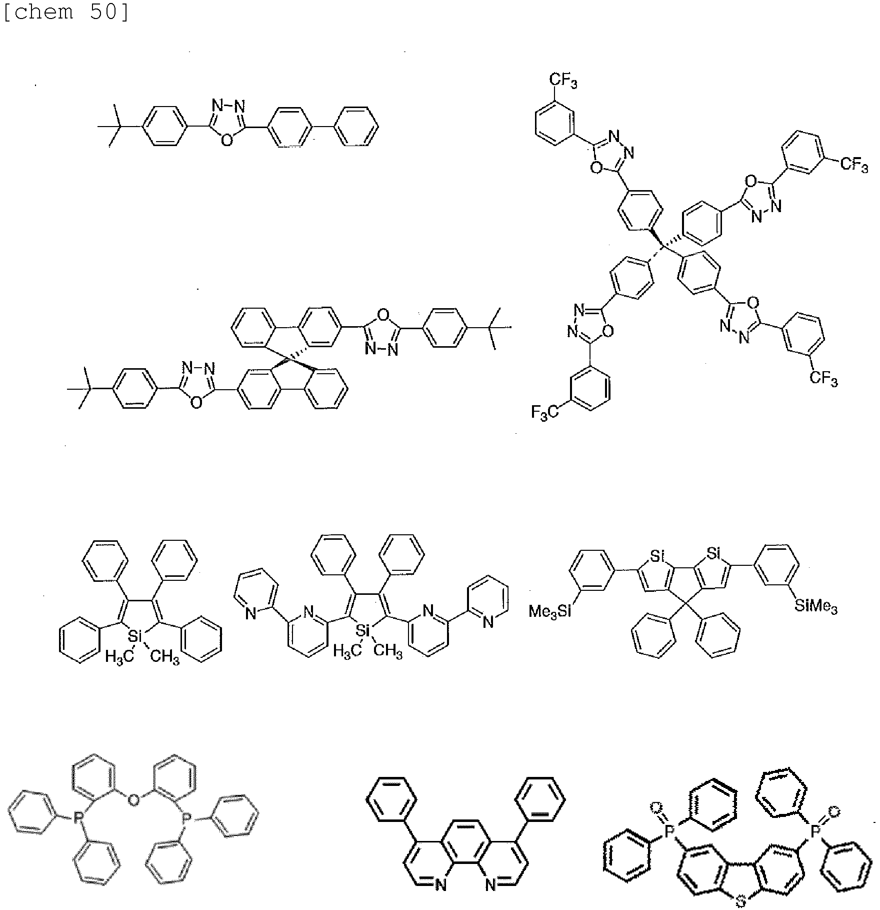

Preferred examples of a compound that may be used as the electron injection material are shown below.

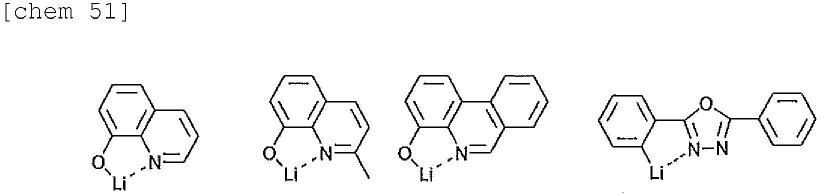

Preferred examples of a compound as a material that may be added are shown below. For example, the compound may be added as a stabilizing material.

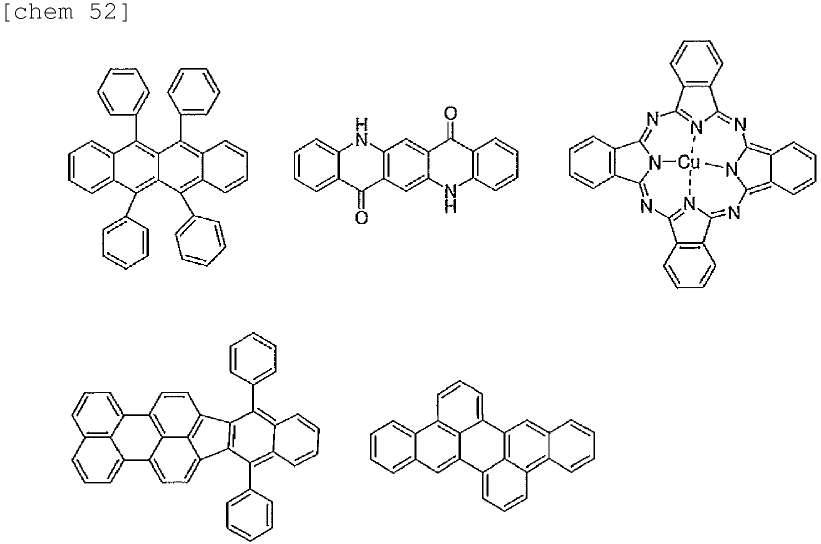

The organic electroluminescent device thus produced by the aforementioned method emits light on application of an electric field between the anode and the cathode of the device. In this case, when the light emission is caused by the excited single energy, light having a wavelength that corresponds to the energy level thereof may be confirmed as fluorescent light and delayed fluorescent light. When the light emission is caused by the excited triplet energy, light having a wavelength that corresponds to the energy level thereof may be confirmed as phosphorescent light. The normal fluorescent light has a shorter light emission lifetime than the delayed fluorescent light, and thus the light emission lifetime may be distinguished between the fluorescent light and the delayed fluorescent light.

The phosphorescent light may substantially not observed with a normal organic compound, such as the compound of the invention, at room temperature since the excited triplet energy is converted to heat or the like due to the instability thereof, and is immediately deactivated with a short lifetime. The excited triplet energy of the normal organic compound may be measured by observing light emission under an extremely low temperature condition.

The organic electroluminescent device of the invention may be applied to any of a single device, a structure with plural devices disposed in an array, and a structure having anodes and cathodes disposed in an X-Y matrix. According to the invention, an organic light emitting device that is largely improved in light emission efficiency may be obtained by adding the compound represented by the general formula (1) in the light emitting layer. The organic light emitting device, such as the organic electroluminescent device, of the invention may be applied to a further wide range of purposes. For example, an organic electroluminescent display apparatus may be produced with the organic electroluminescent device of the invention, and for the details thereof, reference may be made to S. Tokito, C. Adachi and H. Murata, "Yuki EL Display" (Organic EL Display) (Ohmsha, Ltd.). In particular, the organic electroluminescent device of the invention may be applied to organic electroluminescent illumination and backlight which are highly demanded.

### Example

The features of the invention will be described more specifically with reference to synthesis examples and working examples below. The materials, processes, procedures and the like shown below may be appropriately modified unless they deviate from the substance of the invention. Accordingly, the scope of the invention is not construed as being limited to the specific examples shown below.

### Synthesis Example 1

In this synthesis example, compound 1 was synthesized according to the following scheme.

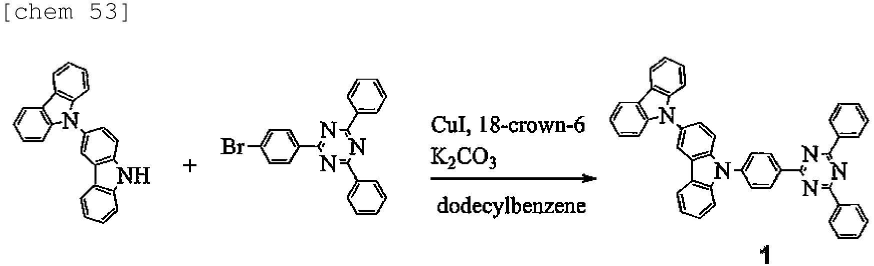

3-Carbazolylcarbazole (0.25 g, 0.75 mmol), 2-bromo-4,6-diphenyl-1,3,5-triazine (0.29 g, 0.75 mmol), copper iodide (0.021 g, 0.11 mmol), 18-crown-6-ether (0.030 g, 0.11 mmol) and potassium carbonate (0.62 g, 4.5 mmol) were added to dodecylbenzene (2.0 mL), and the mixture was heated to 220°C under a nitrogen gas environment for 2 days. After completing the reaction, the product was extracted with dichloromethane, dried over sodium carbonate, and then purified by silica gel column chromatography (developing solvent: hexane/dichloromethane = 80/20), thereby providing the compound 1 (yield amount: 0.25 g (4.0 mmol), yield: 53%).
¹H-NMR (500 MHz, CDCl₃) : **δ** (ppm) 9.08 (d, J = 9.0 Hz, 2H), 8.84 (d, J = 6.5 Hz, 4H), 8.32 (d, J = 2.0 Hz, 1H), 8.20 (d, J = 8.0 Hz, 2H), 8.15 (d, J = 7.5 Hz, 1H), 7.91 (d, J = 8.5 Hz, 2H), 7.75 (d, J = 9.0 Hz, 1H), 7.67-7.59 (m, 8H), 7.55-7.51 (m, 1H), 7.45-7.41 (m, 4H), 7.38-7.35 (m, 1H), 7.33-7.29 (m, 2H)

### Synthesis Example 2

In this synthesis example, compound 2 was synthesized according to the following schemes.

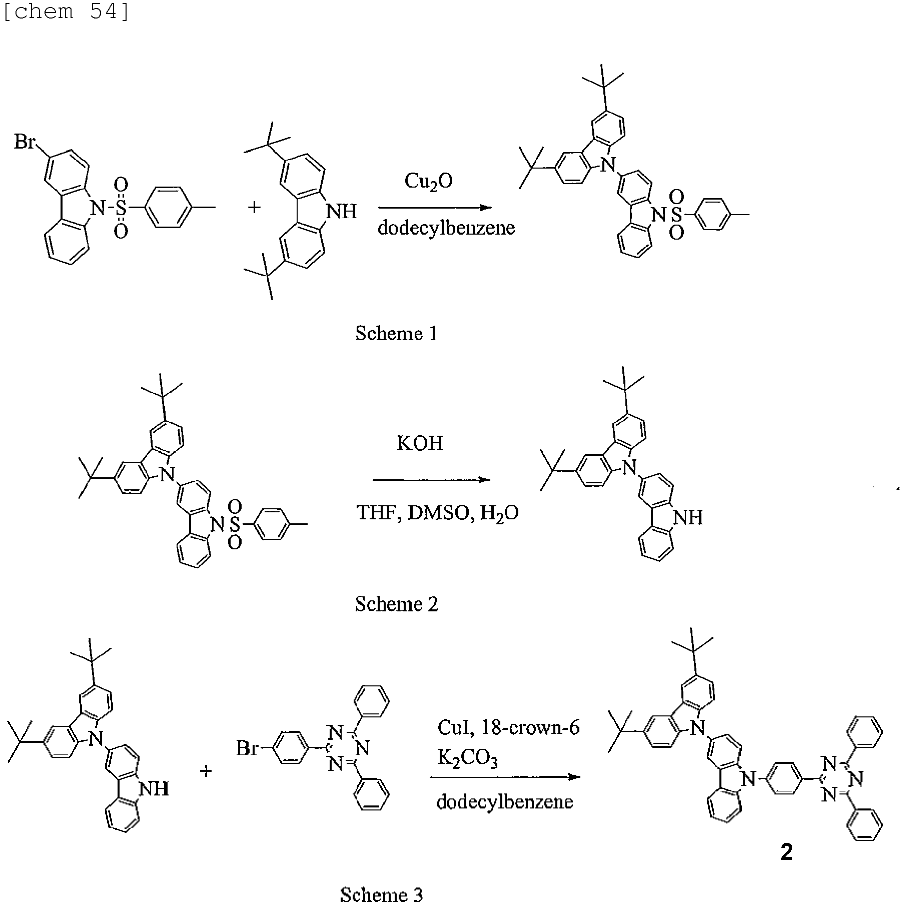

### Scheme 1

### Synthesis of 3,6-Di-tert-butyl-9-(9-tosyl-9H-carbazol-3-yl)-9H-carbazole

3-Bromo-9-tosyl-9H-carbazole (4.47 g, 11.5 mmol), 3,6-di-tert-butyl-9H-carbazole (3.04 g, 10.9 mmol), and copper (I) oxide (3.96 g, 27.6 mmol) were added to dodecylbenzene (2.0 mL), and the mixture was heated to 220°C under a nitrogen gas environment for 20 hours. After completing the reaction, copper (I) oxide was removed by filtration, and then the product was purified by silica gel column chromatography (developing solvent: hexane/dichloromethane = 70/30) (yield amount: 2.21 g (11.5 mmol), yield: 32.2%).
¹H-NMR (500 MHz, CDCl₃): δ (ppm) 8.38 (d, J = 8.5 Hz, 1H), 8.16 (d, J = 1.5 Hz, 1H), 8.07 (d, J = 2.0 Hz, 1H), 7.87 (d, J = 7.5 Hz, 1H), 7.80 (d, J = 8.5 Hz, 2H), 7.66 (dd, J = 2.5 Hz, J = 2.0 Hz, 1H), 7.57-7.53 (m, 1H), 7.46 (dd, J = 2.0 Hz, J = 1.5 Hz, 2H), 7.40-7.37 (m, 1H), 7.32 (d, J = 9.0 Hz, 2H), 7.20 (d, J = 8.0 Hz, 2H), 2.33 (s, 3H), 1.47 (s, 18H)

### Scheme 2

### Synthesis of 3,6-Di-tert-butyl-9-(9H-carbazol-3-yl)-9H-carbazole

3,6-di-tert-butyl-9-(9-tosyl-9H-carbazol-3-yl)-9H-car bazole (2.08 g, 3.47 mmol) and potassium hydroxide (22.2 g, 39.5 mmol) were added to a mixed solvent of tetrahydrofuran (4.6 mL), dimethylsulfoxide (2.3 mL) and water (0.7 mL), and the mixture was stirred at 70°C for 5 hours. After completing the reaction, the reaction mixture was neutralized with sulfuric acid, and the product was extracted with toluene, which was then dried over sodium sulfate. After removing the solvent with an evaporator, the product was purified by recrystallization (isopropanol/methanol = 50/50) (yield amount: 1.17 g (2.51 mmol), yield: 72.5%).
¹H-NMR (500 MHz, DMSO) : δ (ppm) 11.54 (s, 1H), 8.34 (d, J = 2.0 Hz, 1H), 8.30 (d, J = 1.5 Hz, 2H), 8.20 (d, J = 8.0 Hz, 1H), 7.72 (d, J = 8.5 Hz, 1H), 7.56 (d, J = 8.5 Hz, 1H), 7.53 (dd, J = 2.0 Hz, J = 2.0 Hz, 1H) , 7.48-7.44 (m, 3H), 7.25 (d, J = 8.5 Hz, 2H), 7.19-7.16 (m, 1H), 1.43 (s, 18H)

### Scheme 3

### Synthesis of 9'-(4-(4,6-Diphenyl-1,3,5-triazin-2-yl)phenyl)-9'H-(3',6'-d i-tert-butylcarbazolyl) carbazole

Copper iodide (0.01 g, 0.05 mmol), 18-crown-5-ether (0.027 g, 0.10 mmol), potassium carbonate (0.138 g, 1.00 mmol) and dodecylbenzene (0.9 mL) were added to 3-(3,6-di-tert-butylcarbazolyl)carbazole (0.34 g, 0.77 mmol) and 4-bromophenyldiphenyltriazine (0.38 g, 0.97 mmol), and the mixture was reacted at 220°C overnight. After completing the reaction, dichloromethane was added thereto, and the mixture was filtered with Celite. The filtrate was concentrated and then extracted with dichloromethane. The organic layer was dried over magnesium sulfate and concentrated with an evaporator. The product was purified by silica gel column chromatography (developing solvent: cyclohexane/dichloromethane = 70/30), thereby providing the compound 2 (yield amount: 0.3 g, crude yield: 51.9%).
MALDI-TOF-MS m/z = 751 ([M]⁺)
¹H-NMR (500 MHz, DMSO) : δ (ppm) 9.08 (d, J = 8.5 Hz, 2H), 8.82 (d, J = 7.5 Hz, 4H), 8.57 (d, J = 2.0 Hz, 1H), 8.41 (d, J = 7.5 Hz, 1H), 8.33 (d, J = 2.0 Hz, 2H), 8.08 (d, J = 8.5 Hz, 2H), 7.82 (d, 9.0 Hz, 1H), 7.76-7.65 (m, 8H), 7.58-7.55 (m, 1H), 7.50 (dd, J = 1.5 Hz, J = 1.5 Hz, 2H), 7.39-7.36 (m, 1H), 7.33 (d, 9.0 Hz, 2H), 1.43 (s, 18H)

### Synthesis Example 3

In this synthesis example, compound 3 was synthesized according to the following schemes.

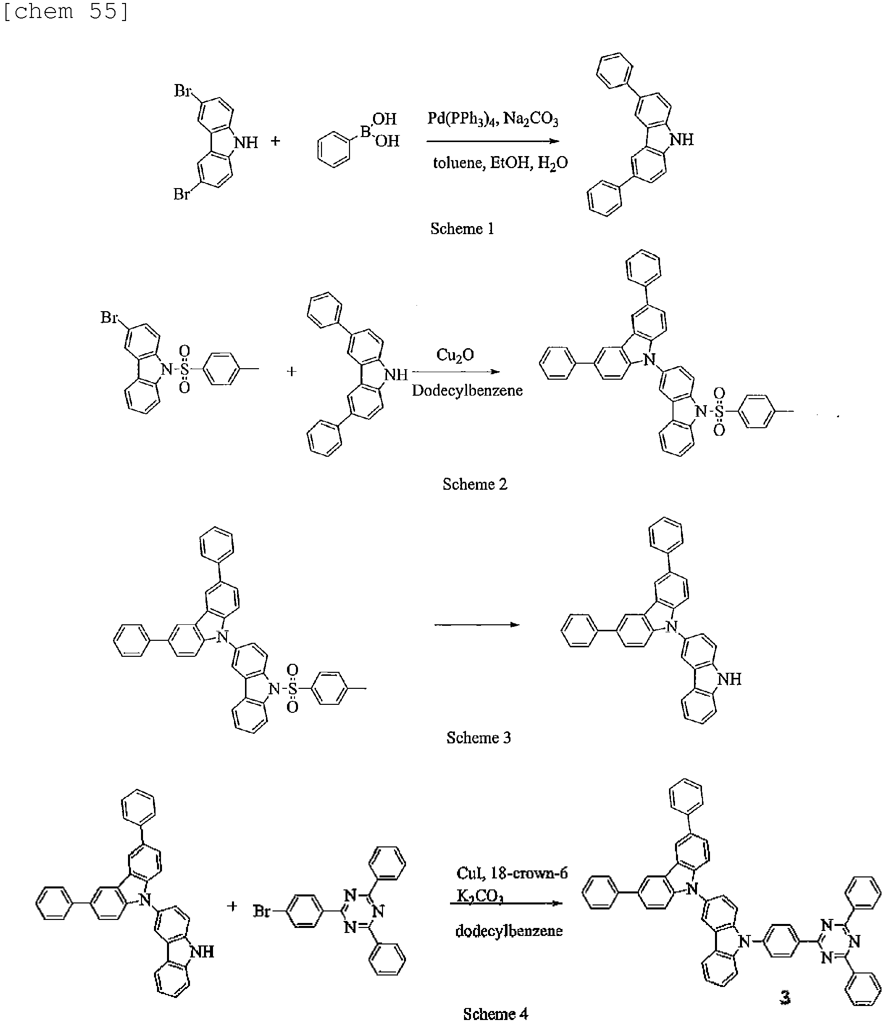

### Scheme 1

### Synthesis of 3,6-Dipehnylcarbazole

Toluene (200 mL), ethanol (100 mL) and water (53 mL) were added to 3, 6-dibromocarbazole (13 g, 40 mmol), phenylboronic acid (11.7 g, 96 mmol) and sodium carbonate (21.9 g, 207 mmol), and after deaeration, the mixture was stirred. Pd (PPH₃)₄ (4.16 g, 3.99 mmol) was further added thereto, and after deaeration, the mixture was reacted at 80°C for 12 hours. After completing the reaction, the product was extracted with dichloromethane. The organic layer was dried over magnesium sulfate and concentrated with an evaporator. The product was purified by silica gel column chromatography (developing solvent: cyclohexane/dichloromethane = 50/50), and recrystallized from hexane (yield amount: 4.32 g, yield: 33.8%).
¹H-NMR (500 MHz, CDCl₃) : δ (ppm) 8.34 (d, J = 1.5 Hz, 2H), 8.11 (s, 1H), 7.72 (dd, J = 1.0 Hz, J = 1.0 Hz, 4H), 7.69 (dd, J = 1.5 Hz, J = 2.0 Hz, 2H), 7.51-7.46 (m, 6H), 7.36-7.33 (m, 2H)

### Scheme 2

### Synthesis of 3,6-Diphenyl-9-tosyl-9H-carbazole

3-Bromo-9-tosyl-9H-carbazole (4.20 g, 13.2 mmol), 3,6-diphenyl-9H-carbazole (4.87 g, 12.5 mmol) and copper(I) oxide (4.54 g, 31.8 mmol) were added to dodecylbenzene (12.3 mL), and the mixture was heated to 220°C under a nitrogen gas environment for 20 hours. After completing the reaction, copper (I) oxide was removed by filtration, and then the filtrate was purified by silica gel column chromatography (developing solvent: hexane/dichloromethane = 50/50) (yield amount: 3.24 g (5.07 mmol), yield: 38.5%).
¹H-NMR (500 MHz, CDCl₃) : δ (ppm) 8.58 (d, J = 8.5 Hz, 1H), 8.43 (d, J = 1.5 Hz, 2H), 8.41 (d, J = 8.5 Hz, 1H), 8.14 (d, J = 2.0 Hz, 1H), 7.92 (d, J = 7.5 Hz, 1H), 7.84 (d, J = 8.5 Hz, 2H), 7.75-7.72 (m, 6H), 7.68 (dd, J = 1.5 Hz, J = 2.0 Hz, 2H), 7.59-7.56 (m, 1H), 7.52-7.45 (m, 5H), 7.42-7.39 (m, 1H), 7.37-7.35 (m, 2H), 2.34 (s, 3H)

### Scheme 3

### Synthesis of 9-(9H-Carbazol-3-yl)-3,6-diphenyl-9H-carbazole

3,6-Diphenyl-9-tosyl-9H-carbazole (3.22 g, 5.03 mmol) and potassium hydroxide (3.22 g, 57.3 mmol) were added to a mixed solvent of tetrahydrofuran (6.7 mL), dimethylsulfoxide (3.3 mL) and water (1.0 mL), and the mixture was stirred at 70°C for 5 hours. After completing the reaction, the reaction mixture was neutralized with sulfuric acid, and the product was extracted with toluene, which was then dried over sodium sulfate. After removing the solvent with an evaporator, the product was purified by recrystallization (chloroform/hexane = 50/50) (yield amount: 1.20 g (2.48 mmol), yield: 49.2%).
¹H-NMR (500 MHz, CDCl₃) : δ (ppm) 8.44 (d, J = 2.0 Hz, 2H), 8.29 (s, 1H), 8.27 (s, 1H), 8.08 (d, J = 8.0 Hz, 1H), 7.75 (d, J = 7.0 Hz, 4H), 7.68 (dd, J = 1.5 Hz, J = 1.5 Hz, 2H), 7.60 (dd, J = 1.5 Hz, J = 2.0 Hz, 1H), 7.50-7.47 (m, 8H), 7.37-7.34 (m, 2H), 7.30-7.27 (m, 1H)

### Scheme 4

### Synthesis of 9'-(4-(4,6-Diphenyl-1,3,5-triazin-2-yl)phenyl)-9'H-(3',6'-d iphenylcarbazolyl)carbazole

Copper iodide (0.010 g, 0.04 mmol), 18-crown-5-ether (0.038 g, 0.14 mmol), potassium carbonate (0.10 g, 0.72 mmol) and dodecylbenzene (0.72 mL) were added to 3-(3,6-diphenylcarbazolyl)carbazole (0.35 g, 0.72 mmol) and 4-bromophenyldiphenyltriazine (0.31 g, 0.80 mmol), and the mixture was reacted at 220°C overnight. After completing the reaction, dichloromethane was added thereto, and the mixture was filtered with Celite. The filtrate was concentrated and then extracted with dichloromethane. The organic layer was dried over magnesium sulfate and concentrated with an evaporator. The product was purified by silica gel column chromatography (developing solvent: hexane/dichloromethane = 70/30), thereby providing the compound 3 (yield amount: 0.10 g, crude yield: 17.5%).
¹H-NMR (500 MHz, DMSO) : δ (ppm) 9.09 (d, J = 8.5 Hz, 2H), 8.82 (d, J = 7.0 Hz, 4H), 8.78 (d, J = 1.5 Hz, 2H), 8.68 (d, J = 2.0 Hz, 1H), 8.43 (d, J = 8.0 Hz, 1H), 8.09 (d, J = 8.5 Hz, 2H), 7.85 (d, 7.5 Hz, 5H), 7.81 (dd, J = 1.5 Hz, J = 1.5 Hz, 2H), 7.76-7.67 (m, 9H), 7.53-7.49 (m, 6H), 7.42-7.35 (m, 3H) MALDI-TOF-MS: m/z = 791 ([M]⁺)

### Synthesis Example 4

In this synthesis example, compound 4 was synthesized according to the following schemes.

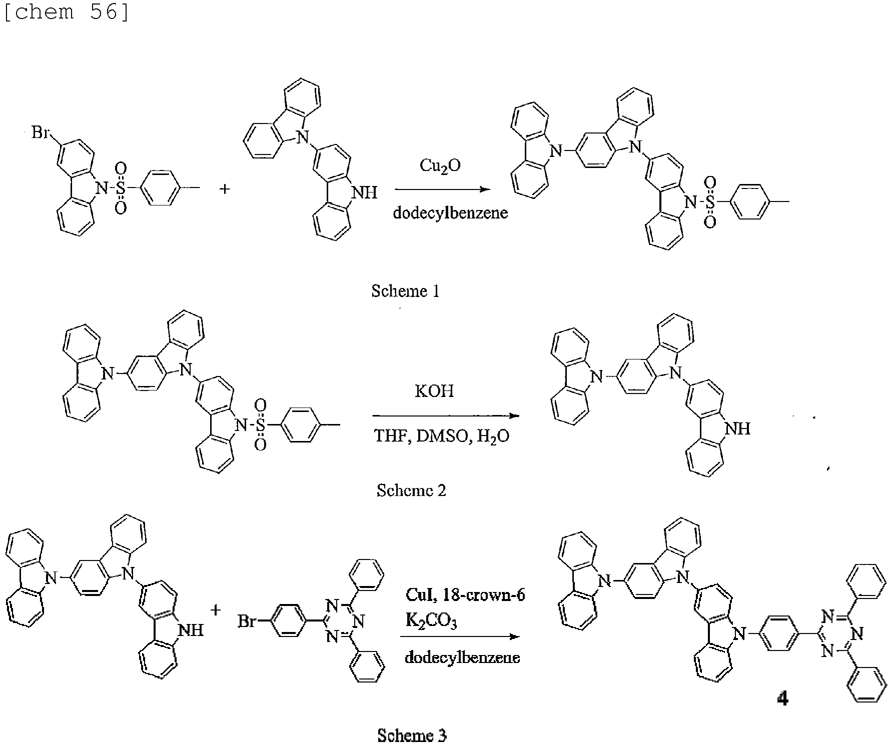

### Scheme 1

### Synthesis of 9-(9-(9-Tosyl-9H-carbazolyl-3-yl)-9H-carbazolyl-3-yl)-9H-ca rbazole

3-Bromo-9-tosyl-9H-carbazole (1.70 g, 4.36 mmol), 3-carbazolylcarbazole (1.52 g, 4.59 mmol) and copper(I) oxide (2.22 g, 15.5 mmol) were added to dodecylbenzene (4.3 mL), and the mixture was reacted at 220°C under an inert atmosphere for 20 hours. After completing the reaction, copper(I) oxide was removed by filtration, and the product was purified by silica gel column chromatography (developing solvent: hexane/dichloromethane = 50/50) (yield amount: 1.14 g (1.75 mmol), yield: 38.1%).
¹H-NMR (500 MHz, CDCl₃): 8.60 (d, J = 9.0 Hz, 1H), 8.41 (d, J = 8.5 Hz, 1H), 8.31 (d, J = 1.0 Hz, 1H), 8.20-8.17 (m, 3H), 8.14 (d, J = 8.0 Hz, 1H), 7.94 (d, J = 7.5 Hz, 1H), 7.84 (d, J = 8.5 Hz, 2H), 7.75 (dd, J = 2.0 Hz, J = 2.0 Hz, 1H),7.58-7.54 (m, 3H), 7.48-7.40 (m, 7H), 7.35-7.29 (m, 3H), 7.22 (d, J = 8.0 Hz, 2H), 2.34 (s, 3H)

### Scheme 2

### Synthesis of 9-(9-(9H-carbazolyl-3-yl)-9H-carbazlyl-3-yl)-9H-carbazole

9-(9-(9-Tosyl-9H-carbazolyl-3-yl)-9H-carbazolyl-3-yl) -9H-carbazole (1.05 g, 2.16 mmol) and potassium hydroxide (1.38 g, 24.6 mmol) were added to a mixed solvent of tetrahydrofuran (2.9 mL), dimethylsulfoxide (1.4 mL) and water (0.4 mL), and the mixture was stirred at 70°C for 5 hours. After completing the reaction, the reaction mixture was neutralized with sulfuric acid, and the product was extracted with toluene, which was then dried over sodium sulfate. After removing the solvent with an evaporator, the product was purified by recrystallization (chloroform/hexane = 20/80) (yield amount: 0.62 g (1.3 mmol), yield: 59%).
¹H-NMR (500 MHz, DMSO) : 11.62 (s, 1H), 8.56 (d, J = 2.0 Hz, 1H), 8.50 (d, J = 2.0 Hz, 1H), 8.37 (d, J = 7.5 Hz, 1H), 8.29 (d, J = 8.0 Hz, 2H), 8.25 (d, J = 8.0 Hz, 1H) 7.80 (d, J = 8.5 Hz, 1H), 7.66 (dd, J = 2.0 Hz, J = 2.0 Hz, 1H), 7.61-7.7.59 (m, 3H), 7.50-7.43 (m, 4H), 7.41-7.38 (m, 3H), 7.32-7.29 (m, 3H), 7.22-7.19 (m, 1H)

### Scheme 3

### Synthesis of 9'-(4-(4,6-Diphenyl-1,3,5-triazin-2-yl)phenyl)-9'H-3,9':3', 9''-tercarbazole

Dodecylbenzene (0.6 mL) was added to 3-(3-(3-carbazolyl)carbazolyl)carbazole (0.26 g, 0.5 mmol), 3,6-dibromo-9-tosylcarbazole (0.23 g, 0.6 mmol), copper iodide (0.005 g, 0.02 mmol), 18-crown-6-ether (0.025 g, 0.09 mmol) and potassium carbonate (0.09 g, 0.65 mmol), and the mixture was reacted at 220°C overnight. After completing the reaction, dichloromethane was added thereto, and the mixture was filtered with Celite. After concentrating, the organic layer was dried over magnesium sulfate and concentrated with an evaporator. The product was purified by silica gel column chromatography (developing solvent: hexane/dichloromethane = 70/30), thereby providing the compound 4 (yield amount: 0.11 g, crude yield: 23.1%) .
¹H-NMR (500 MHz, CDCl₃): 9.10 (d, J = 8.5 Hz, 2H), 8.84 (d, J = 7.0 Hz, 4H), 8.41 (d, J = 2.0 Hz, 1H), 8.33 (d, J = 1.5 Hz, 1H), 8.20-8.15 (m, 4H), 7.92 (d, J = 8.5 Hz, 2H), 7.80 (d, J = 8.5 Hz, 1H), 7.69-7.55 (m, 12H), 7.48 (s, 2H), 7.43 (s, 4H), 7.35-7.29 (m, 3H)
MALDI-TOF-MS: m/z = 803 ([M-1]⁺)

### Synthesis Example 5

In this synthesis example, compound 28 was synthesized according to the following schemes.

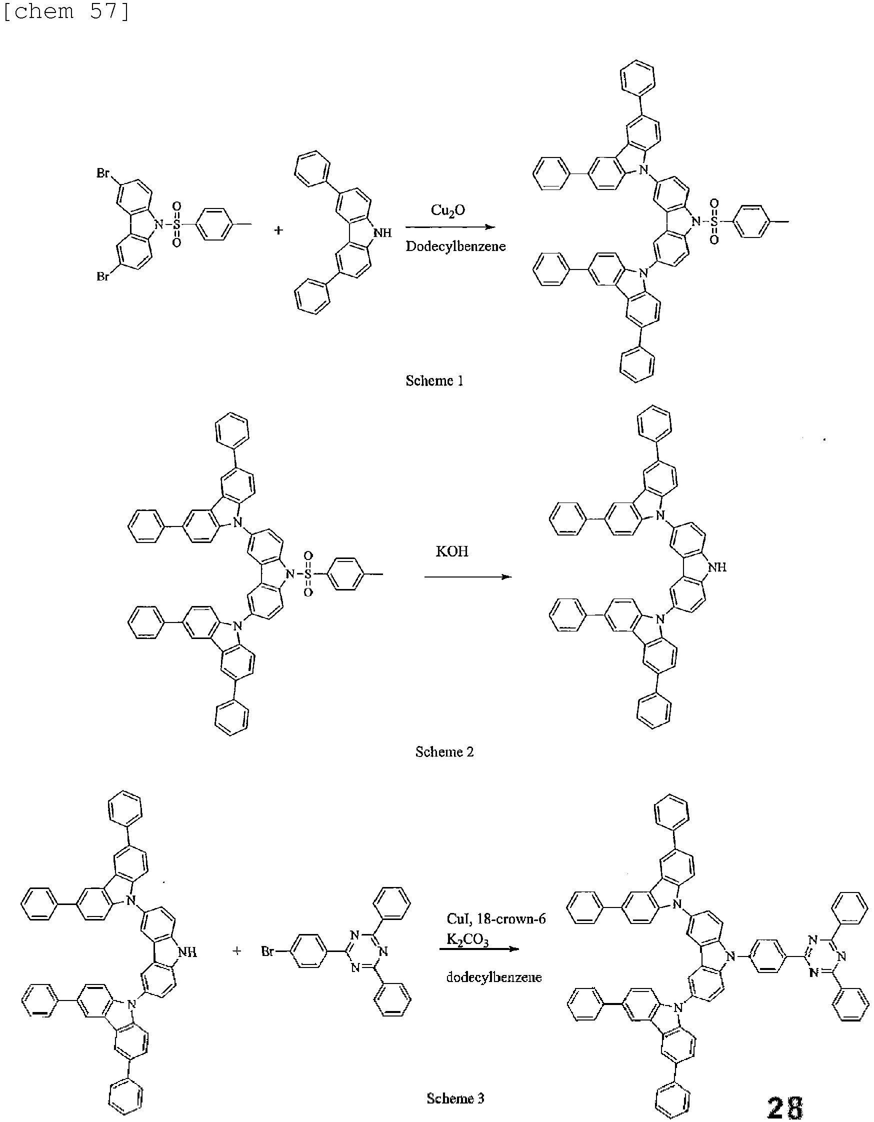

### Scheme 1

### Synthesis of 9-Tosyl-3,3"',6,6" -tetraphenyl-3',6'-dicarbazolylcarbazole

Dodecylbenzene (3.5 mL) was added to 3,6-dibromo-9-tosylcarbazole (1.92 g, 4.0 mmol), 3,6-diphenylcarbazole (2.58 g, 8.08 mmol) and copper oxide (1.39 g, 9.7 mmol), and the mixture was reacted at 220°C overnight. After completing the reaction, the product was extracted with dichloromethane. The organic layer was dried over magnesium sulfate and concentrated with an evaporator. The product was purified by silica gel column chromatography (developing solvent: hexane/dichloromethane = 70/30) (yield amount: 1.42 g, yield: 19.0%).
¹H-NMR (500 MHz, CDCl₃) : δ (ppm) 8.66 (d, J = 9.0 Hz, 2H), 8.40 (d, J = 2.0 Hz, 4H), 8.18 (d, J = 2.0 Hz, 2H), 7.97 (d, J = 8.50 Hz, 2H), 7.82 (dd, J = 2.0 Hz, J = 2.0 Hz, 2H), 7.72 (d, J = 7.0 Hz, 8H), 7.67 (dd, J = 2.0 Hz, J = 2.0 Hz, 4H), 7.48-7.46 (m, 12H), 7.36-7.33 (m, 6H), 2.42 (s, 3H)

### Scheme 2

### Synthesis of 3,3", 6, 6"-Tetraphenyl-3', 6'-dicarbazolylcarbazole

THF (2.3 mL), DMSO (1.4 mL) and water (0.3 mL) were added to 9-tosyl-3, 3", 6, 6"-tetraphenyl-3', 6'-dicarbazolylcarbazole (0.25 g, 0.25 mmol) and potassium hydroxide (0.14 g, 2.5 mmol), and the mixture was refluxed under heating for 4 hours. After completing the reaction, the reaction mixture was neutralized with 1N HCl. The product was extracted with water and dichloromethane, and the organic layer was dried over sodium sulfate and concentrated with an evaporator. The product was recrystallized from hexane and dichloromethane (yield amount: 0.16 g, yield: 80.0%).
¹H-NMR (500 MHz, CDCl₃): δ (ppm) 8.52 (s, 1H), 8.42 (d, J = 2.0 Hz, 4H), 8.30 (d, J = 2.0 Hz, 2H), 7.77 (d, J = 8.5 Hz, 2H), 7.73 (d, J = 8.0 Hz, 8H), 7.70 (d, J = 2.0 Hz, 1H), 7.67 (dd, J = 2.0 Hz, J = 1.5 Hz, 5H), 7.49-7.46 (m, 12H), 7.35-7.33 (m, 4H)

### Scheme 3

### Synthesis of 9'-(4-(4,6-diphenyl-1,3,5-triazin-2-yl)phenyl)-9'H-(3',6'-d iphenylcarbazolyl) carbazole

Dodecylbenzene (1.2 mL) was added to 3, 3", 6, 6"-tetraphenyl-3', 6'-dicarbazolylcarbazole (0.97 g, 1.2 mmol), diphenyltriazine (0.47 g, 1.21 mmol), copper iodide (0.004 g, 0.02 mmol), 18-crown-6-ether (0.035 g, 0.13 mmol) and potassium carbonate (0.17 g, 1.43 mmol), and the mixture was reacted at 220°C for 2 days. After completing the reaction, the product was extracted with dichloromethane. The organic layer was dried over magnesium sulfate and concentrated with an evaporator. The product was purified by silica gel column chromatography (developing solvent: hexane/dichloromethane = 70/30), thereby providing the compound 28 (yield amount: 0.20 g, yield: 15.3%).
¹H-NMR (500 MHz, d6-DMSO) : δ (ppm) 9.16 (d, J = 9.0 Hz, 2H), 8.87-8.83 (m, 6H), 8.77 (d, J = 8.5 Hz, 4H), 8.23 (d, J = 8.5 Hz, 2H), 7.96 (d, J = 8.5 Hz, 2H), 7.86-7.80 (m, 16H), 7.76-7.71 (m, 4H), 7.55 (d, J = 8.5 Hz, 4H), 7.52-7.49 (m, 8H), 7.37-7.34 (m, 4H)

### Synthesis Example 6

In this synthesis example, compound 29 was synthesized according to the following schemes.

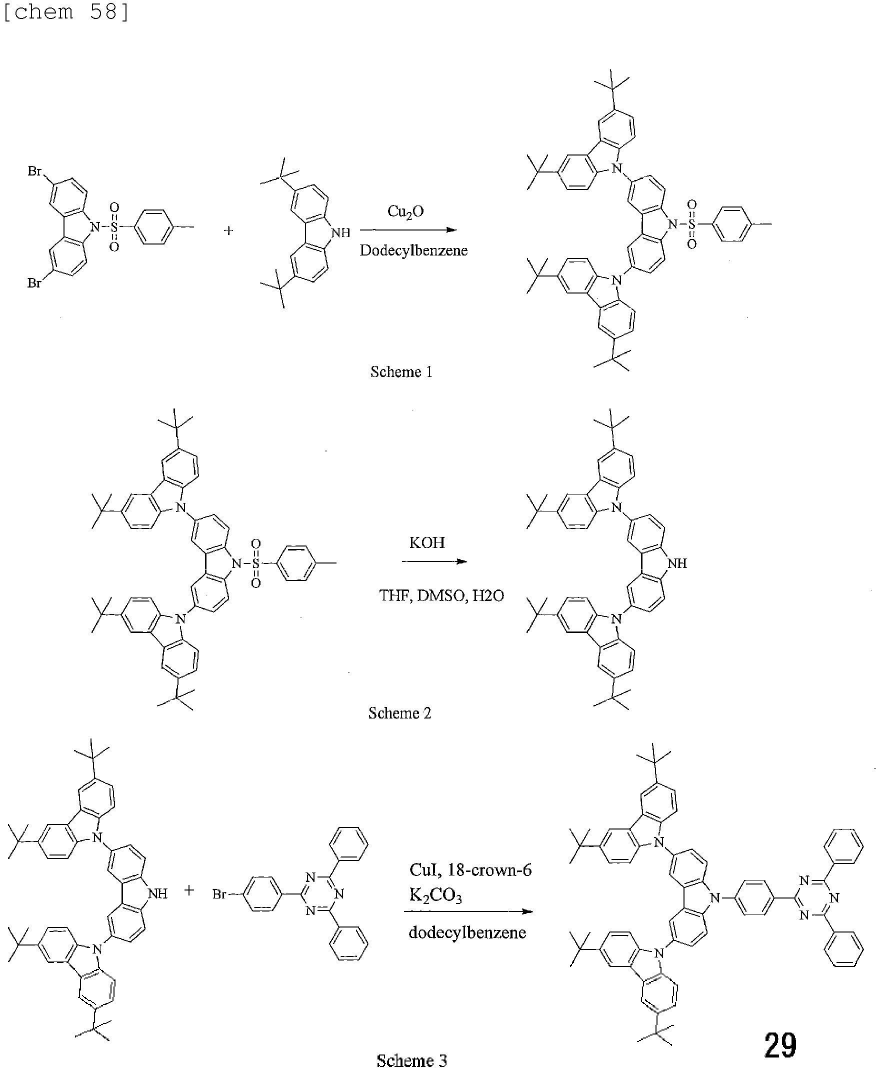

### Scheme 1

### Synthesis of 3,3'',6,6" '-Tetra-tert-butyl-9'-tosyl-3',6'-dicarbazolylcar bazole

Dodecylbenzene (4 mL) and NMP (1 mL) were added to 3,6-di-tert-butylcarbazole (2.69 g, 9.59 mmol), 3,6-dibromocarbazole (2.40 g, 5.00 mmol) and copper oxide (1.71 g, 12.3 mmol), and the mixture was reacted at 220°C overnight. After completing the reaction, copper oxide was removed by filtration, and the filtrate was concentrated with an evaporator. The product was purified by silica gel column chromatography (developing solvent: cyclohexane/dichloromethane = 50/50), thereby providing a matter that was considered to be the target product in an amount of approximately 0.44 g (yield: approximately 10%). Copper (II) oxide (0.85 g, 5.94 mmol) and dodecylbenzene (2 mL) were added to the product containing the mono-substituted compound and the raw materials, and the mixture was heated under refluxing at 220°C overnight (yield amount: 1.02 g, yield: 23.3%) .
¹H-NMR (500 MHz, CDCl₃) : δ (ppm) 8.56 (d, J = 8.5 Hz, 2H), 8.14 (d, J = 1.5 Hz, 4H), 8.05 (d, J = 2.0 Hz, 2H), 7.91 (d, J = 8.5 Hz, 2H), 7.73 (dd, J = 2.0 Hz, J = 2.0 Hz, 2H), 7.44 (dd, J = 2.0 Hz, J = 2.0 Hz, 4H), 7.33-7.2.8 (m, 6H), 2.39 (s, 3H), 1.45 (s, 36H)

### Scheme 2

### Synthesis of (3,6-Di(3,6-di-tert-butylcarbazolyl))carbazole

THF (49 mL), DMSO (24 mL) and water (5 mL) were added to 3,3'',6,6" -tetra-tert-butyl-9'-tosyl-3',6'-dicarbazolylcar bazole (4.78 g, 5.45 mmol) and potassium hydroxide (2.41 g, 56 mmol), and the mixture was stirred under heating for 3 hours. After completing the reaction, the reaction mixture was neutralized with 1N HCl and extracted with dichloromethane. The organic layer was dried over magnesium sulfate and concentrated with an evaporator. The product was purified by silica gel column chromatography (developing solvent: cyclohexane/dichloromethane = 70/30) and recrystallized from hexane (yield amount: 2.95 g, yield: 75%).
¹H-NMR (500 MHz, CDCl₃) : δ (ppm) 8.42 (s, 1H), 8.17-8.15 (m, 6H), 7.69-7.67 (m, 1H), 7.61 (d, J = 8. 5 Hz, 2H), 7.65-7.55 (m, 1H), 7.47-7.43 (m, 4H), 7.32-7.30 (m, 4H), 1.48-1.46 (m, 36H)

### Scheme 3

### Synthesis of 9'-(4-(4,6-diphenyl-1,3,5-triazin-2-yl)phenyl)-9'H-(3',6'-d i-tert-butylcarbazolyl) carbazole

Dodecylbenzene (0.6 mL) was added to (3,6-di(3,6-di-tert-butylcarbazolyl))carbazole (0.51 g, 0.7 mmol), 3,6-dibromo-9-tosylcarbazole (0.31 g, 0.81 mmol), copper iodide (0.007 g, 0.04 mmol), 18-crown-6-ether (0.02 g, 0.08 mmol) and potassium carbonate (0.09 g, 0.65 mmol), and the mixture was refluxed under heating overnight. After completing the reaction, the mixture was filtered with Celite. After concentrating, the organic layer was dried over magnesium sulfate and concentrated with an evaporator. The product was purified by silica gel column chromatography (developing solvent: hexane/dichloromethane = 50/50), thereby providing the compound 29 (yield amount: 0.16 g, crude yield: 22.5%).
MALDI-TOF-MS: m/z = 1030 ([M+1]⁺)

### Example 1

In this example, toluene solutions of the compounds 1 to 4 and 27 to 29 (concentration: 10⁻⁵ mol/L) were prepared and irradiated with light at 300 K under nitrogen bubbling, and thus light emission was observed. The absorption wavelength, the light emission wavelength and the quantum yield are shown in Table 1. The toluene solutions exhibited delayed fluorescent light.

**Table 1**

| Compound | Absorption wavelength (nm) | Light emission wavelength (nm) | Quantum yield (%) |
|---|---|---|---|
| Compound 1 | 360 | 450 | 71.6 |
| Compound 2 | 370 | 461 | 56.9 |
| Compound 3 | 357 | 451 | 68.1 |
| Compound 4 | 360 | 442 | 82.5 |
| Compound 27 | 361 | 448 | 57.1 |
| Compound 28 | 360 | 450 | 74.2 |
| Compound 29 | 370 | 460 | 68.5 |

### Example 2

In this example, organic photoluminescent devices were produced and evaluated for the characteristics.

The compound 1 and DPEPO were vapor-deposited on a silicon substrate from separate vapor deposition sources under condition of a vacuum degree of 5.0 × 10⁻⁴ Pa to form a thin film having a thickness of 100 nm having a concentration of the compound 1 of 6% by weight at a rate of 0.3 nm/sec, and thus an organic photoluminescent device was produced.

Organic photoluminescent devices were produced in the same manner except that the compounds 2 to 4 and 27 to 29 were used instead of the compound 1.

The organic photoluminescent devices thus produced were irradiated with light having a wavelength of 337 nm with a N₂ laser, and the light emission spectrum from the thin film on irradiation was evaluated for the characteristics at 300 K.

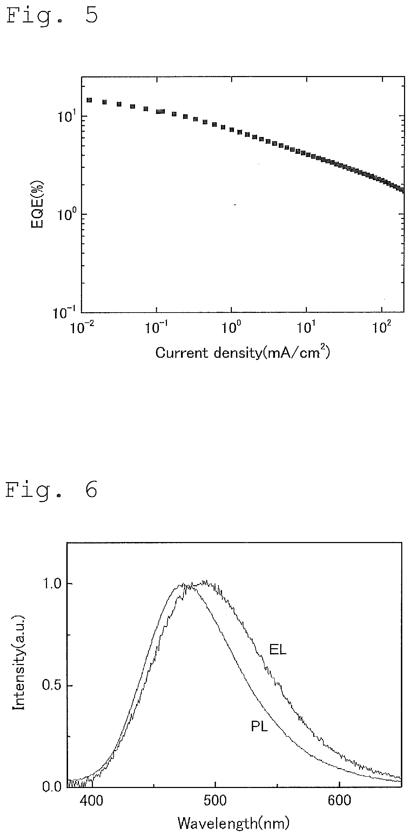

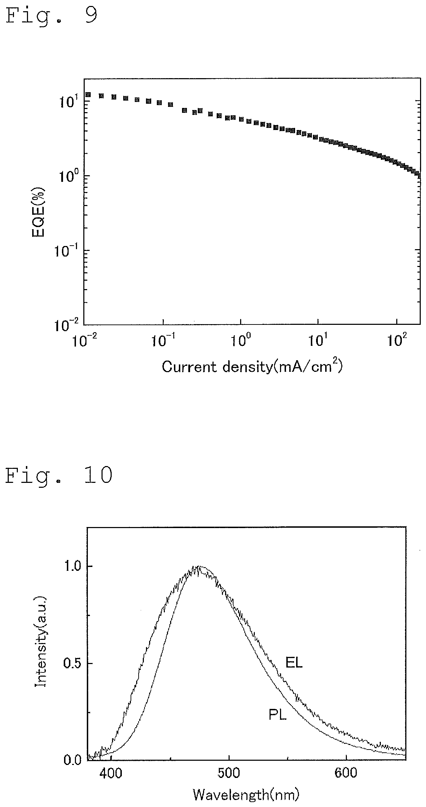

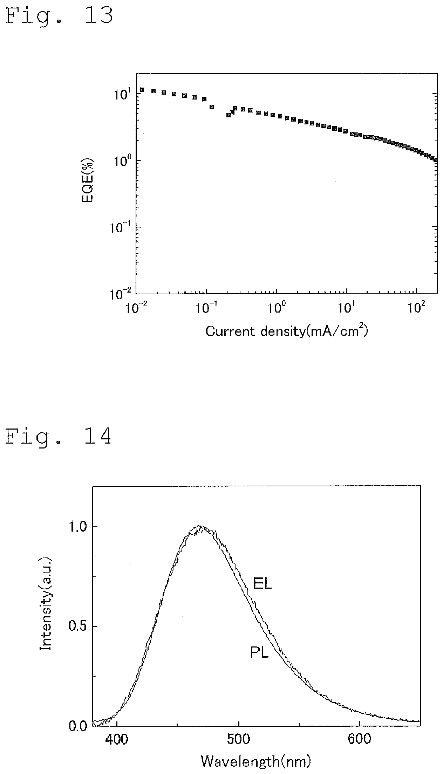

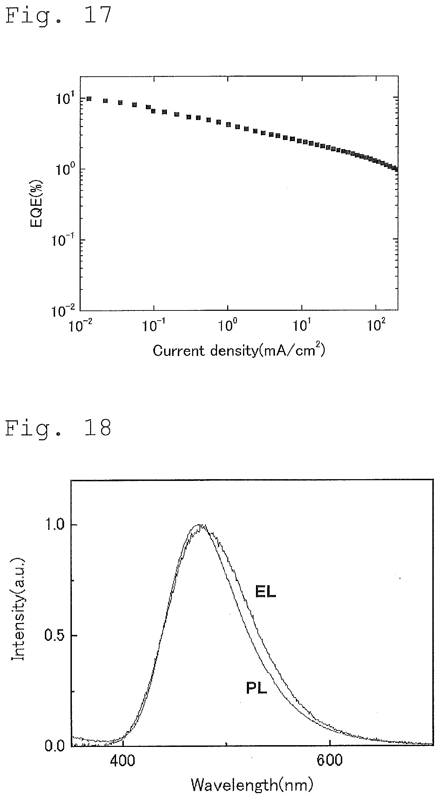

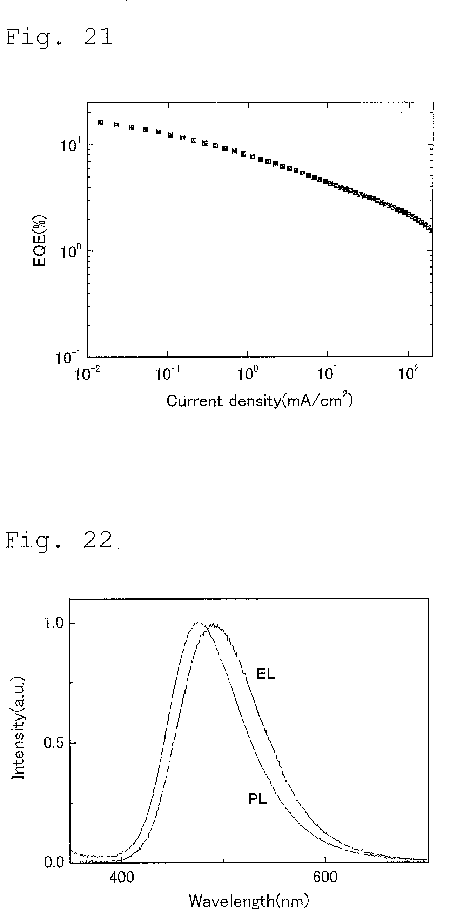

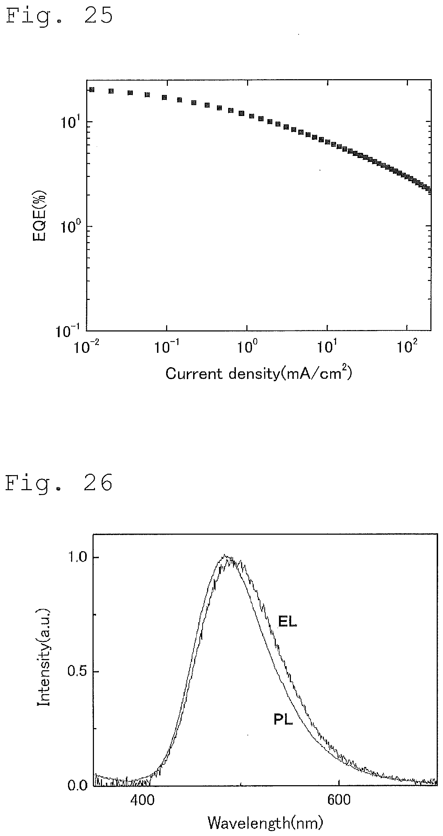

The light emission spectrum of the organic photoluminescent device using the compound 1 is shown in Fig. 2, the light emission spectrum of the organic photoluminescent device using the compound 2 is shown in Fig. 6, the light emission spectrum of the organic photoluminescent device using the compound 3 is shown in Fig. 10, and the light emission spectrum of the organic photoluminescent device using the compound 4 is shown in Fig. 14. The light emission spectrum of the organic photoluminescent device using the compound 27 is shown in Fig. 18, the light emission spectrum of the organic photoluminescent device using the compound 28 is shown in Fig. 22, and the light emission spectrum of the organic photoluminescent device using the compound 29 is shown in Fig. 26.

### Example 3

In this example, organic electroluminescent devices were produced and evaluated for the characteristics.

Thin films were laminated on a glass substrate having formed thereon an anode formed of indium tin oxide (ITO) having a thickness of 100 nm, by a vacuum vapor deposition method at a vacuum degree of 5.0 × 10⁻⁴ Pa. Firstly, α-NPD was formed to a thickness of 35 nm on ITO, and thereon CBP was formed to a thickness of 10 nm. Further thereon, the compound 1 and DPEPO were vapor-deposited from separate vapor deposition sources to form a thin film having a thickness of 15 nm having a concentration of the compound 1 of 6% by weight. Further thereon, TPBi was formed to a thickness of 40 nm, further lithium fluoride (LiF) was vacuum vapor-deposited to a thickness of 0.8 nm, and then aluminum (Al) was vapor-deposited to a thickness of 80 nm to form a cathode, thereby producing an organic electroluminescent device.

Organic electroluminescent devices were produced in the same manner except that the compounds 2 to 4 and 27 to 29 were used instead of the compound 1.

The organic electroluminescent devices thus produced were measured with Semiconductor Parameter Analyzer (E5273A, produced by Agilent Technologies, Inc.), Optical Power Meter (1930C, produced by Newport Corporation), Fiber Optic Spectrometer (USB2000, produced by Ocean Optics, Inc.) and Streak Camera (Model C4334, produced by Hamamatsu Photonics K.K.).

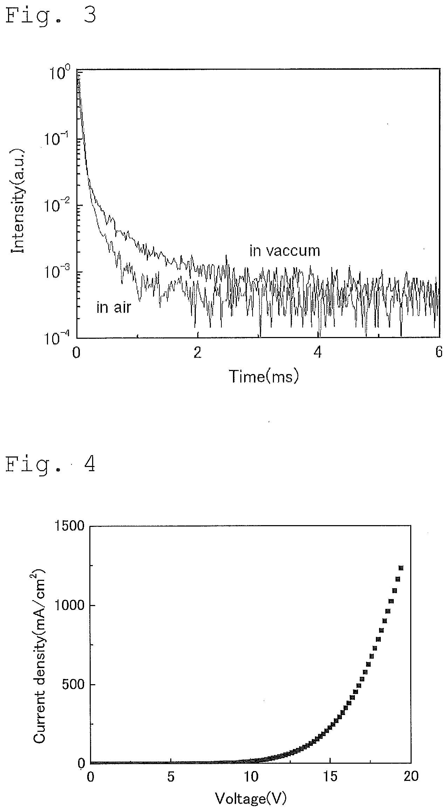

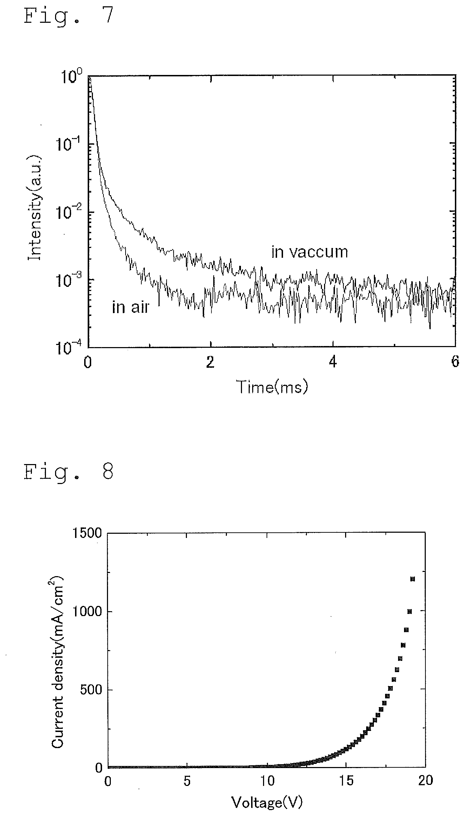

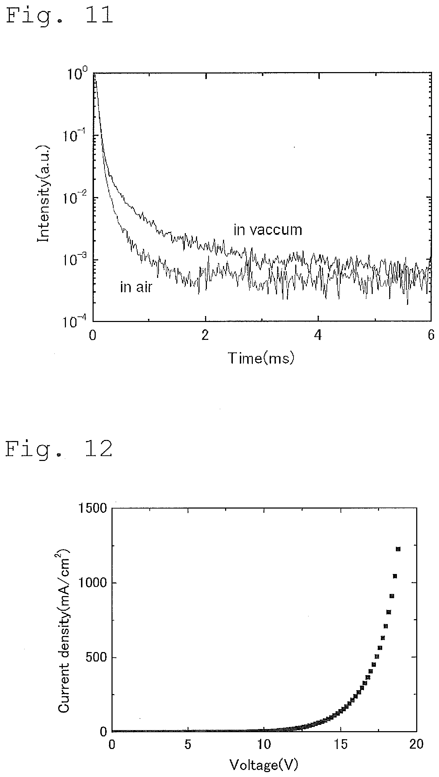

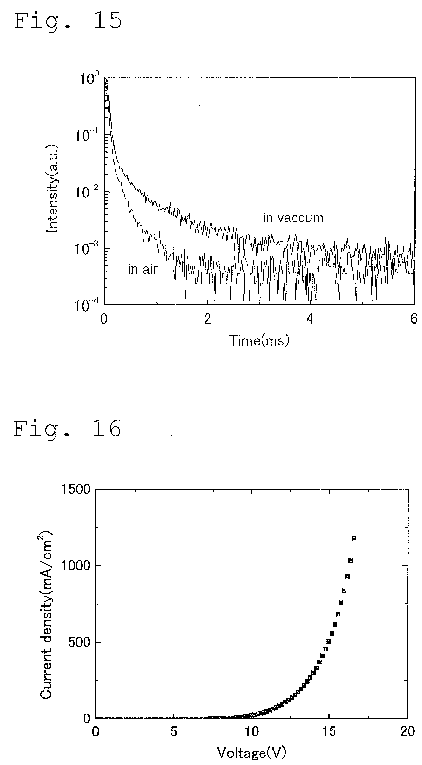

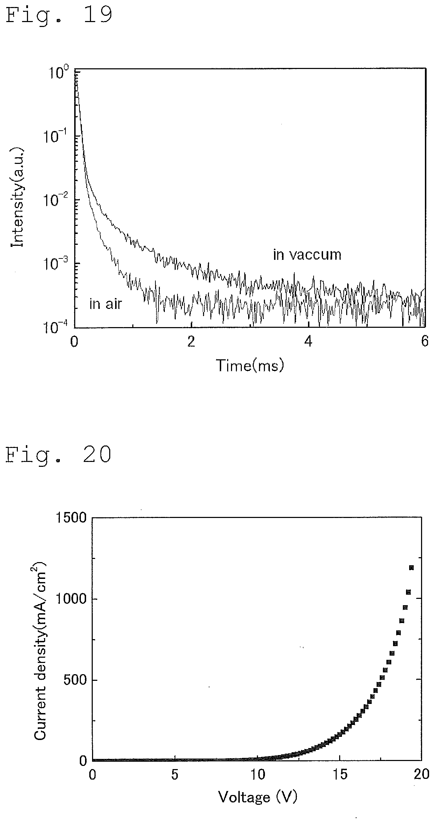

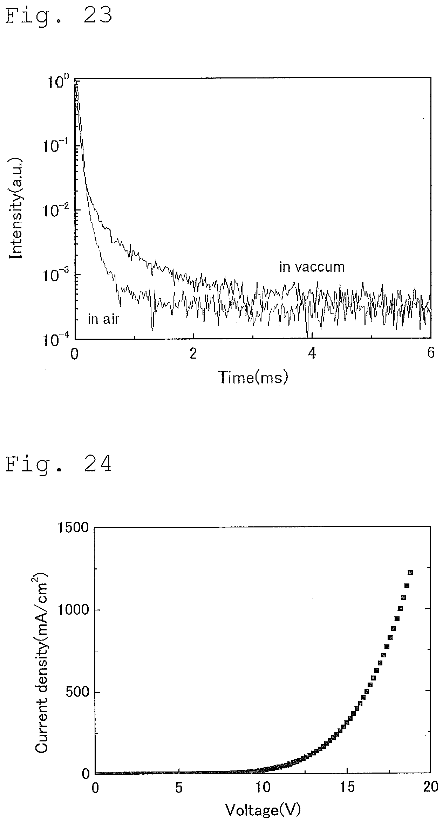

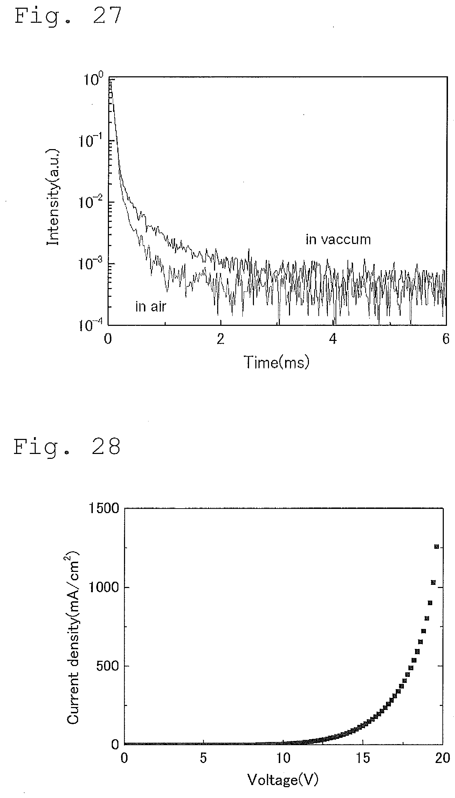

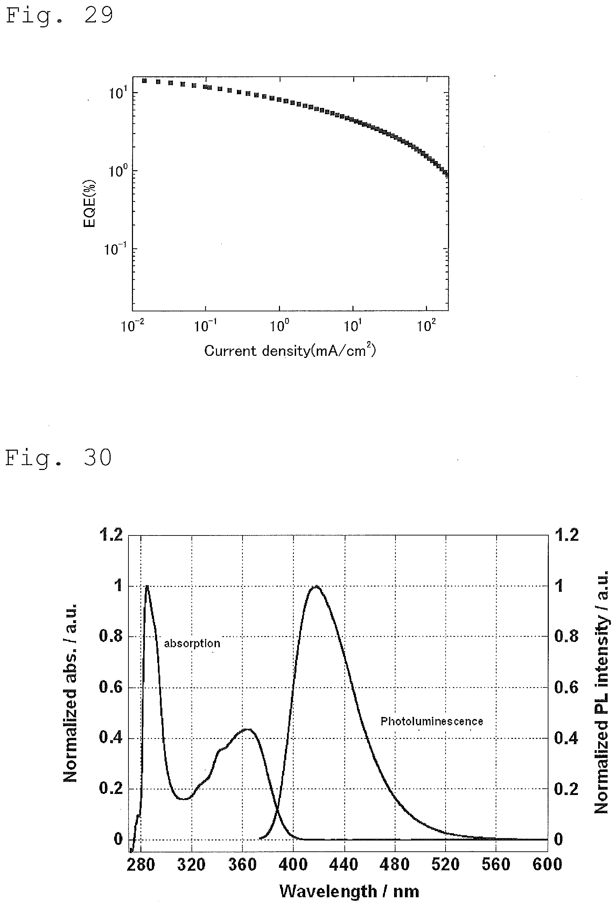

The light emission spectrum of the organic electroluminescent device using the compound 1 is shown in Fig. 2, the transient decay curves under ordinary pressure and in vacuum thereof are shown in Fig. 3, the voltage-electric current density characteristics thereof are shown in Fig. 4, and the electric current density-external quantum efficiency characteristics thereof are shown in Fig. 5. The light emission spectrum of the organic electroluminescent device using the compound 2 is shown in Fig. 6, the transient decay curves under ordinary pressure and in vacuum thereof are shown in Fig. 7, the voltage-electric current density characteristics thereof are shown in Fig. 8, and the electric current density-external quantum efficiency characteristics thereof are shown in Fig. 9. The light emission spectrum of the organic electroluminescent device using the compound 3 is shown in Fig. 10, the transient decay curves under ordinary pressure and in vacuum thereof are shown in Fig. 11, the voltage-electric current density characteristics thereof are shown in Fig. 12, and the electric current density-external quantum efficiency characteristics thereof are shown in Fig. 13. The light emission spectrum of the organic electroluminescent device using the compound 4 is shown in Fig. 14, the transient decay curves under ordinary pressure and in vacuum thereof are shown in Fig. 15, the voltage-electric current density characteristics thereof are shown in Fig. 16, and the electric current density-external quantum efficiency characteristics thereof are shown in Fig. 17. The light emission spectrum of the organic electroluminescent device using the compound 27 is shown in Fig. 18, the transient decay curves under ordinary pressure and in vacuum thereof are shown in Fig. 19, the voltage-electric current density characteristics thereof are shown in Fig. 20, and the electric current density-external quantum efficiency characteristics thereof are shown in Fig. 21. The light emission spectrum of the organic electroluminescent device using the compound 28 is shown in Fig. 22, the transient decay curves under ordinary pressure and in vacuum thereof are shown in Fig. 23, the voltage-electric current density characteristics thereof are shown in Fig. 24, and the electric current density-external quantum efficiency characteristics thereof are shown in Fig. 25. The light emission spectrum of the organic electroluminescent device using the compound 29 is shown in Fig. 26, the transient decay curves under ordinary pressure and in vacuum thereof are shown in Fig. 27, the voltage-electric current density characteristics thereof are shown in Fig. 28, and the electric current density-external quantum efficiency characteristics thereof are shown in Fig. 29.

The transient decay curve shows the measurement result of the light emission lifetime obtained by measuring the process where the light emission intensity is deactivated on irradiating the compound with excitation light. In ordinary one-component light emission (fluorescent light or phosphorescent light), the light emission intensity is decays monoexponentially. This means that the light emission intensity decays linearly on a graph with the semilogarithm as the ordinate. In a transient decay curve of a delayed fluorescence emitter, while a linear component (fluorescent light) is observed in the initial stage of observation, a component that deviates from the linearity appears after several microseconds. The later component is light emission of the delayed component, and the signal thereof added to the initial component appears as a long tail curve on the longer time side. Thus, the measurement of the light emission lifetime revealed that the compound of the invention is a light emitting material that contained a delayed component in addition to a fluorescent component.

### Comparative Example 1

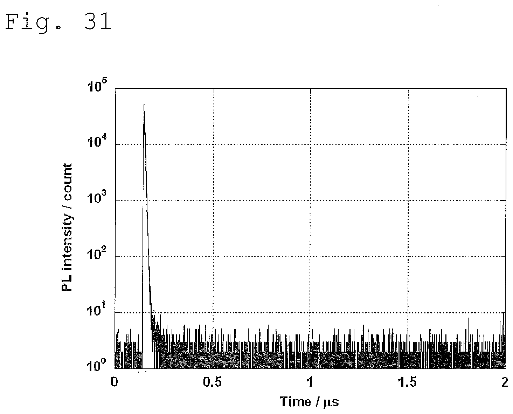

In this comparative example, a toluene solution of the compound A shown below described in JP-A-2009-21336 and JP-A-2002-193952 (concentration: 10⁻⁵ mol/L) was prepared and irradiated with light at 300 K under nitrogen bubbling, and thus the light emission spectrum shown in Fig. 30 was obtained. The solution exhibited the transient decay curve shown in Fig. 31, in which no delayed fluorescent component was observed.

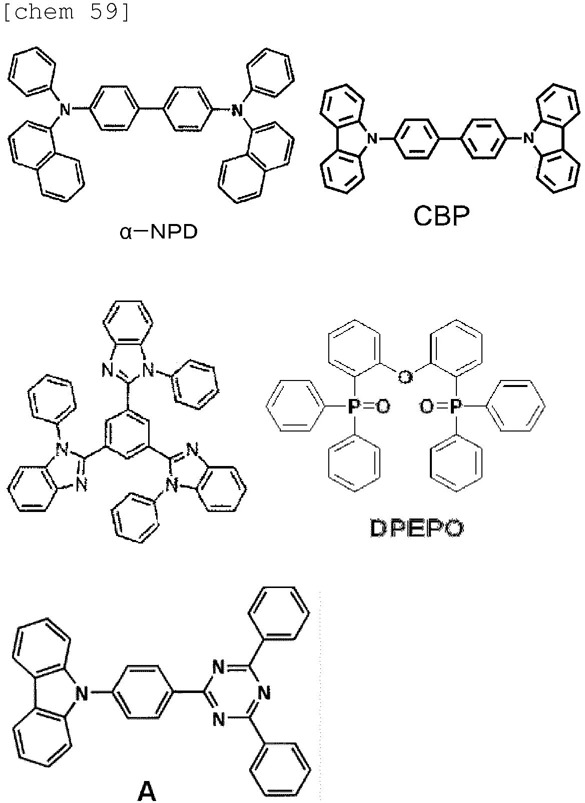

### Industrial Applicability

The organic light emitting device of the invention is capable of achieving a high light emission efficiency. The compound represented by the general formula (1) is useful as a light emitting material of such an organic light emitting device. Accordingly, the invention has high industrial applicability.

### Reference Signs List

- 1: substrate
- 2: anode
- 3: hole injection layer
- 4: hole transporting layer
- 5: light emitting layer
- 6: electron transporting layer
- 7: cathode

## Claims

1. Use of a compound represented by the following general formula (1) as a light emitting material: 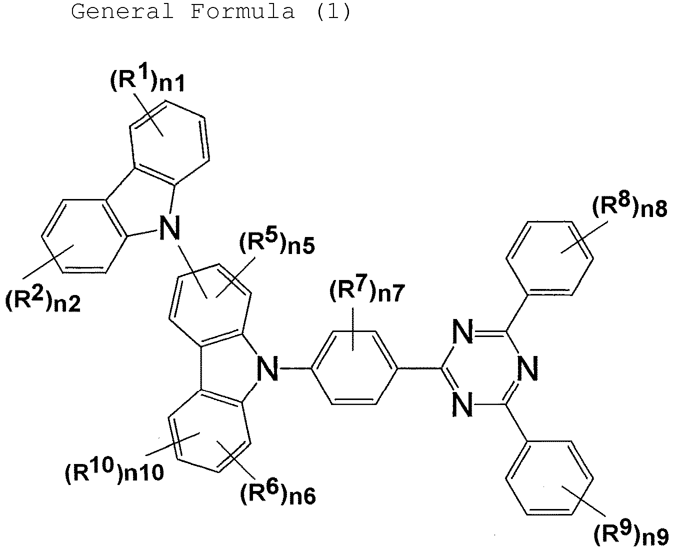 wherein in the general formula (1),
R¹ and R² each independently represent a substituted or unsubstituted carbazolyl group, an substituted or unsubstituted aryl group having 6 to 14 carbon atoms, a heteroaryl group having 3 to 10 carbon atoms, an alkyl group having 1 to 12 carbon atoms, or a cycloalkyl group having 5 to 12 carbon atoms;
R⁵ and R⁶ each independently represent a substituted or unsubstituted alkyl group;
R⁷, R⁸ and R⁹ each independently represent a substituted or unsubstituted aryl group, a substituted or unsubstituted alkyl group or a substituted or unsubstituted carbazolyl group;
whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group
R¹⁰ represents a carbazolyl group which may be substituted with an unsubstituted carbazolyl group, an unsubstituted aryl group, an unsubstituted heteroaryl group, an unsubstituted alkyl group or an unsubstituted cycloalkyl group;
n1, n2, n6 and n7 each independently represent an integer of from 0 to 4;
n5 represents an integer of from 0 to 3;
n8 and n9 each independently represent an integer of from 0 to 5; and
n10 represents 0 or 1,
provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.

2. The use according to claim 1, wherein the compound represented by the general formula (1) is a compound represented by the following general formula (2): 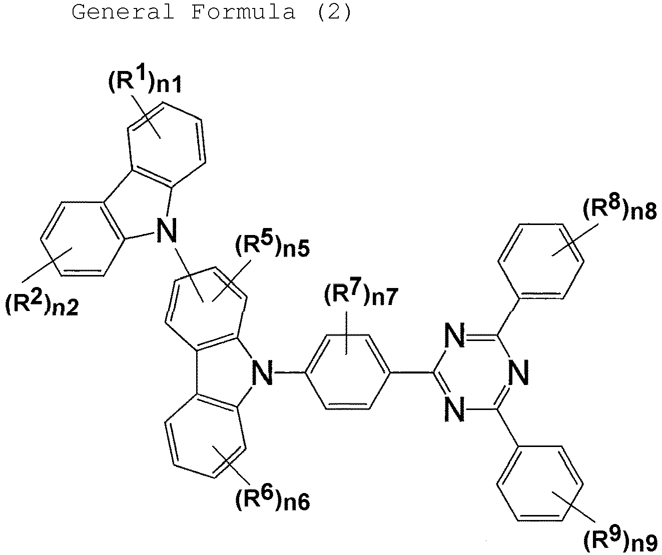 wherein in the general formula (2),
R¹ and R² each independently represent a substituted or unsubstituted carbazolyl group, a substituted or unsubstituted aryl group, a substituted or unsubstituted heteroaryl group having 3 to 10 carbon atoms, a substituted or unsubstituted alkyl group, or a substituted or unsubstituted cycloalkyl group;
R⁵ and R⁶ each independently represent a substituted or unsubstituted alkyl group;
R⁷, R⁸ and R⁹ each independently represent a substituted or unsubstituted aryl group, a substituted or unsubstituted alkyl group or a substituted or unsubstituted carbazolyl group;
whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
n1, n2, n6 and n7 each independently represent an integer of from 0 to 4;
n5 represents an integer of from 0 to 3; and
n8 and n9 each independently represent an integer of from 0 to 5,
provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.

3. The use according to claim 1, wherein the compound represented by the general formula (1) is a compound represented by the following general formula (3): 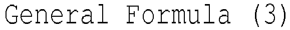 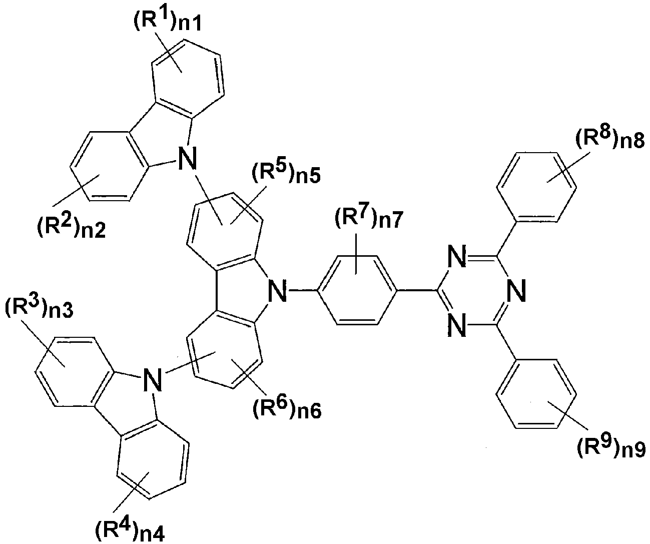 wherein in the general formula (3),
R¹ to R⁴ each independently represent a substituted or unsubstituted carbazolyl group, an substituted or unsubstituted aryl group, a substituted or unsubstituted heteroaryl group having 3 to 10 carbon atoms, an substituted or unsubstituted alkyl grou, or a cycloalkyl group;
R⁵ and R⁶ each independently represent a substituted or unsubstituted alkyl group;
R⁷, R⁸ and R⁹ each independently represent a substituted or unsubstituted aryl group, a substituted or unsubstituted alkyl group or a substituted or unsubstituted carbazolyl group;
whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
n1 to n4 and n7 each independently represent an integer of from 0 to 4;
n5 and n6 each represent an integer of from 0 to 3; and
n8 and n9 each independently represent an integer of from 0 to 5,
provided that when n1 to n9 each represent an integer of 2 or more, plural groups represented by R¹ to R⁹ corresponding to n1 to n9 respectively each may be the same as or different from each other.

4. The use according to claim 1, wherein the light emitting material emits delayed fluorescent light.

5. The use according to claim 2, wherein in the general formula (2), n6 represents 0 or n1 represents an integer of from 1 to 4.

6. The use according to claim 2, wherein in the general formula (2), R¹ is bonded to the 3-position of the carbazolyl group.

7. The use according to claim 2, wherein in the general formula (2), n2 represents an integer of from 1 to 4.

8. The use according to claim 7, wherein in the general formula (2), R² is bonded to the 6-position of the carbazolyl group.

9. The use according to claim 1 or 2, wherein in the general formulae (1) and (2), R¹ and R² each independently represent a substituted or unsubstituted 9-carbazolyl group, a substituted or unsubstituted phenyl group, a substituted or unsubstituted pyridyl group, an alkyl group having from 1 to 6 carbon atoms or a cycloalkyl group having from 5 to 7 carbon atoms, preferably R¹ and R² each independently represent a 9-carbazolyl group, a phenyl group, a tolyl group, a dimethylphenyl group, a trimethylphenyl group, a biphenyl group, a pyridyl group, a pyrrolyl group, a tert-butyl group or a cyclohexyl group.

10. Use of a compound represented by the following general formula (1) as a delayed fluorescence emitter: 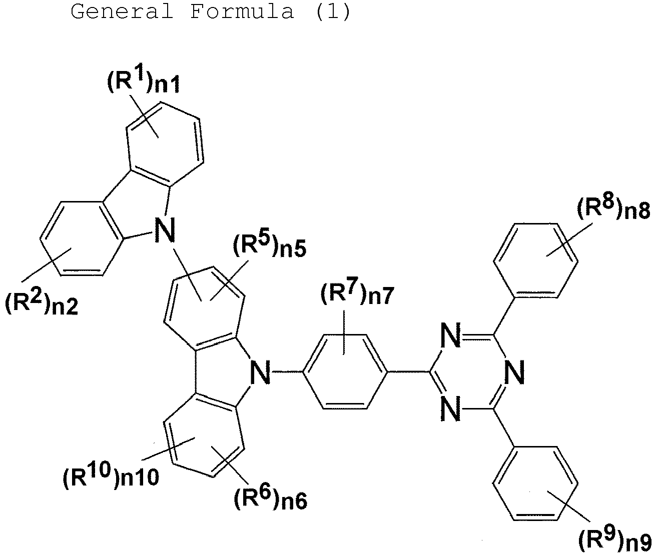 wherein in the general formula (1),
R¹ and R² each independently represent a substituted or unsubstituted carbazolyl group, a substituted or unsubstituted aryl group having 6 to 14 carbon atoms, a substituted or unsubstituted heteroaryl group having 3 to 10 carbon atoms, a substituted or unsubstituted alkyl group having 1 to 12 carbon atoms, or a substituted or unsubstituted cycloalkyl group having 5 to 12 carbon atoms;
R⁷, R⁸ and R⁹ each independently represent a substituted or unsubstituted aryl group, a substituted or unsubstituted alkyl group or a substituted or unsubstituted carbazolyl group;
whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
R¹⁰ represents a carbazolyl group which may be substituted with an unsubstituted carbazolyl group, an unsubstituted aryl group, an unsubstituted heteroaryl group, an unsubstituted alkyl group or an unsubstituted cycloalkyl group;
n1, n2, n6 and n7 each independently represent an integer of from 0 to 4;
n5 represents an integer of from 0 to 3;
n8 and n9 each independently represent an integer of from 0 to 5; and
n10 represents 0 or 1,
provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.

11. A compound represented by the following general formula (2) : 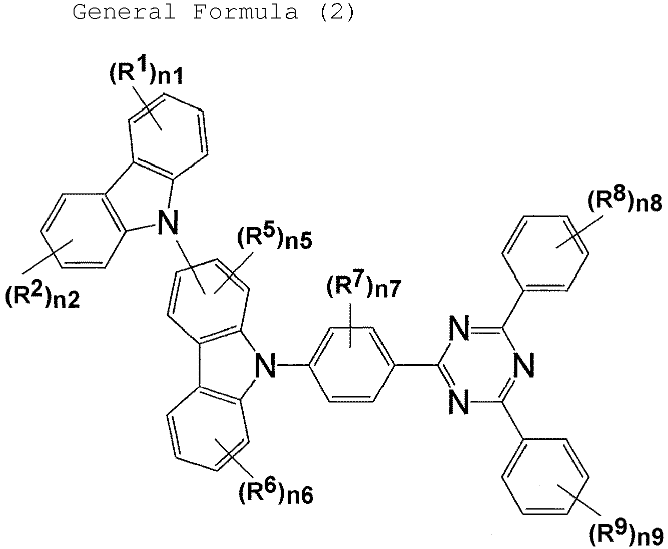 wherein in the general formula (2),
R¹ and R² each independently represent a substituted or unsubstituted carbazolyl group, a substituted or unsubstituted aryl group having 6 to 14 carbon atoms, a substituted or unsubstituted heteroaryl group having 3 to 10 carbon atoms, a substituted or unsubstituted alkyl group having 1 to 12 carbon atoms, or a substituted or unsubstituted cycloalkyl group having 5 to 12 carbon atoms;
R⁵ and R⁶ each independently represent a substituted or unsubstituted alkyl group;
R⁷, R⁸ and R⁹ each independently represent a substituted or unsubstituted aryl group, a substituted or unsubstituted alkyl group or a substituted or unsubstituted carbazolyl group;
whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
n1, n2, n6 and n7 each independently represent an integer of from 0 to 4;
n5 represents an integer of from 0 to 3; and
n8 and n9 each independently represent an integer of from 0 to 5,
provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.

12. The compound according to claim 11, wherein the general formula (2) is represented by the following general formula (4): 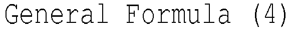 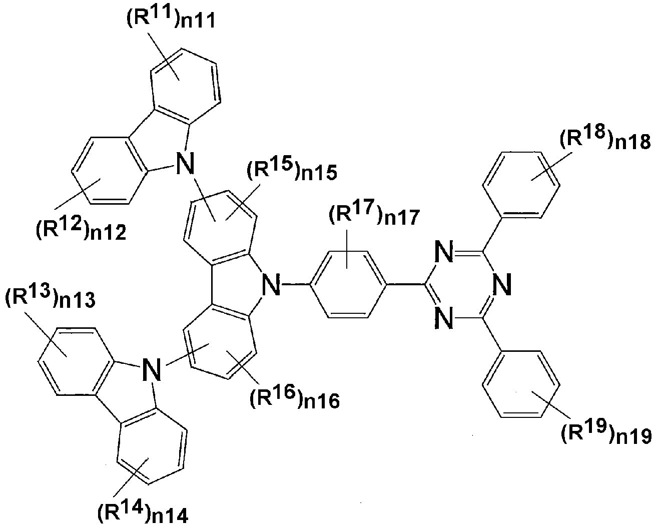 wherein in the general formula (4),
R¹¹ to R¹⁴ each independently represent a carbazolyl group which may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group; an aryl group having 6 to 14 carbon atoms which may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group; a heteroaryl group having 3 to 10 carbon atoms, which heteroaryl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group; an alkyl group having 1 to 12 carbon atoms which may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group; or a cycloalkyl group having 5 to 12 carbon atoms which may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
R¹⁵ and R¹⁶ each independently represent an alkyl group;
R¹⁷, R¹⁸ and R¹⁹ each independently represent a substituted or unsubstituted aryl group, a substituted or unsubstituted alkyl group or a substituted or unsubstituted carbazolyl group;
n11 to n14 and n17 each independently represent an integer of from 0 to 4;
n15 and n16 each represent an integer of from 0 to 3; and n18 and n19 each independently represent an integer of from 0 to 5,
provided that at least one of n11 to n14 represents an integer of from 1 to 4, and
when n11 to n19 each represent an integer of 2 or more, plural groups represented by R¹¹ to R¹⁹ corresponding to n11 to n19 respectively each may be the same as or different from each other.

13. An organic light emitting device containing a substrate having thereon a light emitting layer containing a host material and a light emitting material represented by the following general formula (1): 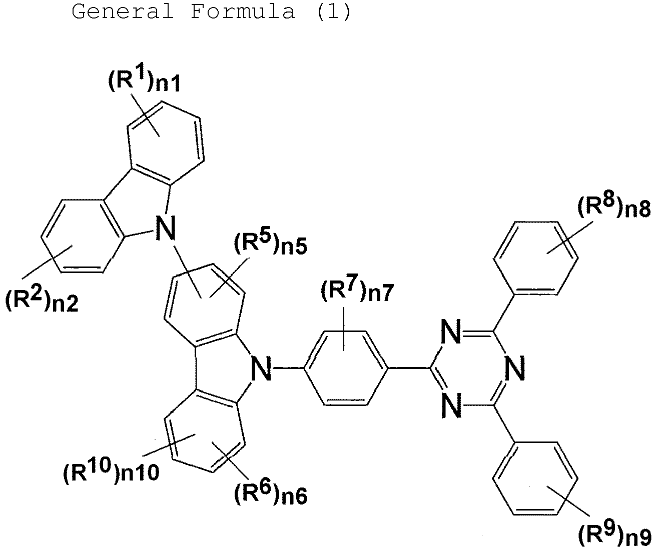 wherein in the general formula (1),
R¹ and R² each independently represent a substituted or
unsubstituted carbazolyl group, a substituted or unsubstituted aryl group having 6 to 14 carbon atoms, a substituted or unsubstituted heteroaryl group having 3 to 10 carbon atoms, a substituted or unsubstituted alkyl group having 1 to 12 carbon atoms, or a substituted or unsubstituted cycloalkyl group having 5 to 12 carbon atoms;
R⁷, R⁸ and R⁹ each independently represent a substituted or unsubstituted aryl group, a substituted or unsubstituted alkyl group or a substituted or unsubstituted carbazolyl group;
whereby the carbazolyl group, the aryl group, the heteroaryl group, the alkyl group, or the cycloalkyl group may be substituted by a hydroxyl group, a halogen atom, a cyano group, an alkyl group having from 1 to 12 carbon atoms, an alkoxy group having from 1 to 12 carbon atoms, an alkylthio group having from 1 to 12 carbon atoms, an alkyl-substituted amino group having from 1 to 12 carbon atoms, an acyl group having from 2 to 12 carbon atoms, an aryl group having from 6 to 14 carbon atoms, a heteroaryl group having from 3 to 13 carbon atoms, a diarylamino group having from 12 to 20 carbon atoms, a substituted or unsubstituted carbazolyl group having from 12 to 20 carbon atoms, an alkenyl group having from 2 to 10 carbon atoms, an alkynyl group having from 2 to 10 carbon atoms, an alkoxycarbonyl group having from 2 to 10 carbon atoms, an alkylsulfonyl group having from 1 to 10 carbon atoms, a haloalkyl group having from 1 to 10 carbon atoms, an amide group, an alkylamide group having from 2 to 10 carbon atoms, a trialkylsilyl group having from 3 to 12 carbon atoms, a trialkylsilylalkyl group having from 4 to 12 carbon atoms, a trialkylsilylalkenyl group having from 5 to 14 carbon atoms, a trialkylsilylalkynyl group having from 5 to 14 carbon atoms, or a nitro group;
R¹⁰ represents a carbazolyl group which may be substituted with an unsubstituted carbazolyl group, an unsubstituted aryl group, an unsubstituted heteroaryl group, an unsubstituted alkyl group or an unsubstituted cycloalkyl group;
n1, n2, n6 and n7 each independently represent an integer of from 0 to 4;
n5 represents an integer of from 0 to 3;
n8 and n9 each independently represent an integer of from 0 to 5; and
n10 represents 0 or 1,
provided that when n1, n2 and n5 to n9 each represent an integer of 2 or more, plural groups represented by R¹, R² and R⁵ to R⁹ corresponding to n1, n2 and n5 to n9 respectively each may be the same as or different from each other.

14. The organic light emitting device according to claim 13, wherein the organic light emitting device emits delayed fluorescent light.

15. The organic light emitting device according to claim 13 or 14, wherein the organic light emitting device is an organic electroluminescent device.

## Patentansprüche

1. Verwendung einer Verbindung, dargestellt durch die folgende allgemeine Formel (1), als ein Licht emittierendes Material: 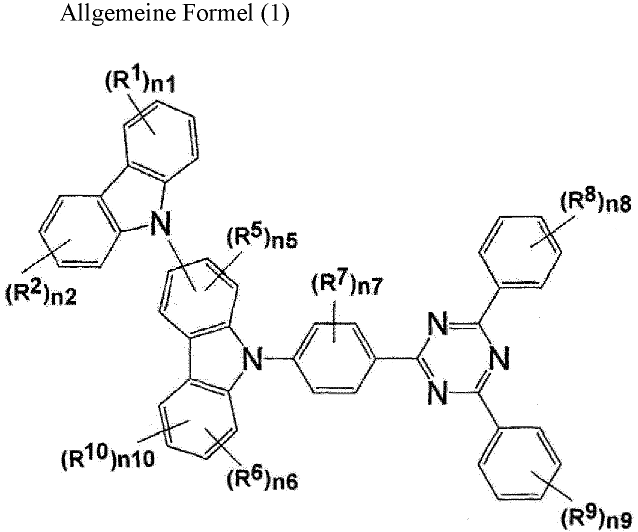 wobei in der allgemeinen Formel (1)
R¹ und R² jeweils unabhängig voneinander eine substituierte oder unsubstituierte Carbazolylgruppe, eine substituierte oder unsubstituierte Arlygruppe mit 6 bis 14 Kohlenstoffatomen, eine Heteroarylgruppe mit 3 bis 10 Kohlenstoffatomen, eine Alkylgruppe mit 1 bis 12 Kohlenstoffatomen, oder eine Cycloalkylgruppe mit 5 bis 12 Kohlenstoffatomen darstellen;
R⁵ und R⁶ jeweils unabhängig voneinander eine substituierte oder unsubstituierte Alkylgruppe darstellen;
R⁷, R⁸ und R⁹ jeweils unabhängig voneinander eine substituierte oder unsubstituierte Arylgruppe, eine substituierte oder unsubstituierte Alkylgruppe oder eine substituierte oder unsubstituierte Carbazolylgruppe darstellen;
wobei die Carbazolylgruppe, die Arylgruppe, die Heteroarylgruppe, die Alkylgruppe oder die Cycloalkylgruppe ersetzt sein kann durch eine Hydroxylgruppe, ein Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
R¹⁰ eine Carbazolylgruppe darstellt, die durch eine unsubstituierte Carbazolylgruppe, eine unsubstituierte Arylgruppe, eine unsubstituierte Heteroarylgruppe, eine unsubstituierte Alkylgruppe oder eine unsubstituierte Cycloalkylgruppe substituiert sein kann;
n1, n2, n6 und n7 jeweils unabhängig voneinander eine ganze Zahl von 0 bis 4 darstellen;
n5 eine ganze Zahl von 0 bis 3 darstellt;
n8 und n9 jeweils unabhängig voneinander eine ganze Zahl von 0 bis 5 darstellen; und
n10 0 oder 1 darstellt,
vorausgesetzt, dass, wenn n1, n2 und n5 bis n9 jeweils eine ganze Zahl von 2 oder größer darstellen, mehrere Gruppen, dargestellt durch R¹, R² und R⁵ bis R⁹ beziehungsweise entsprechend n1, n2 und n5 bis n9, jeweils gleich sein können oder sich voneinander unterscheiden können.

2. Verwendung nach Anspruch 1, wobei die Verbindung, dargestellt durch die allgemeine Formel (1), eine Verbindung ist, die durch die folgende allgemeine Formel (2) dargestellt ist: 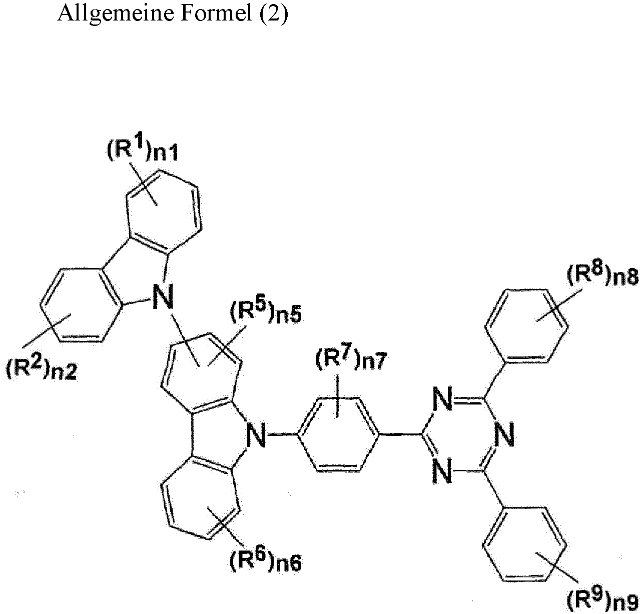 wobei in der allgemeinen Formel (2)
R¹ und R² jeweils unabhängig voneinander eine substituierte oder unsubstituierte Carbazolylgruppe, eine substituierte oder unsubstituierte Arylgruppe, eine substituierte oder unsubstituierte Heteroarylgruppe mit 3 bis 10 Kohlenstoffatomen, eine substituierte oder unsubstituierte Alkylgruppe oder eine substituierte oder unsubstituierte Cycloalkylgruppe darstellen;
R⁵ und R⁶ jeweils unabhängig voneinander eine substituierte oder unsubstituierte Alkylgruppe darstellen;
R⁷, R⁸ und R⁹ jeweils unabhängig voneinander eine substituierte oder unsubstituierte Arylgruppe, eine substituierte oder unsubstituierte Alkylgruppe oder eine substituierte oder unsubstituierte Carbazolylgruppe darstellen;
wobei die Carbazolylgruppe, die Arylgruppe, die Heteroarylgruppe, die Alkylgruppe oder die Cycloalkylgruppe ersetzt sein kann durch eine Hydroxylgruppe, ein Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
n1, n2, n6 und n7 jeweils unabhängig voneinander eine ganze Zahl von 0 bis 4 darstellen;
n5 eine ganze Zahl von 0 bis 3 darstellt; und
n8 und n9 jeweils unabhängig voneinander eine ganze Zahl von 0 bis 5 darstellen, vorausgesetzt, dass, wenn n1, n2 und n5 bis n9 jeweils eine ganze Zahl von 2 oder größer darstellen, mehrere Gruppen, dargestellt durch R¹, R² und R⁵ bis R⁹ beziehungsweise entsprechend n1, n2 und n5 bis n9, jeweils gleich sein können oder sich voneinander unterscheiden können.

3. Verwendung nach Anspruch 1, wobei die Verbindung, dargestellt durch die allgemeine Formel (1), eine Verbindung ist, die durch die folgende allgemeine Formel (3) dargestellt ist: 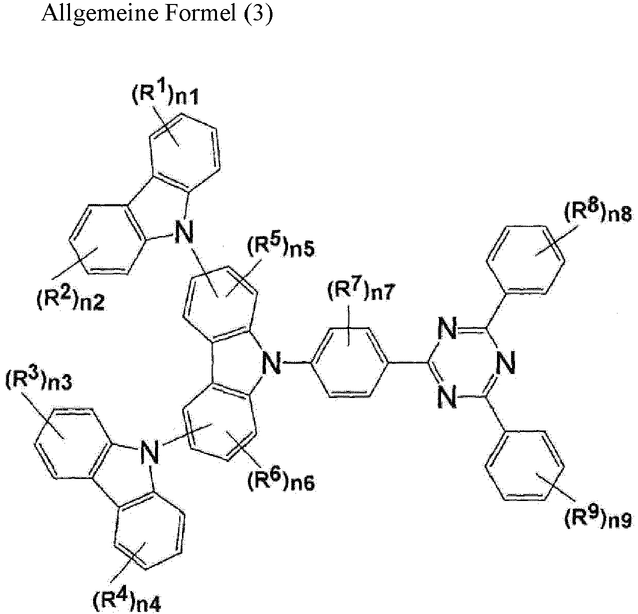 wobei in der allgemeinen Formel (3)
R¹ bis R⁴ jeweils unabhängig voneinander eine substituierte oder unsubstituierte Carbazolylgruppe, eine substituierte oder unsubstituierte Arylgruppe, eine substituierte oder unsubstituierte Heteroarylgruppe mit 3 bis 10 Kohlenstoffatomen, eine substituierte oder unsubstituierte Alkylgruppe oder eine Cycloalkylgruppe darstellen;
R⁵ und R⁶ jeweils unabhängig voneinander eine substituierte oder unsubstituierte Alkylgruppe darstellen;
R⁷, R⁸ und R⁹ jeweils unabhängig voneinander eine substituierte oder unsubstituierte Arylgruppe, eine substituierte oder unsubstituierte Alkylgruppe oder eine substituierte oder unsubstituierte Carbazolylgruppe darstellen;
wobei die Carbazolylgruppe, die Arylgruppe, die Heteroarylgruppe, die Alkylgruppe oder die Cycloalkylgruppe ersetzt sein kann durch eine Hydroxylgruppe, ein Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
n1 bis n4 und n7 jeweils unabhängig voneinander eine ganze Zahl von 0 bis 4 darstellen;
n5 und n6 jeweils eine ganze Zahl von 0 bis 3 darstellen; und
n8 und n9 jeweils unabhängig voneinander eine ganze Zahl von 0 bis 5 darstellen, vorausgesetzt, dass, wenn n1 bis n9 jeweils eine ganze Zahl von 2 oder größer darstellen, mehrere Gruppen, dargestellt durch R¹ bis R⁹ beziehungsweise entsprechend n1 bis n9, jeweils gleich sein können oder sich voneinander unterscheiden können.

4. Verwendung nach Anspruch 1, wobei das Licht emittierende Material verzögertes fluoreszierendes Licht aussendet.

5. Verwendung nach Anspruch 2, wobei in der allgemeinen Formel (2) n6 0 darstellt oder n1 eine ganze Zahl von 1 bis 4 darstellt.

6. Verwendung nach Anspruch 2, wobei in der allgemeinen Formel (2) R¹ an die 3-Position der Carbazolylgruppe gebunden ist.

7. Verwendung nach Anspruch 2, wobei in der allgemeinen Formel (2) n2 eine ganze Zahl von 1 bis 4 darstellt.

8. Verwendung nach Anspruch 7, wobei in der allgemeinen Formel (2) R² an die 6-Position der Carbazolylgruppe gebunden ist.

9. Verwendung nach einem der Ansprüche 1 oder 2, wobei in den allgemeinen Formeln (1) und (2) R¹ und R² jeweils unabhängig voneinander eine substituierte oder unsubstituierte 9-Carbazolylgruppe, eine substituierte oder unsubstituierte Phenylgruppe, eine substituierte oder unsubstituierte Pyridylgruppe, eine Alkylgruppe mit 1 bis 6 Kohlenstoffatomen oder eine Cycloalkylgruppe mit 5 bis 7 Kohlenstoffatomen darstellen, vorzugsweise R¹ und R² jeweils unabhängig voneinander eine 9-Carbazolylgruppe, eine Phenylgruppe, eine Tolylgruppe, eine Dimethylphenylgruppe, eine Trimethylphenylgruppe, eine Biphenylgruppe, eine Pyridylgruppe, eine Pyrrolylgruppe, eine Tert-butylgruppe oder eine Cyclohexylgruppe darstellen.

10. Verwendung einer Verbindung, dargestellt durch die folgende allgemeine Formel (1), als verzögerter Fluoreszenz-Emitter: 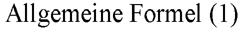 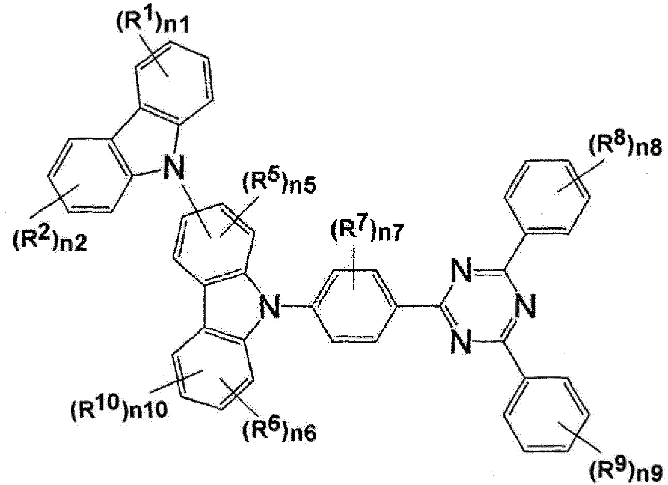 wobei in der allgemeinen Formel (1)
R¹ und R² jeweils unabhängig voneinander eine substituierte oder unsubstituierte Carbazolylgruppe, eine substituierte oder unsubstituierte Arylgruppe mit 6 bis 14 Kohlenstoffatomen, eine substituierte oder unsubstituierte Heteroarylgruppe mit 3 bis 10 Kohlenstoffatomen, eine substituierte oder unsubstituierte Alkylgruppe mit 1 bis 12 Kohlenstoffatomen oder eine substituierte oder unsubstituierte Cycloalkylgruppe mit 5 bis 12 Kohlenstoffatomen darstellen;
R⁷, R⁸ und R⁹ jeweils unabhängig voneinander eine substituierte oder unsubstituierte Arylgruppe, eine substituierte oder unsubstituierte Alkylgruppe oder eine substituierte oder unsubstituierte Carbazolylgruppe darstellen;
wobei die Carbazolylgruppe, die Arylgruppe, die Heteroarylgruppe, die Alkylgruppe oder die Cycloalkylgruppe ersetzt sein kann durch eine Hydroxylgruppe, ein Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
R¹⁰ eine Carbazolylgruppe darstellt, die mit einer unsubstituierten Carbazolylgruppe, einer unsubstituierten Arylgruppe, einer unsubstituierten Heteroarylgruppe, einer unsubstituierten Alkylgruppe oder einer unsubstituierten Cycloalkylgruppe substituiert sein kann;
n1, n2, n6 und n7 jeweils unabhängig voneinander eine ganze Zahl von 0 bis 4 darstellen;
n5 eine ganze Zahl von 0 bis 3 darstellt;
n8 und n9 jeweils unabhängig voneinander eine ganze Zahl von 0 bis 5 darstellen; und
n10 0 oder 1 darstellt,
vorausgesetzt, dass, wenn n1, n2 und n5 bis n9 jeweils eine ganze Zahl von 2 oder höher darstellen, mehrere Gruppen dargestellt durch R¹, R² und R⁵ bis R⁹ beziehungsweise entsprechend n1, n2, n5 bis n9, jeweils gleich sein können oder sich voneinander unterscheiden können.

11. Verbindung, dargestellt durch die folgende allgemeine Formel (2): 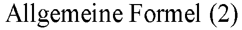 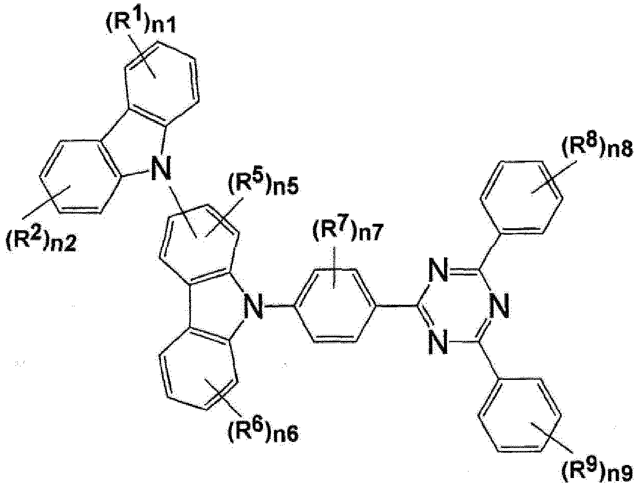 wobei in der allgemeinen Formel (2)
R¹ und R² jeweils unabhängig voneinander eine substituierte oder unsubstituierte Carbazolylgruppe, eine substituierte oder unsubstituierte Arylgruppe mit 6 bis 14 Kohlenstoffatomen, eine substituierte oder unsubstituierte Heteroarylgruppe mit 3 bis 10 Kohlenstoffatomen, eine substituierte oder unsubstituierte Alkylgruppe mit 1 bis 12 Kohlenstoffatomen oder eine substituierte oder unsubstituierte Cycloalkylgruppe mit 5 bis 12 Kohlenstoffatomen darstellen;
R⁵ und R⁶ jeweils unabhängig voneinander eine substituierte oder unsubstituierte Alkylgruppe darstellen;
R⁷, R⁸ und R⁹ jeweils unabhängig voneinander eine substituierte oder unsubstituierte Arylgruppe, eine substituierte oder unsubstituierte Alkylgruppe oder eine substituierte oder unsubstituierte Carbazolylgruppe darstellen;
wobei die Carbazolylgruppe, die Arylgruppe, die Heteroarylgruppe, die Alkylgruppe oder die Cycloalkylgruppe ersetzt sein kann durch eine Hydroxylgruppe, ein Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
n1, n2, n6 und n7 jeweils unabhängig voneinander eine ganze Zahl von 0 bis 4 darstellen;
n5 eine ganze Zahl von 0 bis 3 darstellt; und
n8 und n9 jeweils unabhängig voneinander eine ganze Zahl von 0 bis 5 darstellen, vorausgesetzt, dass, wenn n1, n2 und n5 bis n9 jeweils eine ganze Zahl von 2 oder größer darstellen, mehrere Gruppen, dargestellt durch R¹, R² und R⁵ bis R⁹ beziehungsweise entsprechend n1, n2 und n5 bis n9, jeweils gleich sein können oder sich voneinander unterscheiden können.

12. Verbindung nach Anspruch 11, wobei die allgemeine Formel (2) durch die folgende allgemeine Formel (4) dargestellt ist: 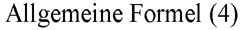 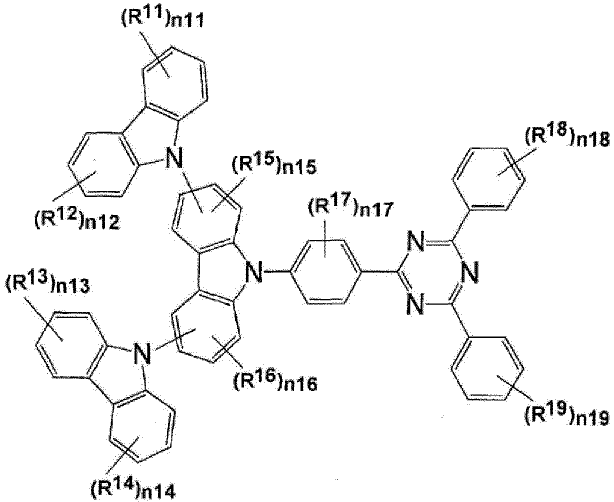 wobei in der allgemeinen Formel (4)
R¹¹ bis R¹⁴ jeweils unabhängig voneinander eine Carbazolylgruppe darstellen, die substituiert sein kann durch eine Hydroxylgruppe, eine Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
eine Arylgruppe mit 6 bis 14 Kohlenstoffatomen, die substituiert sein kann durch eine Hydroxylgruppe, eine Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
eine Heteroarylgruppe mit 3 bis 10 Kohlenstoffatomen, die substituiert sein kann durch eine Hydroxylgruppe, eine Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
eine Alkylgruppe mit 1 bis 12 Kohlenstoffatomen, die substituiert sein kann durch eine Hydroxylgruppe, eine Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
oder eine Cycloalkylgruppe mit 5 bis 12 Kohlenstoffatomen, die substituiert sein kann durch eine Hydroxylgruppe, eine Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
R¹⁵ und R¹⁶ jeweils unabhängig voneinander eine Alkylgruppe darstellen;
R¹⁷, R¹⁸ und R¹⁹ jeweils unabhängig voneinander eine substituierte oder unsubstituierte Arylgruppe, eine substituierte oder unsubstituierte Alkylgruppe oder eine substituierte oder unsubstituierte Carbazolylgruppe darstellen;
n11 bis n14 und n17 jeweils unabhängig voneinander eine ganze Zahl von 0 bis 4 darstellen;
n15 und n16 jeweils eine ganze Zahl von 0 bis 3 darstellen; und
n18 und n19 jeweils unabhängig voneinander eine ganze Zahl von 0 bis 5 darstellen, vorausgesetzt, dass mindestens eine von n11 bis n14 eine ganze Zahl von 1 bis 4 darstellt, und
wenn n11 bis n19 jeweils eine ganze Zahl von 2 oder größer darstellen, mehrere Gruppen, dargestellt durch R¹¹ bis R¹⁹ beziehungsweise entsprechend n11 bis n19, jeweils gleich sein können oder sich voneinander unterscheiden können.

13. Organisches Licht emittierendes Gerät, das ein Substrat enthält, welches eine Licht emittierende Schicht trägt, die ein Wirtsmaterial und ein Licht emittierendes Material enthält, dargestellt in der folgenden allgemeinen Formel (1): 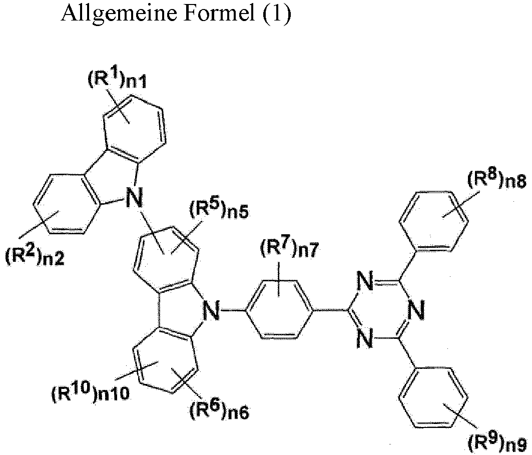 wobei in der allgemeinen Formel (1)
R¹ und R² jeweils unabhängig voneinander eine substituierte oder unsubstituierte Carbazolylgruppe, eine substituierte oder unsubstituierte Arylgruppe mit 6 bis 14 Kohlenstoffatomen, eine substituierte oder unsubstituierte Heteroarylgruppe mit 3 bis 10 Kohlenstoffatomen, eine substituierte oder unsubstituierte Alkylgruppe mit 1 bis 12 Kohlenstoffatomen oder eine substituierte oder unsubstituierte Cycloalkylgruppe mit 5 bis 12 Kohlenstoffatomen darstellen;
R⁷, R⁸ und R⁹ jeweils unabhängig voneinander eine substituierte oder unsubstituierte Arylgruppe, eine substituierte oder unsubstituierte Alkylgruppe oder eine substituierte oder unsubstituierte Carbazolylgruppe darstellen;
wobei die Carbazolylgruppe, die Arylgruppe, die Heteroarylgruppe, die Alkylgruppe oder die Cycloalkylgruppe ersetzt sein kann durch eine Hydroxylgruppe, ein Halogenatom, eine Cyanogruppe, eine Alkylgruppe mit 1 bis 12 Kohlenstoffatomen, eine Alkoxygruppe mit 1 bis 12 Kohlenstoffatomen, eine Alkylthiogruppe mit 1 bis 12 Kohlenstoffatomen, eine Alkyl-substituierte Aminogruppe mit 1 bis 12 Kohlenstoffatomen, eine Acylgruppe mit 2 bis 12 Kohlenstoffatomen, eine Arylgruppe mit 6 bis 14 Kohlenstoffatomen, eine Heteroarylgruppe mit 3 bis 13 Kohlenstoffatomen, eine Diarylaminogruppe mit 12 bis 20 Kohlenstoffatomen, eine substituierte oder unsubstituierte Carbazolylgruppe mit 12 bis 20 Kohlenstoffatomen, eine Alkenylgruppe mit 2 bis 10 Kohlenstoffatomen, eine Alkynylgruppe mit 2 bis 10 Kohlenstoffatomen, eine Alkoxycarbonylgruppe mit 2 bis 10 Kohlenstoffatomen, eine Alkylsulfonylgruppe mit 1 bis 10 Kohlenstoffatomen, eine Haloalkylgruppe mit 1 bis 10 Kohlenstoffatomen, eine Amidgruppe, eine Alkylamidgruppe mit 2 bis 10 Kohlenstoffatomen, eine Trialkylsilylgruppe mit 3 bis 12 Kohlenstoffatomen, eine Trialkylsilylalkylgruppe mit 4 bis 12 Kohlenstoffatomen, eine Trialkylsilylalkenylgruppe mit 5 bis 14 Kohlenstoffatomen, eine Trialkylsilylalkynylgruppe mit 5 bis 14 Kohlenstoffatomen, oder eine Nitrogruppe;
R¹⁰ eine Carbazolylgruppe darstellt, die mit einer unsubstituierten Carbazolylgruppe, einer unsubstituierten Arylgruppe, einer unsubstituierten Heteroarylgruppe, einer unsubstituierten Alkylgruppe oder einer unsubstituierten Cycloalkylgruppe substituiert sein kann;
n1, n2, n6 und n7 jeweils unabhängig voneinander eine ganze Zahl von 0 bis 4 darstellen;
n5 eine ganze Zahl von 0 bis 3 darstellt;
n8 und n9 jeweils unabhängig voneinander eine ganze Zahl von 0 bis 5 darstellen; und n10 0 oder 1 darstellt,
vorausgesetzt, dass, wenn n1, n2 und n5 bis n9 jeweils eine ganze Zahl von 2 oder höher darstellen, mehrere Gruppen dargestellt durch R¹, R² und R⁵ bis R⁹ beziehungsweise entsprechend n1, n2, n5 bis n9, jeweils gleich sein können oder sich voneinander unterscheiden können.

14. Organisches Licht emittierendes Gerät nach Anspruch 13, wobei das organisches Licht emittierende Gerät verzögertes fluoreszierendes Licht emittiert.

15. Organisches Licht emittierendes Gerät nach Anspruch 13 oder14, wobei das organisches Licht emittierende Gerät eine organische Elektrolumineszenz-Vorrichtung ist.

## Revendications

1. Utilisation d'un composé représenté par la formule générale (1) suivante en tant que matériau luminescent : 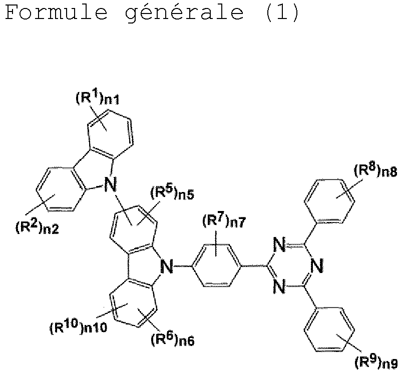 dans laquelle dans la formule générale (1),
R¹ et R² représentent chacun indépendamment un groupe carbazolyle substitué ou non substitué, un groupe aryle substitué ou non substitué ayant 6 à 14 atomes de carbone, un groupe hétéroaryle ayant 3 à 10 atomes de carbone, un groupe alkyle ayant 1 à 12 atomes de carbone, ou un groupe cycloalkyle ayant 5 à 12 atomes de carbone ;
R⁵ et R⁶ représentent chacun indépendamment un groupe alkyle substitué ou non substitué ;
R⁷, R⁸ et R⁹ représentent chacun indépendamment un groupe aryle substitué ou non substitué, un groupe alkyle substitué ou non substitué ou un groupe carbazolyle substitué ou non substitué ;
moyennant quoi le groupe carbazolyle, le groupe aryle, le groupe hétéroaryle, le groupe alkyle, ou le groupe cycloalkyle peuvent être substitués par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro
R¹⁰ représente un groupe carbazolyle qui peut être substitué par un groupe carbazolyle non substitué, un groupe aryle non substitué, un groupe hétéroaryle non substitué, un groupe alkyle non substitué ou un groupe cycloalkyle non substitué ;
n1, n2, n6 et n7 représentent chacun indépendamment un entier de 0 à 4 ;
n5 représente un entier de 0 à 3 ;
n8 et n9 représentent chacun indépendamment un entier de 0 à 5 ; et
n10 représente 0 ou 1,
à la condition que lorsque n1, n2 et n5 à n9 représentent chacun un entier de 2 ou plus, plusieurs groupes représentés par R¹, R² et R⁵ à R⁹ correspondant à n1, n2 et n5 à n9 chacun respectivement puissent être identiques ou différents les uns des autres.

2. Utilisation selon la revendication 1, dans laquelle le composé représenté par la formule générale (1) est un composé représenté par la formule générale (2) suivante : 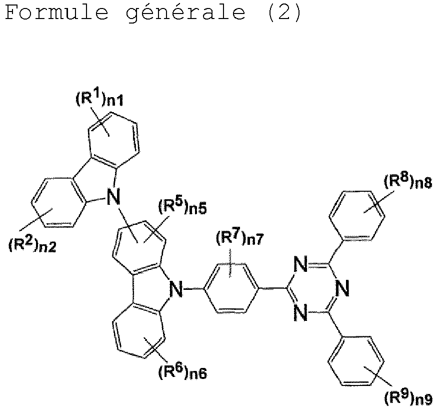 dans laquelle dans la formule générale (2),
R¹ et R² représentent chacun indépendamment un groupe carbazolyle substitué ou non substitué, un groupe aryle substitué ou non substitué, un groupe hétéroaryle ayant 3 à 10 atomes de carbone, un groupe alkyle substitué ou non substitué, ou un groupe cycloalkyle substitué ou non substitué ;
R⁵ et R⁶ représentent chacun indépendamment un groupe alkyle substitué ou non substitué ;
R⁷, R⁸ et R⁹ représentent chacun indépendamment un groupe aryle substitué ou non substitué, un groupe alkyle substitué ou non substitué ou un groupe carbazolyle substitué ou non substitué ;
moyennant quoi le groupe carbazolyle, le groupe aryle, le groupe hétéroaryle, le groupe alkyle, ou le groupe cycloalkyle peuvent être substitués par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ;
n1, n2, n6 et n7 représentent chacun indépendamment un entier de 0 à 4 ;
n5 représente un entier de 0 à 3 ; et
n8 et n9 représentent chacun indépendamment un entier de 0 à 5,
à la condition que lorsque n1, n2 et n5 à n9 représentent chacun un entier de 2 ou plus, plusieurs groupes représentés par R¹, R² et R⁵ à R⁹ correspondant à n1, n2 et n5 à n9 chacun respectivement puissent être identiques ou différents les uns des autres.

3. Utilisation selon la revendication 1, dans laquelle le composé représenté par la formule générale (1) est un composé représenté par la formule générale (3) suivante : 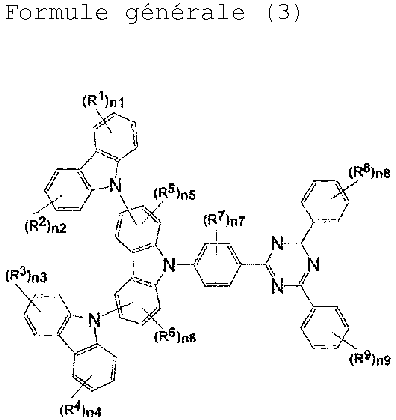 dans laquelle dans la formule générale (3),
R¹ à R⁴ représentent chacun indépendamment un groupe carbazolyle substitué ou non substitué, un groupe aryle substitué ou non substitué, un groupe hétéroaryle substitué ou non substitué ayant 3 à 10 atomes de carbone, un groupe alkyle substitué ou non substitué, ou un groupe cycloalkyle ;
R⁵ et R⁶ représentent chacun indépendamment un groupe alkyle substitué ou non substitué ;
R⁷, R⁸ et R⁹ représentent chacun indépendamment un groupe aryle substitué ou non substitué, un groupe alkyle substitué ou non substitué ou un groupe carbazolyle substitué ou non substitué ;
moyennant quoi le groupe carbazolyle, le groupe aryle, le groupe hétéroaryle, le groupe alkyle, ou le groupe cycloalkyle peuvent être substitués par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ;
n1 à n4 et n7 représentent chacun indépendamment un entier de 0 à 4 ;
n5 et n6 représentent chacun un entier de 0 à 3 ; et
n8 et n9 représentent chacun indépendamment un entier de 0 à 5,
à la condition que lorsque n1 à n9 représentent chacun un entier de 2 ou plus, plusieurs groupes représentés par R¹ à R⁹ correspondant à n1 à n9 chacun respectivement puissent être identiques ou différents les uns des autres.

4. Utilisation selon la revendication 1, dans laquelle le matériau luminescent émet une lumière fluorescente retardée.

5. Utilisation selon la revendication 2, dans laquelle dans la formule générale (2), n6 représente 0 ou n1 représente un entier de 1 à 4.

6. Utilisation selon la revendication 2, dans laquelle dans la formule générale (2), R¹ est lié à la position 3 du groupe carbazolyle.

7. Utilisation selon la revendication 2, dans laquelle dans la formule générale (2), n2 représente un entier de 1 à 4.

8. Utilisation selon la revendication 7, dans laquelle dans la formule générale (2), R² est lié à la position 6 du groupe carbazolyle.

9. Utilisation selon la revendication 1 ou 2, dans laquelle dans les formules générales (1) et (2), R¹ et R² représentent chacun indépendamment un groupe 9-carbazolyle substitué ou non substitué, un groupe phényle substitué ou non substitué, un groupe pyridyle substitué ou non substitué, un groupe alkyle ayant de 1 à 6 atomes de carbone ou un groupe cycloalkyle ayant de 5 à 7 atomes de carbone, de préférence R¹ et R² représentent chacun indépendamment un groupe 9-carbazolyle, un groupe phényle, un groupe tolyle, un groupe diméthylphényle, un groupe triméthylphényle, un groupe biphényle, un groupe pyridyle, un groupe pyrrolyle, un groupe tert-butyle ou un groupe cyclohexyle.

10. Utilisation d'un composé représenté par la formule générale (1) suivante en tant qu'émetteur de fluorescence retardée : 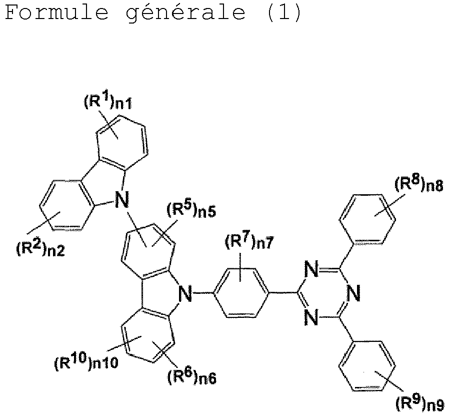 dans laquelle dans la formule générale (1),
R¹ et R² représentent chacun indépendamment un groupe carbazolyle substitué ou non substitué, un groupe aryle substitué ou non substitué ayant 6 à 14 atomes de carbone, un groupe hétéroaryle substitué ou non substitué ayant 3 à 10 atomes de carbone, un groupe alkyle substitué ou non substitué ayant 1 à 12 atomes de carbone, ou un groupe cycloalkyle substitué ou non substitué ayant 5 à 12 atomes de carbone ;
R⁷, R⁸ et R⁹ représentent chacun indépendamment un groupe aryle substitué ou non substitué, un groupe alkyle substitué ou non substitué ou un groupe carbazolyle substitué ou non substitué ;
moyennant quoi le groupe carbazolyle, le groupe aryle, le groupe hétéroaryle, le groupe alkyle, ou le groupe cycloalkyle peuvent être substitués par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ;
R¹⁰ représente un groupe carbazolyle qui peut être substitué par un groupe carbazolyle non substitué, un groupe aryle non substitué, un groupe hétéroaryle non substitué, un groupe alkyle non substitué ou un groupe cycloalkyle non substitué ;
n1, n2, n6 et n7 représentent chacun indépendamment un entier de 0 à 4 ;
n5 représente un entier de 0 à 3 ;
n8 et n9 représentent chacun indépendamment un entier de 0 à 5 ; et
n10 représente 0 ou 1,
à la condition que lorsque n1, n2 et n5 à n9 représentent chacun un entier de 2 ou plus, plusieurs groupes représentés par R¹, R² et R⁵ à R⁹ correspondant à n1, n2 et n5 à n9 chacun respectivement puissent être identiques ou différents les uns des autres.

11. Composé représenté par la formule générale (2) suivante : 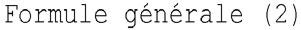 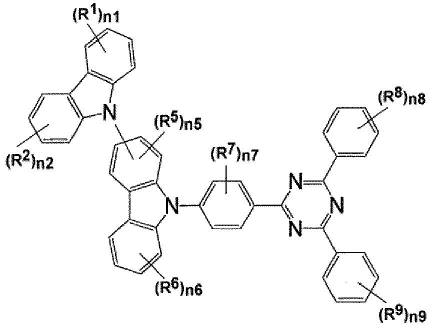 dans laquelle dans la formule générale (2),
R¹ et R² représentent chacun indépendamment un groupe carbazolyle substitué ou non substitué, un groupe aryle substitué ou non substitué ayant 6 à 14 atomes de carbone, un groupe hétéroaryle substitué ou non substitué ayant 3 à 10 atomes de carbone, un groupe alkyle substitué ou non substitué ayant 1 à 12 atomes de carbone, ou un groupe cycloalkyle substitué ou non substitué ayant 5 à 12 atomes de carbone ;
R⁵ et R⁶ représentent chacun indépendamment un groupe alkyle substitué ou non substitué ;
R⁷, R⁸ et R⁹ représentent chacun indépendamment un groupe aryle substitué ou non substitué, un groupe alkyle substitué ou non substitué ou un groupe carbazolyle substitué ou non substitué ;
moyennant quoi le groupe carbazolyle, le groupe aryle, le groupe hétéroaryle, le groupe alkyle, ou le groupe cycloalkyle peuvent être substitués par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ;
n1, n2, n6 et n7 représentent chacun indépendamment un entier de 0 à 4 ;
n5 représente un entier de 0 à 3 ; et
n8 et n9 représentent chacun indépendamment un entier de 0 à 5,
à la condition que lorsque n1, n2 et n5 à n9 représentent chacun un entier de 2 ou plus, plusieurs groupes représentés par R¹, R² et R⁵ à R⁹ correspondant à n1, n2 et n5 à n9 chacun respectivement puissent être identiques ou différents les uns des autres.

12. Composé selon la revendication 11, dans lequel la formule générale (2) est représentée par la formule générale (4) suivante : 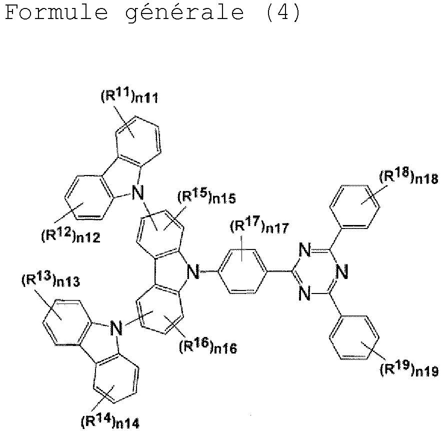 dans lequel dans la formule générale (4),
R¹¹ à R¹⁴ représentent chacun indépendamment un groupe carbazolyle qui peut être substitué par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ; un groupe aryle ayant de 6 à 14 atomes de carbone qui peut être substitué par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ; un groupe hétéroaryle ayant de 3 à 10 atomes de carbone, lequel groupe hétéroaryle peut être substitué par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ; un groupe alkyle ayant de 1 à 12 atomes de carbone qui peut être substitué par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ; un groupe cycloalkyle ayant de 5 à 12 atomes de carbone qui peut être substitué par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ;
R¹⁵ et R¹⁶ représentent chacun indépendamment un groupe alkyle ;
R¹⁷, R¹⁸ et R¹⁹ représentent chacun indépendamment un groupe aryle substitué ou non substitué, un groupe alkyle substitué ou non substitué ou un groupe carbazolyle substitué ou non substitué ;
n11 à n14 et n17 représentent chacun indépendamment un entier de 0 à 4 ;
n15 et n16 représentent chacun un entier de 0 à 3 ; et
n18 et n19 représentent chacun indépendamment un entier de 0 à 5,
à la condition qu'au moins un de n11 à n14 représente un entier de 1 à 4, et
lorsque n11 à n19 représentent chacun un entier de 2 ou plus, plusieurs groupes représentés par R¹¹ à R¹⁹ correspondant à n11 à n19 chacun respectivement puissent être identiques ou différents les uns des autres.

13. Dispositif luminescent organique contenant un substrat ayant sur celui-ci une couche luminescente contenant un matériau hôte et un matériau luminescent représenté par la formule générale (1) suivante : 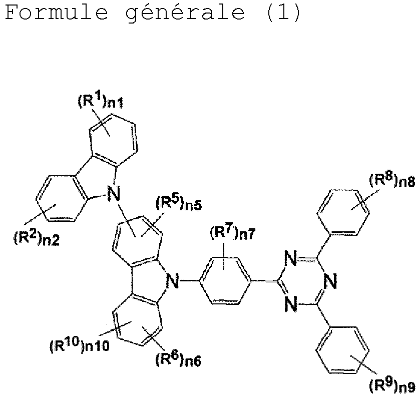 dans laquelle dans la formule générale (1),
R¹ et R² représentent chacun indépendamment un groupe carbazolyle substitué ou non substitué, un groupe aryle substitué ou non substitué ayant 6 à 14 atomes de carbone, un groupe hétéroaryle substitué ou non substitué ayant 3 à 10 atomes de carbone, un groupe alkyle substitué ou non substitué ayant 1 à 12 atomes de carbone, ou un groupe cycloalkyle substitué ou non substitué ayant 5 à 12 atomes de carbone ;
R⁷, R⁸ et R⁹ représentent chacun indépendamment un groupe aryle substitué ou non substitué, un groupe alkyle substitué ou non substitué ou un groupe carbazolyle substitué ou non substitué ;
moyennant quoi le groupe carbazolyle, le groupe aryle, le groupe hétéroaryle, le groupe alkyle, ou le groupe cycloalkyle peuvent être substitués par un groupe hydroxyle, un atome d'halogène, un groupe cyano, un groupe alkyle ayant de 1 à 12 atomes de carbone, un groupe alcoxy ayant de 1 à 12 atomes de carbone, un groupe alkylthio ayant de 1 à 12 atomes de carbone, un groupe amino, substitué par un alkyle, ayant de 1 à 12 atomes de carbone, un groupe acyle ayant de 2 à 12 atomes de carbone, un groupe aryle ayant de 6 à 14 atomes de carbone, un groupe hétéroaryle ayant de 3 à 13 atomes de carbone, un groupe diarylamino ayant de 12 à 20 atomes de carbone, un groupe carbazolyle substitué ou non substitué ayant de 12 à 20 atomes de carbone, un groupe alcényle ayant de 2 à 10 atomes de carbone, un groupe alcynyle ayant de 2 à 10 atomes de carbone, un groupe alcoxycarbonyle ayant de 2 à 10 atomes de carbone, un groupe alkylsulfonyle ayant de 1 à 10 atomes de carbone, un groupe haloalkyle ayant de 1 à 10 atomes de carbone, un groupe amide, un groupe alkylamide ayant de 2 à 10 atomes de carbone, un groupe trialkylsilyle ayant de 3 à 12 atomes de carbone, un groupe trialkylsilylalkyle ayant de 4 à 12 atomes de carbone, un groupe trialkylsilylalcényle ayant de 5 à 14 atomes de carbone, un groupe trialkylsilylalcynyle ayant de 5 à 14 atomes de carbone, ou un groupe nitro ;
R¹⁰ représente un groupe carbazolyle qui peut être substitué par un groupe carbazolyle non substitué, un groupe aryle non substitué, un groupe hétéroaryle non substitué, un groupe alkyle non substitué ou un groupe cycloalkyle non substitué ;
n1, n2, n6 et n7 représentent chacun indépendamment un entier de 0 à 4 ;
n5 représente un entier de 0 à 3 ;
n8 et n9 représentent chacun indépendamment un entier de 0 à 5 ; et
n10 représente 0 ou 1,
à la condition que lorsque n1, n2 et n5 à n9 représentent chacun un entier de 2 ou plus, plusieurs groupes représentés par R¹, R² et R⁵ à R⁹ correspondant à n1, n2 et n5 à n9 chacun respectivement puissent être identiques ou différents les uns des autres.

14. Dispositif luminescent organique selon la revendication 13, dans lequel le dispositif luminescent organique émet une lumière fluorescente retardée.

15. Dispositif luminescent organique selon la revendication 13 ou 14, dans lequel le dispositif luminescent organique est un dispositif électroluminescent organique.
